(19) Europäisches Patentamt
European Patent Office
Office européen des brevets

(11) EP 3 290 020 B1

(12) # EUROPÄISCHE PATENTSCHRIFT

(45) Veröffentlichungstag und Bekanntmachung des Hinweises auf die Patenterteilung:
**24.11.2021 Patentblatt 2021/47**

(21) Anmeldenummer: **17020389.7**

(22) Anmeldetag: **25.08.2017**

(51) Int Cl.:
***A61K 8/45*** *(2006.01)* ***A61Q 5/00*** *(2006.01)*
***A61K 8/60*** *(2006.01)* ***A61Q 9/02*** *(2006.01)*
***A61Q 11/00*** *(2006.01)* ***A61Q 19/00*** *(2006.01)*

(54) **MILDE ZUBEREITUNGEN MIT ALKOXYLIERTEN FETTSÄUREAMIDEN**

MILD PREPARATIONS CONTAINING ALKOXYLATED FATTY ACID AMIDES

PRÉPARATIONS DOUCES CONTENANT DES AMIDES D’ACIDES GRAS ALCOXYLÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.08.2016 CH 11122016**

(43) Veröffentlichungstag der Anmeldung:
**07.03.2018 Patentblatt 2018/10**

(73) Patentinhaber: **Perfect Ideas GmbH**
**6043 Adligenswil (CH)**

(72) Erfinder:
- **Der Erfinder hat auf sein Recht verzichtet, als solcher bekannt gemacht zu werden.**

(56) Entgegenhaltungen:
**WO-A1-2013/182759**
**DE-A1- 4 409 189**
**DE-A1- 19 600 743**
**DE-A1-102009 046 169**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 290 020 B1

## Beschreibung

### Zusammenfassung

Gegenstand dieser Erfindung sind milde, kosmetische Zubereitungen mit alkoxylierten Fettsäureamiden zur nicht-therapeutischen Verwendung als kosmetisches oder medizinisches Reinigungs- oder Pflegemittel.

### Gegenstand

Gegenstand dieser Anmeldung sind kosmetische Zubereitungen, die mindestens ein Glycolipid-Biotensid und mindestens ein alkoxyliertes Fettsäureamid enthalten. Das oder die alkoxylierten Tenside basieren auf Fettsäuren von Pflanzenölen mit einem aussergewöhnlich hohen Anteil an langkettigen, ungesättigten Kohlenwasserstoffketten. Weiterer Gegenstand ist deren nicht-therapeutische Verwendung als oder zur Herstellung von kosmetischen oder medizinischen Reinigungs- oder Pflegemitteln.

### Problem - Stand der Technik

Die vorliegende Erfindung richtet sich auf eine kosmetische Zubereitung mit verbesserter dermatologischer Verträglichkeit und die nicht-therapeutische Verwendung der Mittel zur Körperreinigung und -pflege, der Haarwäsche, dem Abschminken, der Zahnpflege, und der Rasur insbesondere für die Entfernung von Kohlenhydraten und Anfärbungen unterschiedlichster Art.

Die Entfernung von kohlenhydrathaltigen Verunreinigungen ist in der Körperpflege ausserordentlich wichtig. Insbesondere bei erkrankter Haut spielen Pilze oder Bakterien eine zentrale Rolle wie z.B. Propionibacterium acnes (Akne), Streptococcen (Zahnbelag), Malassezia furfur (Haarschnuppen). Bekannt ist, dass oberflächenaktive Mittel die Permeabilität der biologischen Membranen modifizieren und damit antibakteriell, antiviral oder fungizid wirken. So offenbart z.B. EP 209 783 (Wella) Sophorolipide und deren Verwendung als Antischuppenmittel und bakteriostatisches Mittel. Abgesehen von der antimikrobiellen Wirkung, ist es jedoch von grösster Bedeutung, der Besieldung durch Mikroorganismen von vornherein präventiv entgegenzutreten. Dies ist möglich, indem man den Mikroorganismen ihre Nahrungsgrundlage - welche in erster Linie aus Kohlenhydraten bestehen- entzieht oder- im Falle von Zahnbelag- eine frühe Matrixbildung aus Kohlenhydraten und Eiweiss - frühzeitig unterbindet. Insbesondere kommt erschwerend hinzu, dass Kohlenhydrate zusätzlich die Zahngesundheit beeinträchtigen können, da sie von bestimmten Bakteriensorten in der Mundhöhle zu beispielsweise Milchsäure abgebaut werden und den Zahnschmelz der juvenilen oder adulten Zähne angreifen können (Karies).

Die Kohlenhydratentfernung ist auch in der Gesichtspflege und Rasur wichtig. Insbesondere das Gesicht ist Umwelteinflüssen und -verschmutzung ausgesetzt. So können sich z.B. Pollen, deren Hauptbestandteil Kohlenhydrate sind sowie Russ- und Staubteilchen an Haut und Haaren adsorbieren.
Weiterhin ist die Kohlenhydratreinigung wichtig bei der Haarpflege um Reste von Styling-Produkten leicht aus dem Haar auszuwaschen. Ein Ziel der Körperhygiene stellt daher die effektive Entfernung von Kohlenhydraten dar.

Ein weiterer Aspekt hygienischer Körperreinigung ist die Entfernung der häufig pathogenen Stoffwechselprodukte. So produziert beispielsweise Malassezia furfur fluoreszierende Pigmente, welche mit der pathogenen Wirkung in Zusammenhang gebracht werden. So ist es für eine Reinigung, sei es in der Mundhygiene, der Haut oder den Haaren, wichtig, neben der guten Entfernung von Kohlenhydraten, auch die Entfernung von Farb-bzw. Pigmentschmutz zu erzielen.

Anders als bei der Materialreinigung, ist eine gute Reinigungsleistung insbesondere bei der Reinigung von Körper oder Fell problematisch, da die Reinigungsleistung auf die Anwendung auf einen schmalen Temperaturbereich von ca. 15 - ca. 40°C begrenzt ist und auch die mechanische Entfernung von Schmutz nur in limitiertem Masse möglich ist. Weiterhin muss auf agressive Inhaltsstoffe sowie extreme pH-Werte < 2 oder > 12 verzichtet werden. Insbesondere bei der Gesichtsreinigung und Entfernung von Augenmakeup ist zudem erwünscht, eine sanfte Reinigung ohne mechanische Kräfte zu erzielen.

Üblicherweise werden für die Entfernung von hartnäckigem Schmutz Tensidgemische eingesetzt. Häufig enthalten Reinigungsprodukte zur mechanischen Reinigung Reibekörper wie Holzmehl, Cellulose, Plastikpartikel, Kaolin, Sand und andere. Besonders hartnäckige Verschmutzungen werden unter Zusatz agressiver organischer Lösungsmittel entfernt, wie z.B. aliphatische Kohlenwasserstoffe.

Je häufiger die Produkte angewendet werden, z.B. bei empfindlichen Partien wie Augenbereich, Intimbereich oder auch im gewerblichen Bereich, desto deutlicher werden die nachteiligen Wirkungen der enthaltenen Inhaltsstoffe. So ist zum Beispiel eine Vielzahl der üblicherweise verwendeten Tenside als hautirritierend bekannt. Insbesondere führen auch die üblicherweise verwendeten Lösungsmittel zu einer Entfettung und Austrocknung der Haut durch die Zerstörung des Hydro-Lipid-Mantels. Die Haut wird damit permeabler für toxische oder allergene Stoffe und reagiert

durch Hautreizungen und allergene Hautreaktionen.

Zur Vermeidung der vorgenannten Hautproblematik sind in der Patentliteratur eine Vielzahl von Formulierungen vorgeschlagen worden. Nichtsdestoweniger gehen die verbesserte Hautverträglichkeit sowie der verbesserte Schutz vor Hautaustrocknung mit einem Verlust an Reinigungswirkung einher.

Die Entfernung von Farb- und Pigmentresten aus der dekorativen Kosmetik ist hier eine weitere Herausforderung - eine milde Reinigung insbesondere von empfindlichen Hautstellen, wie den Augenbereich, nimmt eine Schlüsselposition ein. Technisch herausfordernd ist, dass die dekorative Kosmetik auf unterschiedlichen Formulierungskonzepten beruht und der Konsument oftmals zu mehreren Produkten zur Entfernung von wasser- bzw. öllöslicher Kosmetik greifen muss. Alternativ gibt es 2-Phasenprodukte.

Eine milde Reinigung von hartnäckigen Verunreinigungen ist weiterhin im gewerblichen Bereich wünschenswert, wie z.B. Lackierer, Maler, Friseure, Automechaniker, Gärtner u.a. Aber auch im künstlerischen, Freizeitbereich und Familienbereich ist die Entfernung von Farbflecken von Haut und Haaren eine häufige Notwendigkeit. Nicht zuletzt ist auch in der Tierpflege häufig eine Grobreinigung notwendig, zum Beispiel die Fellpflege von Haustieren.

Neben dem ehrgeizigen Ziel eine bessere Hautverträglichkeit bei hoher Reinigungskraft zu erreichen, sollen gleichzeitig die Inhaltsstoffe aus nachwachsenden Rohstoffen bezogen werden. Hierbei haben sich in den vergangenen Jahren Palmöle, für Wasch- und Reinigungsmittel v.a. Palmkernöl, als pflanzliche Basis für Tenside etabliert.

Jedoch wird die Nachhaltigkeit der Tenside auf Basis Palmöle zunehmend in Frage gestellt, da sie aus tropischen Monokulturen stammen, deren Anbaufläche oftmals durch unkontrollierte Rodung wertvoller tropischer Regenwälder gewonnen wird. Diese Pflanzenöle werden aufgrund ihrer technischen Eigenschaften wie vorteilhafte Schaum-, Wasch- und Reinigungsleistung eingesetzt, die sie dank ihrem hohen Laurinsäuregehalt (C12- Fettsäure, gesättigt) besitzen.

Alternativ werden in einem geringeren Mass auch andere Palmöle mit hohem ' Laurinsäuregehalt wie bspw. Kokosöl oder Babassuöl eingesetzt. Zudem finden seit jeher auch tierische Öle und Fette-Anwendung. Aufgrund der ungünstigeren Fettsäurezusammensetzung, aus hygienischen (z.B. TSE-Problematik) und weltanschaulichen Gründen (z.B. vegan-Trend) sind allerdings tierische Rohstoffe aus Konsumentensicht oft nicht erwünscht. Weiterhin wird Pflanzenseife seit Jahrtausenden für Wasch- und Reinigungszwecke eingesetzt, deren Anwendung ist aufgrund der Bildung von Kalkseifen und Bindung an einen alkalischen pH-Wert ebenfalls begrenzt.

Die Herausforderung dieser Erfindung besteht daher darin, auf Erdöl, tierische Fette und Palmöle (Palmöl, Palmkernöl, Kokosöl, Babassuöl) als Fettsäurequelle für die Tenside als Hauptkomponente zu verzichten und in einem grösstmöglichen Mass Tenside aus weniger problematischen Quellen, wie zum Beispiel pflanzlichen Ölen aus europäischem Anbau oder Fermentation einzusetzen.

Technisch ist dies ein Problem, da aus verfügbaren Ölen, zum Beispiel aus Mitteleuropa, die gewünschte Laurinsäure nicht in ausreichendem Mass gewonnen werden kann und damit heutzutage übliche Reinigungsmittel weitestgehend auf Erdöl oder Palmölen basieren.

Glycolipid-Biotenside, welche über Fermentation aus unterschiedlichsten Substraten hergestellt werden können, erfüllen die Anforderungen an Nachhaltigkeit und zeichnen sich zudem durch geringes Hautirritationspotential und Toxizität aus. Weiterhin verfügen sie über ein ausgezeichnetes Fett- und Öllösevermögen, was für die kosmetische Verwendung von Vorteil ist; jedoch die Haut stark austrocknen kann. Um alle Anforderungen an kosmetische Mittel zu erfüllen, wie z.B. Schaum, Reinigungskraft, Stabilität u.a. werden die Glycolipid-Biotenside in der Regel mit anderen Tensiden kombiniert.

Hierbei werden die Glycolipid-Biotenside nach Stand der Technik wiederum mit Tensiden auf Laurinsäurebasis aus Palmen oder petrochemischer Herkunft kombiniert z.B. DE19600743 (Henkel) hautverträgliche Geschirrspülmittel, WO 2011120776 (Unilever) milde und schäumende Waschzusammensetzungen für die Körperreinigung, JP 2009275145 (Seraya) Hautreinigung mit Sophorolipidenin Kombination mit Seifen, WO 2016050439 (Evonik) bioterisidhaltige Formulierungen mit verbessertem Schaumbildungs- und Fettlösevermögen. Weiterhin bekannt aus EP 0499434, US 5520839 ist die Kombination von Biotensiden mit anionischen Tensiden u.a. Rapsseife für eine verbesserte Öl- und Fettlöslichkeit.

Alle bisher vorgestellten Kombinationen von Biotensid-Glycolipiden mit Tensiden enthalten hydrophobe Teile der Tenside, welche von Laurinsäure abgeleitet sind. Die Kombination von Glycolipiden mit Lösungsmittel für nicht-kosmetische Anwendungen ist aus DE 102009046169 bekannt. WO 2013/182759 offenbart die Verwendung von Sophorolipiden als Lösungsvermittler von schlecht wasserlöslichen Substanzen.

Desweiteren ist die Verwendung von langkettigen, ungesättigten alkoxylierten Fettsäureamiden für kosmetische Zwecke in der Literatur bekannt. Diese Tensidklasse wird eingesetzt für die milde Körperreinigung, dank Ihrer guten Verdickbarkeit, der Möglichkeit transparenter Formulierungen, deren gutes Schaumvermögen (DE 44 09 189, Chemische Fabrik, Chem-Y) sowie für eine erhöhte Stabilität der Zusammensetzungen (z.B. Grobhandreinigung EP 2 455 062, Stockhausen). Kombiniert werden die Amide nach Stand der Technik in den offenbarten Ausführungsbeispielen wiederum bevorzugt mit Natrium Laurethsulfat.

Damit ist aber das Problem nicht gelöst. Nachhaltige Zubereitungen mit Biotensid-Glycolipiden und Tensiden auf Basis von Pflanzölen mit einem hohen Gehalt an langkettigen, vorwiegend ungesättigten Fettsäuren herzustellen,

welche eine hohe Reinigungs- und Pflegeleistung, insbesondere auf Kohlenhydrate und Anfärbungen, und gleichzeitig eine hervorragende dermatologische Verträglichkeit aufweisen und zudem über den gesamten pH-Bereich angewendet werden können.

Die komplexe technische Aufgabe der Erfindung hat darin bestanden, Tensidkombinationen zu identifizieren, die neben einem vorteilhaften toxikologischen Profil ausgezeichnete kosmetische Reinigungseigenschaften ausweisen. Gleichzeitig sollen aus Nachhaltigkeitsbetrachtungen entgegen dem Stand der Technik möglichst keine Tenside abgeleitet aus den Fettsäuren der Palmöle, Kokos-, Palm-, Babassu- oder Palmkernöl, oder Erdöl verwendet werden. Dies ist technisch insofern anspruchsvoll, da gebräuchliche Tenside auf Basis Palmkernöl in ihrer Fettsäurezusammensetzung einen hohen Anteil an Laurinsäure (C12) aufweisen, welche dem Tensid vorteilhafte Eigenschaften wie Löslichkeit, Stabilität, Benetzungsfähigkeit, Kompatibilität, u.a. verleihen. Die Herausforderung besteht somit darin, pflegende und reinigende Zubereitungen von Tensiden auf Basis von Pflanzenölen mit einem hohen Gehalt an Fettsäuren mit Kettenlängen über 18 Kohlenstoffatomen und überwiegend ungesättigt, und Biotensid-Glycolipiden herzustellen. Weiterhin soll die Tensidkombination über einen breiten pH-Bereich anwendbar sein.

Die erfindungsgemässen Zubereitungen sollten vorzugsweise zu einem grösstmöglichen.Umfang auf natürlichen Rohstoffen basieren und biologisch gut abbaubar sein.

**Beschreibung der Erfindung**

Überraschenderweise wurde gefunden, dass binäre Kombinationen von Glycolipid-Biotensiden (B) mit alkoxylierten Fettsäureamide auf Basis von C18-Pflanzenölen (A) wie in den Ansprüchen beschrieben, eine oder mehrere der genannten Aufgaben lösen.

Unerwarteterweise zeigte sich, dass die erfindungsgemässen Zubereitungen eine für den Fachmann in keiner Weise vorhersehbare Reinigungswirkung auf spezifische Verschmutzungen zeigt. Dies ermöglicht die Herstellung von hautmilden Zubereitungen selbst für hartnäckige Verschmutzungen, wie Anfärbungen oder Kohlenhydratverunreinigungen.

Es wurde festgestellt, dass die erfindungsgemässen Zubereitungen für den Fachmann nicht vorhersehbar synergistisch auf das Lösen von Kohlenhydratverschmutzungen wirken. Hierbei wird unter synergistisch verstanden, dass die Reinigungskraft der Mischung höher ist, als die Reinigungskraft der einzelnen Komponenten.

Überraschenderweise wurde auch eine synergistische Wirkung bei Farbverschmutzungen festgestellt. Farbreste unterschiedlicher Art können durch die erfindungsgemässe Zusammensetzung mit nur einem Mittel entfernt werden, was sich beispielsweise in der Verwendung als Makeup-Entferner für fett- und wasserlösliche Kosmetik vorteilhaft erweist.

Weitere Erfindungsgegenstände basieren auf Inhaltstoffen, die mit der erfindungsgemässen Zubereitung kombiniert werden können und erstaunlicherweise die synergistische Wirkung zwischen (A) und (B) nicht beeinträchtigen. Die Synergie bleibt überraschenderweise in vollem Umfang erhalten - dies ist insofern erstaunlich, da die Zugabe einer dritten Komponente die Interaktion (A), (B) und die zu lösende Verunreinigung deutlich- und meist negativ- beeinflusst.

Weiterer Gegenstand der Erfindung ist daher die optionale zusätzliche Zugabe eines alkoxylierten Tensids (C) wie in den Ansprüchen beschrieben. Bei gleichbleibend hoher Reingungskraft auf Kohlenhydrate, Farben und Pigmente verbessert sich zudem die Reinigungsleistung auf spezifische Anfärbungen wie z.B. Makeup sowie die Schaumeigenschaften der Zubereitung.

Eine deutliche Reduktion von Farbverschmutzungen wird durch die erfinderischen Zusammensetzungen ohne zwingende Zugabe zusätzlicher Lösungsmittel beobachtet. Ein weiterer Gegenstand der Erfindung ist die optionale zusätzliche Zugabe von milden Lösungsmitteln, welche die Entfernung bestimmter Farb- und Pigmentverunreinigungen, wie z.B. Lidschatten weiter verbessert. Die synergistische Wirkung zwischen (A) und (B) bleibt erstaunlicherweise erhalten.

Weiterhin ist Gegenstand der Erfindung die optionale zusätzliche Zugabe von Chelatbildnern. Auch hier wird die synergistische Wirkung zwischen (A) und (B) nicht beeinträchtigt, im Gegenteil, die Reinigungsleistung auf Makeup wird verbessert sowie die Schaumbildung und -stabilität verstärkt. Es entstehen klare Formulierungen.

Weiterer Gegenstand dieser Anmeldung ist die nicht-therapeutische Verwendung der erfindungsgemässen Zubereitungen als oder zur Herstellung von Reinigungs- und Pflegemitteln für Körper, Mund und Zähne, Haut und Haare sowie zur Tierpflege.

Zu den Reinigungs- und Pflegemittel zählen im Rahmen der Erfindung auch Hilfsmittel, die bei der Reinigung und Pflege zum eigentlichen Mittel zudosiert werden. Ferner zählen zu Reinigungs- und Pflegemittel im Rahmen der Erfindung Vor- und Nachbehandlungsmittel, also solche Mittel, die vor der eigentlichen Reinigung angewendet werden, beispielsweise zum Anlösen von hartnäckigen Verschmutzungen.

In einem weiteren Erfindungsgegenstand richtet sich die Erfindung auf ein Reinigungs- und Pflegeverfahren umfassend

a) die Bereitstellung eines Reinigungs- und Pflegemittel umfassend eine Zubereitung gemäss den vorhergehenden Erfindungsgegenständen

b) in Kontakt bringen einer biologischen oder natürlichen Oberflächen, z.B. Zähne, Zahnfleisch, Haare, Haut, Fell u.a. mit dem Reinigungs - und Pflegemittel gemäss (a).

Eine besondere Produktform stellen feste Substrate, wie Tücher dar. Diese werden mit einer kosmetischen Zubereitung getränkt und haben den Vorteil, dass in ihnen die Zubereitung bereits in der richtigen Dosierung vorgegeben ist. Dies kommt insbesondere dem Konsumentenwunsch der Convenience entgegen. Sie sind einfach handhabbar, direkt zu verwenden ohne zusätzliche Arbeitsschritte und können auch unterwegs, z.B. auf Reisen gut angewendet werden, selbst wenn kein Wasser zur Verfügung steht.

Tücher werden aus Textilien hergestellt, welche gewebt, gestrickt, oder gewirkt sein können oder als Verbundstoff in Vlies, Papier, Watte oder Filz vorliegen, wobei Vliese meist aus Polypropylen, Polyester oder Viskose hergestellt werden.

Mit kosmetischen Zubereitungen imprägnierte Substrate und Tücher können auf unterschiedliche Weisen hergestellt werden - dem Tauch-, dem Abstreif- und dem Sprühverfahren. Letzteres wird insbesondere für nicht oder schwach schäumende Zubereitungen angewendet.

Vorteilhaft ist, dass die erfindungsgemässen Zubereitungen über den gesamten pH-Bereich eingesetzt werden kann, sowie eine hohe Kompatibilität mit anderen Inhaltsstoffen aufweist - seien es anionische, kationische, amphotere, oder nichtionische Inhaltsstoffe.

Zudem zeigt die Zubereitung eine rückfettende Wirkung verbunden mit einem, angenehmen Hautgefühl.

Die erfindungsgemässe Zubereitung wird auch in einer konservierungsmittelfreien Ausführungsform offenbart.

Ein weiterer Vorteil der Erfindung ist, dass die Zubereitungen auch ohne Sulfattenside hergestellt werden können und somit dem Kosmetiktrend "sulfatfrei" Sorge tragen.

Weiterhin zeigen die Zubereitungen überraschenderweise eine so gute Reinigungsleistung, dass auf die gängigen Tenside Laurylsulfat, Laurylethersulfat und Cocoamidpropylbetain auf Basis Palmkernöl vollständig verzichtet werden kann, welche in höheren Dosen haut- und schleimhautreizend wirken können.

Definitionen:

Technisch unterscheiden sich die Pflanzenöle aus Ölpalmen, Babassu, Palmkernen, oder Kokosnüssen deutlich in der Fettsäurezusammensetzung von den erfindungsgemässen C-18-Pflanzenölen:

In dieser Erfindung werden folgende Pflanzenöle, - fette, -wachse oder -harze als C-18-Pflanzenöl bezeichnet:

Bevorzugt handelt es sich bei den C-18-Pflanzenölen um natürliche Triglyceride. C-18-Pflanzenöle weisen ein Gemisch an gesättigten und ungesättigten Fettsäuren auf, wobei die Fettsäureverteilung von Fettsäuren mit 18 und mehr Kohlenstoffatomen über 60 Gew.-%, besonders bevorzugt über 72 Gew.-% und ganz besonders bevorzugt von über 77 Gew.-% liegt und wobei der Anteil an ungesättigten Fettsäuren über 55 Gew.-%, vorzugsweise über 65 Gew.-% und besonders bevorzugt über 72 Gew.-% liegt.

Bevorzugt liegt der Anteil an Fettsäuren mit 16 und weniger Kohlenstoffatomen unter 30 Gew.-%, bevorzugt unter 27 Gew.-% und besonders bevorzugt unter 17 Gew.-%.

Bevorzugt enthalten die C-18-Pflanzenöle einen Anteil von < 0.5%, besonders bevorzugt > 0.05% Fettsäuren mit 6 Kohlenstoffatomen.

Bevorzugt enthalten die C-18-Pflanzenöle einen Anteil von < 75 Gew-% Hydroxyfettsäuren, bevorzugt < 25 Gew.-%, besonders bevorzugt < 5 Gew.-%.

Bevorzugt enthalten C-18-Pflanzenöle gesättigte oder ungesättigte Fettsäuren mit 20 und mehr Kohlenstoffatomen, wobei deren Gehalt bis zu 96 Gew.-% betragen kann. Bevorzugt enthalten C-18-Pflanzenöle einen Anteil von gesättigten oder ungesättigten Fettsäuren mit 20 und mehr Kohlenstoffatomen von > 0.01 Gew.-% und besonders bevorzugt > 0.05 Gew.-% und ganz besonders bevorzugt > 0.1 Gew-% und äusserst bevorzugt >= 0.2 Gew.-%

Bevorzugt enthalten die C-18-Pflanzenöle einen Anteil von weniger als 95 Gew.-% Ölsäure, besonders bevorzugt unter 85 Gew.-% Ölsäure

Gew.-% hier jeweils bezogen auf den Gesamtgehalt an Fettsäuren im Pflanzenöl.

Aus folgenden Pflanzen bzw. Pflanzenteilen wie bspw. Samen, Kerne, Fruchtfleisch, Blätter, Wurzeln und andere, im folgenden C-18-Pflanzen genannt, können C-18 Pflanzenöle gewonnen werden: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kreuzblütengewächse, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam,

Sesamblatt, Senf, Sonnenblume, Soja, Tabak, Walnuss, Weintraube, Weizen, Wiesenschaumkraut und Wildrose; sowie deren Kombinationen.

Vorzugsweise ist das Öl ausgewählt aus der Gruppe: Aprikose, Avocado, Baumwolle, Brokkoli, Buche, Distel, Dinkel, Erdmandel, Gerste, Hanf, Haselnuss, Jojoba, Kirsche, Königskerze, Krambe, Kreuzblättrige Wolfsmilch, Kürbis, Iberischer Drachenkopf, Leindotter, Leinsamen, Lupine, Luzerne, Macademia, Mandel, Mais, Mohn, Nachtkerze, Olive, Ölrettich, Ölrauke, Pfirsich, Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube und Weizen, sowie deren Kombinationen.

Ganz besonders bevorzugt ist das Öl ausgewählt aus der Gruppe Aprikose, Distel, Erdmandel, Hanf, Krambe, Iberischer Drachenkopf, Leindotter, Leinsamen, Lupine, Luzerne, Mais, Mandel, Olive, Ölrettich, Pfirsich, Raps, Rübsen, Sesam, Sesamblatt, Sonnenblume, Soja, Weintraube und Weizen, sowie deren Kombinationen.

Der Begriff Öle wird in dieser Erfindung stellvertretend für Fette, Wachse und Harze verwendet.

Unter Tensid werden im Zusammenhang dieser Erfindung amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Micellen, lamellare Strukturen u.a. Im Zusammenhang dieser Erfindung werden Emulgatoren unter den Begriff der Tenside gefasst, jedoch nicht umgekehrt.

Im Rahmen der vorliegenden Erfindung steht - soweit nicht anders angegeben- auf Basis von oder abgeleitet von Pflanzenölen, -fetten oder -wachsen stellvertretend für Derivate aus Fettsäuren - gereinigt oder als Gemisch - und/oder deren Reaktionsprodukte, wie beispielsweise Additionsprodukte an die Doppelbindung, Reaktionen an der Fettsäurefunktion, wie z.B. Fettalkohole und deren Ether und/ oder Carboxylether, Amine oder Fettsäureamide, Fettsäureester, sowie Imine. Bevorzugt liegen diese Fettsäurederivate als Mischung gemäss der Fettsäurenverteilung im nativen Öl vor oder wie sie bei der Umsetzung von natürlich vorkommenden Pflanzenölen oder -fetten anfallen.

Im Rahmen der vorliegenden Erfindung stehen Fettsäuren bzw. Fettalkohol bzw. deren Derivate soweit nicht anders angegeben - stellvertretend für verzweigte oder unverzweigte, gesättigte, einfach oder mehrfach ungesättigte Carbonsäuren bzw. Alkohole bzw. deren Derivaten mit vorzugsweise 6 bis 24 Kohlenstoffatomen.

PEGylierte Pflanzenöle sind ethoxylierte Pflanzenöle wie definiert in "Safety Assessment of PEGylated Oils as Used in Cosmetics", International Journal of Toxicology November/December 2014, 33. Im Rahmen dieser Erfindung wird die Terminologie verwendet, welche bei kosmetischen Inhaltsstoffen Anwendung findet, welche die Veretherungs- und Veresterungsprodukte von Glyceriden und Fettsäuren mit Ethylenoxid beschreibt. Im Rahmen dieser Erfindung sind insbesondere Vertreter der C-18-Pflanzen bevorzugt; Beispiele sind unter den Tensiden (C) gelistet.

PEGylierten Fettsäureglyceride sind Mono-, Di- und/oder Triglyceride, welche mit einer spezifischen Anzahl an Alkylenglycol-Einheiten, meist Ethylenglycoleinheiten modifiziert wurden und Nebenprodukte der Reaktion enthalten können. Im Rahmen dieser Erfindung werden PEGylierten Fettsäureglyceride definiert wie in "Safety Assessment of PEGylated Alkyl Glycerides as Used in Cosmetics", Cosmetic Ingredient Review (CIR) 2014. Zu bemerken ist, dass CIR unter "Alkyl" auch ungesättigte Fettsäuren berücksichtigt.

Im Rahmen dieser Erfindung sind insbesondere alkoxylierte Fettsäureglyceride (PEGyliert und andere) der C-18-Pflanzen bevorzugt; Beispiele alkoxylierten Fettsäureglyceridester der C-18-Pflanzen sind unter den Tensiden (C) gelistet.

Im Rahmen dieser Anmeldung wird unter "Schwefeltenside" anionische oder amphotere Tenside mit einem schwefelhaltigen hydrophilen Rest verstanden wie z.B. Alkylsulfate, Alkylethersulfate, (alkoxylierte) Sulfosuccinate, (alkoxylierte) Sulfonate, (alkoxylierte) Isethionate, (alkoxylierte) Taurate, Sulfobetaine und Sultaine. Beispiele für sulfathaltige Tenside stellen Sodium Laureth Sulfate, Sodium Lauryl Sulfate, Ammonium Laureth Sulfate, Ammonium Lauryl Sulfate, Sodium Myreth Sulfate, Sodium Coco Sulfate, Sodium Trideceth Sulfate oder MIPA-Laureth Sulfate dar.

Im Rahmen dieser Erfindung steht soweit nicht anders vermerkt Biotensid für die erfindungsgemäss definierten Biotensid-Glycolipide.

Frei von Schwefeltensiden, Phosphaten, Phosphonaten bedeutet, dass die Zubereitung keine nennenswerten Mengen an Schwefeltensiden, Phosphaten, Phosphonaten aufweisen. Insbesondere ist hierunter zu verstehen, dass Schwefeltenside, Phosphate, Phosphonate jeweils in Mengen von kleiner 0.1 Gew.-%, bevorzugt von kleiner 0.01 Gew.-% bezogen auf die Gesamtmenge der Zubereitung, insbesondere keine nachweisbaren Mengen, enthalten sind.

"Mindestens ein" wie hierin verwendet, bezieht sich auf 1 oder mehr, beispielsweise 1, 2, 3, 4, 5, 6, 7, 8, 9 oder mehr.

Unter "Reinigungs- und Pflegemittel" wird im Rahmen der Erfindung eine kosmetische oder nicht-therapeutische medizinische Zubereitung zur Pflege von Haut und Haar, Zähne, Zahnfleisch, Nägel sowie zur Entfernung unerwünschter Verunreinigungen, z.B. Make-up, externe Verschmutzungen, Farbe, Erde, u.a., Stoffwechselprodukten von biologischen Vorgängen, z.B. Schweiss, Hautfett u.a. oder Gerüchen verstanden.

Die Zubereitungen können durch einreiben, zudosieren, sprayen, schäumen und andere Methoden (z.B. salben, auflegen, etc.) direkt oder über ein Hilfsmittel, verdünnt oder konzentriert, auf den zu behandelnden Stellen aufgebracht werden. Das Mittel kann reinigen, die Attraktivität einer Person erhöhen, die Gesundheit von Haut und Haar erhalten,

inklusive der Rasur, verschönern und pflegen. In den Begriff eingeschlossen ist die Tierpflege und -reinigung.

Unter "Reinigungsleistung" oder "Waschkraft" wird im Rahmen dieser Erfindung die Entfernung von einer oder mehreren Verschmutzungen, Stoffwechselprodukten oder Gerüchen verstanden.

Die Entfernung kann über eine Aufhellung oder Verringerung der Anschmutzung messtechnisch erfasst, oleofaktorisch oder visuell beurteilt werden.

Der HLB (hydrophile-lipophile balance) Wert ist ein Mass für die Hydrophilie, bzw. Lipophilie eines Stoffes, in der Regel eines nichtionischen Tensids. Der Wert kann theoretisch wie in einschlägiger Literatur beschrieben (z.B. nach der Griffin-Methode) oder experimentell durch den Vergleich des Löslichkeitsverhaltens von Standardzusammensetzungen mit bekanntem HLB gemessen werden.

Stoffe, die als Inhaltsstoffe von kosmetischen Mitteln dienen, werden nachfolgend gegebenenfalls gemäss der International Nomenclature Cosmetic Ingredient- (INCI-) Nomenklatur bezeichnet. Die INCI-Bezeichnungen sind dem "International Cosmetic Ingredient Dictionary and Handbook, 13th Edition (2010)" zu entnehmen. Herausgeber: The Personal Care Products Council.

Soweit nicht explizit anders angegeben, beziehen sich die angegeben Menge in Gewichtsprozent (Gew.-%) auf die gesamte Zubereitung. Dabei beziehen sich die prozentualen Mengenangaben auf Aktivgehalte.

Ein erster Gegenstand der Erfindung richtet sich auf eine Zubereitung enthaltend mindestens ein alkoxyliertes Tensid (A) aus der Gruppe der alkoxylierten Fettsäureamide (I) und mindestens ein Glycolipid-Biotensid (B) umfassend Rhamnolipide, Sophorolipide, Mannosylerythritollipide, Cellobiose Lipide und Trehaloselipde.

**Tensid (A)**

Alkoxylierte Fettsäureamide

Für die Erfindung geeignete alkoxylierte Fettsäureamide folgen der Formel (I),

(I) $R\text{- }CO\text{-}NH\text{-}(C_mH_{2m}O)_n\text{ -}H$

wobei

m die ganze Zahlen 2 oder 3 ist, bevorzugt 2,

n Zahl im Bereich von 2-10, bevorzugt im Bereich 2-8, ganz besonders bevorzugt 2-4,

mit R = gesättigte, ein- oder mehrfach ungesättigter Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt;

und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (I);

und wobei das Tensid (I) aus einem Gemisch unterschiedlicher Kettenlängen und Sättigungsgrade des Fettsäurerests RCO wie oben definiert besteht und abgeleitet ist von einem C-18-Pflanzenöl aus der Gruppe umfassend: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Moringa, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose; sowie deren Kombinationen;

und wobei bevorzugt der Anteil an Fettsäureresten RCO von 20 oder mehr Kohlenstoffatomen > 0.01 Gew.-%, besonders bevorzugt > 0.05 Gew.-% und ganz besonders bevorzugt > 0.1 Gew.-% und äusserst bevorzugt ≥ 0.2 Gew.-% beträgt;

und wobei bevorzugt der Anteil an Fettsäureresten RCO mit Fettsäuren von 16 und weniger Kohlenstoffatomen

unter 30 Gew.-%, bevorzugt unter 27 Gew.% und besonders bevorzugt unter 17 Gew.% liegt;

und wobei bevorzugt der Anteil an Fettsäureresten RCO mit Fettsäuren von 6 und weniger Kohlenstoffatomen < 0.5 Gew.-%, besonders bevorzugt < 0.05 Gew.-% liegt;

und wobei bevorzugt der Anteil an Fettsäureresten RCO mit Hydroxyfettsäuren < 75 Gew-%, bevorzugt < 25 Gew.-%, besonders bevorzugt < 5 Gew.-% liegt;

und wobei bevorzugt der Anteil an Fettsäureresten RCO des Ölsäureacylrestes RCO bei weniger als 95 Gew.-%, besonders bevorzugt unter 85 Gew.-% liegt;

jeweils bezogen auf den Gesamtanteil an Fettsäureresten RCO des eingesetzten Tensids (I).

Besonders bevorzugt sind Fettsäureamide der Formel (I) abgeleitet von Distel, Erdmandel, Hanf, Krambe, Iberischer Drachenkopf, Leindotter, Leinsamen, Lupine, Luzerne, Mais, Olive, Ölrettich, Raps, Rübsen, Sesamblatt, Sonnenblume, Soja, Weintraube und Weizen, sowie deren Kombinationen.

Besonders bevorzugt sind Fettsäureamide der Formel (I) mit einem HLB > 10.5 und <12.0.

Erfindungsgemäss äusserst bevorzugt ist das ethoxylierte Fettsäureamid auf Basis Rapsöl, IUPAC Name: Amides, rape oil, *N*-(hydroxyethyl), ethoxylated; INCI Name: PEG-4 Rapssamenamid, oder Rübsamenamid bzw. PEG-4 Rapeseedamide. Handelsname: Amidet® N der Firma Kao.

Im Vergleich zu dem meist verwendeten Tensid Natrium Laureth Sulfat, weist PEG-4 Rapssamenamid eine niedrigere akute orale Toxizität (Ratte > 2,000 mg/kg),eine vergleichbar niedrige akute dermale Toxizität (Ratte > 2,000 mg/kg), eine vergleichbare Hautirritation (Cat.2), und ein deutlich niedrigeres Augenirritationspotential auf und eignet sich damit hervorragend für die erfindungsgemässen Verwendungen.

Verwendung von Fettsäuregemischen

Im Sinne dieser Anmeldung liegt dem alkoxylierten Tensid (A) eine Mischung von Fettsäurederivaten auf Basis von C18-Pflanzenölen mit unterschiedlicher Kettenlänge und Sättigungsgrad zugrunde. Die Mischung folgt bevorzugt der Fettsäureverteilung im nativen Öl oder wie sie bei der Umsetzung von natürlich vorkommenden Pflanzenöle oder Fette anfallen. Indem für die Synthese der-Tensidklasse Fettsäureamidgemische verwendet werden - wie sie bei der Umsetzung von natürlich vorkommenden Pflanzenöle oder Fette anfallen - können die Tenside kostengünstig, ressourceneffizient und umweltschonend produziert werden. Zusätzliche Reinigungsverfahren, wie z.B. die Trennung der Fettsäuren, bzw. Fettsäureester oder -amide durch fraktionierte Destillation oder zusätzliche Syntheseschritte, wie z.B. zum Fettalkohol, werden hier nicht benötigt. Neben den ökologischen und ökomischen Vorteilen der Verwendung von natürlichen Fettsäuregemischen, zeigen die verwendeten Tensidmischungen eine erhöhte Reinigungsleistung.

Die Zubereitungen enthalten bevorzugt 0.1 bis 50 Gew.-% eines oder mehrerer alkoxylierter Tenside (A), bevorzugter 0.1 bis 25 Gew-%, besonders bevorzugt 0.1 bis 10 Gew.-% und ganz besonders bevorzugt 0.25 Gew.-% bis 5 Gew.-%. Gew.-% bezogen auf die Gesamtmenge der Zubereitung.

**Biotenside allgemein**

Als Biotenside werden allgemein Tenside biologischen Ursprungs bezeichnet. Sie erfahren keine chemische Umsetzung wie die synthetischen Glycolipide z.B. Alkylpolyglycoside (APG). Biotenside können in lebenden Organismen vorkommen, oder entstehen bei der Kultivierung diverser Mikroorganismen wie z.B. Pilze, Hefen, Viren, Bakterien oder Enzyme.

Glycolipid-Biotenside können unmittelbar durch die Verwendung von natürlichen Rohstoffen durch mikrobiologische Prozesse gewonnen werden, während die Herstellung von synthetischen Glycolipiden meist weitere chemische Schritte, wie bspw. die Reduktion der Fettsäure zum Fettalkohol bedingt. Durch die biologische Modifizierung des Zuckerteils weisen Glycolipid-Biotenside spezielle Eigenschaften auf, wie zum Beispiel dem Fachmann bekannt, eine gute Hautverträglichkeit und eine sehr geringe Toxizität, womit sie für die erfindungsgemässen Verwendungen bevorzugt geeignet sind. Die Struktur der Glycolipid-Biotenside sowie die Kettenlängen des hydrophoben Teils variiert je nach verwendetem Mikroorganismus bzw. Substrat.

Im Rahmen dieser Erfindung umfassen Glycolipid-Biotenside (B) Rhamnolipide, insbesondere Mono-, Di- oder Polyrhamnolipide, Sophorolipide in ihrer Säure- oder Laktonform oder als deren Gemische, diacetyliert, acetyliert oder nicht-acetyliert, Trehaloselipiden, Mannosylerythritollipide und Cellobioselipide.

Als Substrate für Glycolipid-Biotenside eignen sich die unterschiedlichsten, literaturbekannten Kohlenstoffquellen, wie z.B. Pflanzenöle und den daraus erhaltenen Glyceriden oder Fettsäuremethylestern, Fettsäuren, Fettalkohole,

Fettsäuremethylester oder -ethylester, Kohlenhydrate, z.B. Cellulose, Glucose, Stärke, C4-Quellen wie Succinate, Butan, Buttersäure, C1-Quellen wie $CO_2$, CO oder Methan, öl- und/oder kohlenhydrathaltige Abwässer aus Raffinerien oder der Lebensmittelindustrie und auch Mischungen derselben.

Biotensid Mikroorganismen

Bevorzugte Mikroorganismen zur Herstellung der Biotenside sind Bacillus, Candida, Pseudomonas, Trichosporan und/oder Pseudozyma Stämme und weitere wie in einschlägiger Literatur beschrieben, insbesondere Arthrobacter spec., Bacillus licheniformis, Bacillus subtilis, Burkholderia, Candida apicola, Candida antarctica, Candida batistae, Candida bogoriensis, Candida bombicola, Candida sp., Cryptococcus humicola, Gordonia, Mycobacterium tuberculosis, Nocardia cornynebacterium, Pichia anomala, Pseudomonas aeruginosa, Pseudozyma aphidis (MEL), Pseudomonas sp, Pseudozyma antarctica (MEL), Pseudozyma parantarctica, Pseudozyma fusiformata, Rhodococcus, Rhodococcus erythropolis, Rhodotorula bogoriensis, Sphingomonas sp. NM05, Starmerella bombicola, Trichosporon cutaneum, Trichosporan loubieri; Torulopsis magnoliae, Torulopsis bombicola, Tsukamurella spec., Yarrowia alipolytica, Yarrowia lipolytica, Ustilago maydis (MEL), Wickerhamiella domercqiae Y2A.

Die Glycolipid-Biotenside können z. B. wie in EP 0 499 434, US 7,985 722, WO 03/006146, JP 60-183032, DE 19648439, DE 19600743, JP 01-304034, CN 1337439, JP 2006- 274233, KR 2004033376, JP 2006-083238, JP 2006-070231 , WO 03/002700, FR 2740779, DE 2939519, US 7,556,654, FR 2855752, EP 1445302, JP 2008-062179 und JP 2007-181789, Mannosylerythritol: WO 2004/020647, JP 20042544595, oder den dort zitierten Schriften hergestellt werden.

**Biotenside Strukturen**

Rhamnolipide

Unter dem Begriff Rhamnolipid im Zusammenhang mit der vorliegenden Erfindung werden insbesondere Verbindungen der allgemeinen Formel (II) oder deren Salze verstanden,

(II)

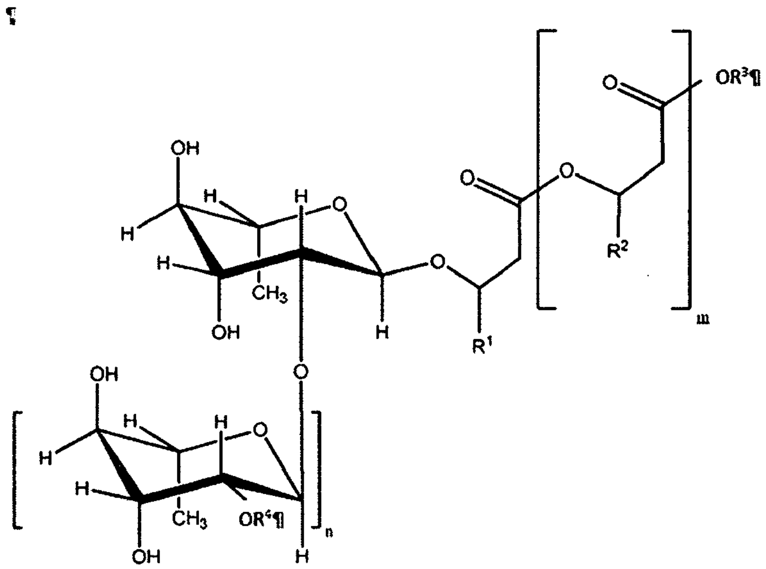

wobei

- m = 2, 1 oder 0,
- n = 1 oder 0,
- $R^1$ und $R^2$ = unabhängig voneinander gleicher oder verschiedener organischer Rest mit 2 bis 24 Kohlenstoffatomen, insbesondere gesättigter, ein- oder mehrfach ungesättigter, linear oder verzweigter, gegebenenfalls substituierter, insbesondere hydroxy-substituierter Kohlenwasserstoffrest, bevorzugt mit 5 bis 13 Kohlenstoffatomen, bevorzugt ausgewählt aus der Gruppe bestehend aus Pentenyl, Heptenyl, Nonenyl, Undecenyl und Tridecenyl und $-(CH_2)_o-CH_3$ mit o = 1 bis 23, besonders bevorzugt 4 bis 12, äusserst bevorzugt o= 6.
- $R^3$ - H, $-CH_3$, oder Kation, insbesondere Alkalikation, bevorzugt H

- $R^4$ - H oder die Gruppe $CH_3(CH_2)_aCH=CH-CO-$, mit a Zahlen zwischen 4 und 10, bevorzugt H.

Unter dem Begriff "di-Rhamnolipid" im Zusammenhang mit der vorliegenden Erfindung werden Verbindungen der allgemeinen Formel (II) oder deren Salze verstanden, bei denen n =1 ist.

Unter dem Begriff "mono-Rhamnolipid" im Zusammenhang mit der vorliegenden Erfindung werden Verbindungen der allgemeinen Formel (II) oder deren Salze verstanden, bei denen n =0 ist.

Rhamnolipide sind über Organismen wie Pseudomonas und Burkholderia, beides Wildtypen zugänglich. Desweiteren werden sie über rekombinante Zellen hergestellt. Kommerziell erhältlich sind Rhamnolipide bei der Firma Evonik unter dem Handelsnamen Rewoferm®, The Gene Biotech oder bei Jeneil Biosurfactant Co.LLC.

Besonders bevorzugt in dieser Erfindung sind die Rhamnolipide der Firma Evonik Rewoferm® und JBR 425 von Jeneil Biosurfactant, insbesondere JBR 425.

Sophorolipide

Unter dem Begriff Sophorolipid im Zusammenhang mit der vorliegenden Erfindung werden insbesondere Verbindungen der offenen Form oder Säureform, der Ring- oder Laktonform, oder deren Gemische verstanden, vorzugsweise Formel (III) folgend

(III)

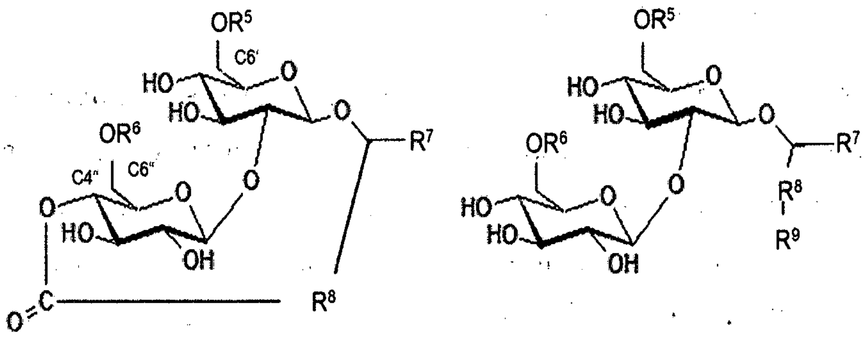

Ringform

Offene Form

wobei

- $R^5$ und $R^6$ unabhängig voneinander H oder Acetylgruppen sind
- $R^7$ ist H oder reine gesättigte oder ungesättigte, hydroxylierte oder nichthydroxylierte Kohlenwasserstoffkette mit 1-9 Kohlenstoffatomen, bevorzugt H oder $-CH_3$
- $R^8$ ist eine gesättigte oder ungesättigte, hydroxylierte oder nichthydroxylierte, lineare oder verzweigte Kohlenwasserstoffkette mit 1-22 Kohlenstoffatomen, bevorzugt besteht $R^8$ aus einer gesättigten Kohlenwasserstoffkette aus 11 bis 22 Kohlenstoffatomen oder aus einer einfach oder zweifach ungesättigten Kohlenwasserstoffkette aus 13 bis 22 Kohlenstoffatomen, besonders bevorzugt aus einer einfachen oder zweifach ungesättigten Kohlenwasserstoffkette aus 15 oder 16 Kohlenstoffatomen stammend aus Rapsöl als Substrat.
- $R^9$ ist -COOH oder ein kationisches Salz desselben, oder $-COO(CH_2)_mCH_3$ mit m zwischen 0 und 3,

In der Laktonkonfiguration kann die Laktonbildung der Carboxylgruppe mit Hydroxylgruppe an C4", wie hier exemplarisch in Formel (III) dargestellt, oder alternativ an C6" oder C6' erfolgen, an der C4" Position befindet sich dann eine Hydroxylgruppe.

Zusätzlich können die Sophorolipide Verunreinigungen enthalten, wie Fettalkohole, Fettsäuren, Fettsäureester, Triglyceride oder Öle, Zucker, besonders Glucose, Sophorose, oder organische Säuren.

Erfindungsgemäss können unterschiedlichste Substrate verwendet werden, wie in C. Mulligan, Biosurfactants: Research Trends and Application, CRC Press 2014, S. 112. Beispiele für bevorzugten Substrate für Sophorolipide aus der Literatur sind: Maisöl (EP 0282942), Öl von C22 Fettsäuren (KR20100022289), Olivenöl (ES 2018637, CN 1431312), Rapsöl oder -ester (CN 102250790, US2008032383), Sonnenblumen- oder Rapsfettsäureester (DE4319540, FR

2692593) oder Abfallöle bzw. -fette (JP 2004254595, JP 2007252279, CN 101845468, CN 101948786).

Sophorolipide sind durch eine Vielzahl unterschiedlicher dem Fachmann bekannten Organismen wie Candida bombicola, Starmerella oder Wickerhamiella zugänglich.

Weiterhin sind sie kommerziell erhältlich, z.B. von der Firma Evonik z.B. unter dem Handelsnamen Rewoferm SL 446 oder von Soliance/Givaudan.

Besonders bevorzugt in dieser Erfindung sind die Sophorolipide mit den Handelsnamen Sophogreen, Sophoclean, Soliance S.

Mannosylerythritollipide

Unter dem Begriff Mannosylerythritollipide im Zusammenhang mit der vorliegenden Erfindung werden insbesondere Verbindungen der allgemeinen Formel (IV) oder deren Salze verstanden,

(IV)

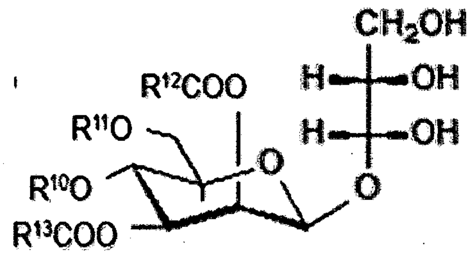

wobei

$R^{10}$ und $R^{11}$ unabhängig voneinander H oder $-COCH_3$,

$R^{12}$ und $R^{13}$ unabhängig voneinander, lineare oder verzweigte $C_1$ bis $C_{23}$, vorzugsweise $C_1$ bis $C_{17}$, und besonders bevorzugt $C_7$ bis $C_{15}$Alkylgruppen; oder lineare oder verzweigte $C_2$ bis $C_{23}$, bevorzugt $C_2$ bis $C_{17}$, und besonders bevorzugt $C_7$ bis $C_{15}$Alkenylgruppen; oder lineare oder verzweigte $C_5$ bis $C_{23}$, vorzugsweise $C_5$ bis $C_{17}$, und besonders bevorzugt $C_7$ bis $C_{15}$ Alkadienylgruppen; oder lineare oder verzweigte $C_8$ bis $C_{19}$, bevorzugt $C_8$ bis $C_{17}$, und besonders bevorzugt $C_8$ bis $C_{15}$ Alkatrienylgruppen.

Mannosylerythritollipide können durch Pseudozyma Antarctica NBRC 10736 in einem Nährmedium mit Sojaöl erhalten werden, alternativ sind sie durch den Brandpilz Ustilago maydis oder Candida spec. bzw. Antarctica zugänglich.

Cellobiose Lipid

Unter dem Begriff Cellobioselipid im Zusammenhang mit der vorliegenden Erfindung werden insbesondere Verbindungen der allgemeinen Formel (V) oder deren Salze verstanden

(V)

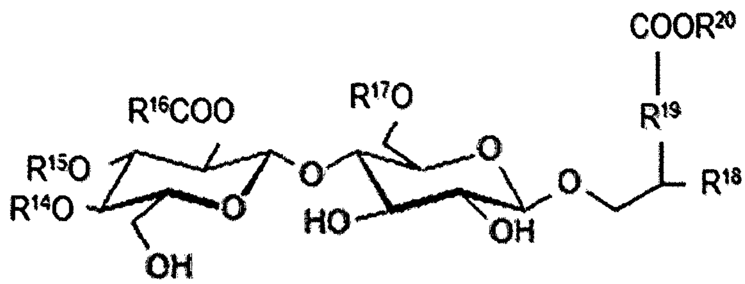

$R^{14}$, $R^{15}$ und $R^{17}$= unabhängig voneinander H oder $-COCH_3$,

$R^{16}$ = gesättigter oder ungesättigter, hydroxylierter oder nicht-hydroxylierter Kohlenwasserstoff mit 5 bis 23 Kohlenstoffatomen, oder $-CH_3$, bevorzugt $- CH_3$ oder $- CH_2-CH(OH)-(CH_2)_n-CH_3$ mit n= 2-4,

$R^{18}$= H oder -OH,

$R^{19}$= gesättigter oder ungesättigter, hydroxylierter oder nicht-hydroxylierter Kohlenwasserstoff mit 9 bis 21 Kohlenstoffatomen, bevorzugt 13 Kohlenstoffatome, besonders bevorzugt - $(CH_2)_n$-CH(OH)- mit n= 12,

$R^{20}$= H, oder ein Kation,

Cellobioselipide können durch literaturbeschriebe Verfahren durch Crypotcoccus humicola, Pseudozyma fusiformata oder durch den Brandpilz Ustilago maydis erhalten werden.

Trehaloselipide

Unter dem Begriff Trehaloselipide im Zusammenhang mit der vorliegenden Erfindung werden insbesondere Verbindungen der allgemeinen Formel (VI) oder deren Salze verstanden.

(VI)

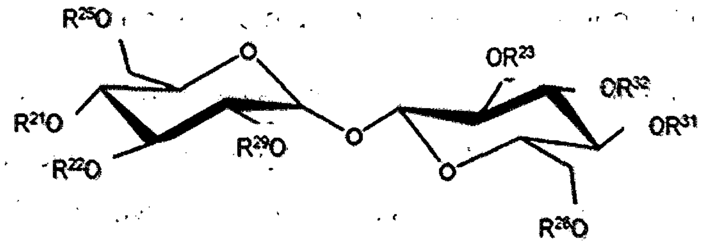

$R^{21}$, $R^{22}$, $R^{31}$ und $R^{32}$= jeweils unabhängig voneinander Wasserstoff oder -COR $^{27}$ mit $R^{27}$ gesättigtem oder ungesättigtem, hydroxyliertem oder nicht-hydroxyliertem Kohlenwasserstoff mit 5 bis 23 Kohlenstoffatomen;

$R^{25}$ und $R^{26}$ jeweils unabhängig voneinander Wasserstoff oder -$COR^{28}$ mit $R^{28}$ gesättigtem oder ungesättigtem, verzweigt oder nicht-verzweigtem, hydroxyliertem oder nicht-hydroxyliertem Kohlenwasserstoff mit 5 bis 23 Kohlenstoffatomen oder $R^{28}$ = $-CH[(CH_2)_cCH_3]CHOH(CH_2)_dCH_3$ mit c+d = 27;

$R^{29}$ und $R^{23}$ jeweils unabhängig voneinander Wasserstoff, -$COR^{30}$ mit $R^{30}$ gesättigtem oder ungesättigtem, verzweigt oder nicht-verzweigtem, hydroxyliertem oder nicht-hydroxyliertem Kohlenwasserstoff mit 5 bis 23 Kohlenstoffatomen oder $R^{30}$ = $-(CH)_2COOR^{24}$ mit $R^{24}$= H oder Kation.

Erfindungsgemäss eignen sich vorzugsweise Tetralose Dimycolate, Tetralose Monomycolate, Succinyl Trehalose Lipide und Trehalose Tetraester oder Gemische derselben.

Trehaloselipide können durch literaturbeschriebene Verfahren durch Rhodococcus erythropolis, Arthobacter spec., Gordonia, Mycobacterium tuberculosis, Nocardia Cornynebacterium spec. oder Tsukamurella spec. erhalten werden.

Die Zubereitungen enthalten bevorzugt 0.1 bis 50 Gew.-% eines oder mehrerer Glycolipid-Biotenside, bevorzugter 0.1 bis 25 Gew.-%, besonders bevorzugt 0.1 bis 10 Gew.-% und ganz besonders bevorzugt 0.3 bis 8 Gew.-%, bezogen auf die Gesamtmenge der Zubereitung.

Verhältnis Tensid (A): Biotensid-Glycolipid (B)

Das oder die alkoxylierten Tenside (A) und das oder die Glycolipid-Biotenside (B) können in jedem beliebigen Verhältnis kombiniert werden.

Bevorzugt liegen die Tenside (A) zu den Glycolipid-Biotensiden (B) in einem Verhältnis zwischen (A) :(B) ≤ 20:1 und ≥1:20 vor; beispielsweise 20:1, 9:1, 8:1 u.s.w. Besonders bevorzugt liegen die Tenside (A) zu den Glycolipid-Biotensiden (B) in einem Verhältnis zwischen (A) :(B) ≤ 10:1 und ≥1:10 vor und ganz besonders bevorzugt liegen die Tenside (A) zu den Glycolipid-Biotensiden (B) in einem Verhältnis zwischen (A) :(B) ≤ 2:1 und ≥1:6 vor, äusserst bevorzugt liegt (A) :(B) ≤ 1:1 und ≥1:6; jeweils bezogen auf Gewichtsprozent Aktivgehalt im gesamten Mittel.

## Zusätzliches Tensid (C)

Ein weiterer Gegenstand der Erfindung richtet sich auf eine Zubereitung zusätzlich enthaltend mindestens ein alkoxyliertes Tensid (C). In dieser weiteren Ausführungsform enthält die Zubereitung zusätzlich ein oder mehrere alkoxylierte Tenside (C), ausgewählt aus der Gruppe der alkoxylierten Fettsäureester, der alkoxylierten Fettsäureglyceridester, oder der alkoxylierten Pflanzenölester vorzugsweise der Formel (VIII) folgend,

(VIII) $$RCO\text{-}O(C_mH_{2m}O)_o\text{-}\{CH_2\text{-}CH[O(C_mH_{2m}O)_pR'']\text{-}CH_2O\}_w\text{-}(C_mH_{2m}O)_q\text{-}R'''$$

mit R und RCO definiert wie in Formel (I), d.h. gesättigte, ein- oder mehrfach ungesättigter Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt;

und wobei der Anteil an ungesättigten Fettsäureresten RCO über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (C);

und wobei das Tensid (C) aus einem Gemisch unterschiedlicher Kettenlängen und Sättigungsgrade des Fettsäurerests RCO wie oben definiert besteht und abgeleitet ist von einem C-18-Pflanzenöl.

m ist 2, 3 oder 4, bevorzugt 2 oder 3, besonders bevorzugt 2,
o, p, q sind unabhängig voneinander Zahlen von 0 bis 75, bevorzugt 0-10, bevorzugter 0-8, wobei $o+p+q \neq 0$. Die Zahlen o, p, q repräsentieren den Alkoxylierungsgrad. Obwohl diese Zahlen auf molekularer Ebene nur ganze Zahlen einschliesslich der Null annehmen können, kann der Gesamtalkoxylierungsgrad x als Dezimalzahl angegeben werden, da dieser die stöchiometrischen Äquivalente von Alkylenoxid darstellt,
R" ist H, oder COR,
R'" ist H, COR, oder ein linearer oder verzweigter Alkylrest mit 1-8 C-Atomen.
w ist eine ganze Zahl zwischen 0 und 10;

Überraschenderweise zeigen die Kombinationen (A), (B) und (C) eine unverändert hohe oder sogar bessere Reinigungskraft auf Kohlenhydratflecken oder Farbstoff- und Pigmentflecken.

Dies ist insofern erstaunlich, da die Kombination (A)/(B) auch mit einem dritten Tensid (C) synergistisch auf Kohlenhydratflecken oder Farbstoff- und Pigmentflecken wirkt. Zudem wurde eine noch bessere Reinigungskraft, welche für den Fachmann nicht vorhersehbar ist, bei der Entfernung von Farbstoffen und Pigmenten von Makeup auf Haut festgestellt.

Ein weiterer Vorteil der Kombinationen (A), (B) und (C) ist eine stärkere Schaumbildung als bei der reinen Kombination (A) und (B), insbesondere überraschend war, dass der Schaum zudem deutlich länger stabil bleibt. Die schäumende Ausführungsform ist für einige Verwendungen wünschenswert und ist in den Ausführungsbeispielen exemplarisch dargestellt.

Überraschenderweise können die Mittel bestehend aus dem oder den Tensiden (A) und dem oder den Glycolipid-Biotensiden (B) bei beliebigem pH-Wert zusätzlich mit einem Tensid (C) kombiniert werden.

Beispielhafte Vertreter der erfindungsgemäss geeigneten Tenside (C) sind PEG-6 Mandelöl, PEG-8 Mandelöl, PEG-8 Aprikosenkernöl, PEG-8 Buxus Chinensis Oil, PEG-6 Aprikosenkernöl, PEG-40 Aprikosenkernöl, PEG-8 Arganöl, PEG-8 Avocadoöl, PEG-11 Avocadoöl, PEG-8 Borretschsamenöl, PEG-8 Macademia Tenuifolia Öl, PEG-6 Maisöl, PEG-8 Maisöl, PEG-8 Traubenkernöl, PEG-8 Haselnussöl, PEG-8 Leinsamenöl, PEG-6 Olivenöl, PEG-7 Olivenöl, PEG-7 Olivenöl, PEG-8 Olea Europaea Öl, PEG-7 Olivenöl, PEG-7 Olivenöl, PEG-8 Olivenöl, PEG-10 Olivenöl, PEG-8 Oryza Sativa Öl, PEG-8 Prunus Dulcis, PEG-8 Persea Gratissma Öl, PEG-8 Passiflora edulis seed oil, PEG-6 Erdnussöl, PEG-45 Crambe Absyssinica Seed oil, PEG-75 Wiesenschaumkrautöl, PEG-8 Kürbiskernöl, PEG-3 Rapssamenöl, PEG-20 Rapssamenöl, PEG-8 Diestelöl, PEG-8 Schinziophyton Rautaneii Kernöl, PEG-8 Sesamsamenöl, PEG-8 Senum Indicum Öl, PEG-8 Sojabohnenöl, PEG-20 Sojabohnenöl, PEG-36 Sojabohnenöl, PEG-8 Sonnenblumenöl, PEG-32 Sonnenblumenöl, PEG-8 Süssmandelöl, PEG-8 Wassermelonenkernöl, PEG-8 Weizenkeimöl, PEG-8 Zea Mais Öl

PEG-6 Mandelglycerid, Mandelöl Glycereth-8 Ester, PEG-20 Mandelglycerid, PEG-35 Mandelglycerid, PEG-60 Mandelglycerid, Avocadoöl Glycereth-8 Ester, PEG-11 Avocadoglycerid, Arganöl Glycereth-8 Ester, Mandelöl Glycereth-8 Ester, PEG-14 Mandelglycerid, Maisöl Glycereth-8 Ester, PEG-20 Maisglycerid, PEG-60 Maisglycerid, PEG-20 Nachtkerzenglycerid, PEG-60 Nachtkerzenglycerid, Traubenkernöl Glycereth-8 Ester, Cannabis Sativa Kernöl Glycereth-8 Ester, Jojobaöl Glycereth-8 Ester, PEG-16 Macademiaglycerid, PEG-25 Moringaglycerid, PEG-2 Olivenglycerid, PEG-6 Olivenglycerid, PEG-7 Olivenglycerid, Olivenöl Glycereth-8 Ester, PEG-10 Olivenglycerid, PEG-40 Olivenglycerid, Pfirsichkernöl Glycereth-8 Ester, PEG-60 Passiflora edulis seed glycerid, PEG-60 Passiflora Incarnata seed glycerid, PEG-40 Diestelglycerid, Sojaöl Glycereth-8 Ester, PEG-35 Soja glycerid, PEG-75 Soja glycerid Ester, PEG-2 Sonnenblumenglycerid, PEG-7 Sonnenblumenglycerid, Sonnenblumenöl Glycereth-8 Ester, PEG-10 Sonnenblumenglycerid, PEG-13 Sonnenblumenglycerid, PEG-7 Rapsglycerid, PEG-4 Rapsglycerid, PEG-10 Canolaglycerid, PEG-5 Tsubakiateglycerid, PEG-10 Tsubakiateglycerid, PEG-20 Tsubakiateglycerid, PEG-60 Tsubakiateglycerid, Baumwollöl Glycereth-8 Ester, Reisöl Glycereth-8 Ester, Sesamöl Glycereth-8 Ester, Weizenkeimöl Glycereth-8 Ester.

Ethoxylierte Rapsmethylester (EO 7-15), Ethoxylierte Rapsethylester (EO 7-15), Ethoxylierte Sojamethylester (EO 7-15), Ethoxylierte Sojaethylester (EO 7-15).

Besonders bevorzugt sind alkoxylierte Tenside mit einem HLB > 10.5 und <12.0. Beispiele dieser Vertreter sind Rapsmethylester oxylat 7 EO, Olivenöl Glycereth-PEG-8 Ester, Mandelöl PEG-7 Ester, PEG-10 Oliven Glyceride. Ganz besonders bevorzugt sind die Vertreter Pflanzenöl Glycereth-y Ester, Pflanze-PEG-y-ester, Ethoxylierter Rapsmethylester (y EO), mit Pflanze = Aprikose(nkern), Avocado, Baumwolle, Distel, Hanf, Jojoba, Leinsamen, Macademia, Mandel, Mais, Olive, Pfirsichkern, Reis, Sesam, Soja, Sonnenblume, Traube(nkern), Weizen(keim), Raps, Rüböl mit y= 6-10, bevorzugt 7-8.

In dieser Ausführungsform bevorzugt sind Zubereitungen, die dadurch gekennzeichnet sind, dass sie 0.1 bis 50 Gew.-%, bevorzugt 0.1 bis 20 Gew.-% und besonders bevorzugt 0.2 bis 10 Gew.-% eines oder mehrerer zusätzlicher alkoxylierter Tenside enthalten; Gew-% bezogen auf die Gesamtmenge der Zubereitung.

Das oder die Tenside (C) können in jedem beliebigen Verhältnis mit (A) und (B) kombiniert werden.

Bevorzugt liegen die Tenside (C) zu dem Tensid (A)/ Glycolipid-Biotensid (B)-Gemisch in einem Verhältnis zwischen (C) : (A/B) ≤ 20:1 und ≥1:20 vor; beispielsweise 20:1, 9:1, 8:1 u.s.w.
Besonders bevorzugt liegen die Tenside (C) in einem Verhältnis zwischen (C) : (A/B) ≤ 10:1 und ≥1:10 vor und ganz besonders bevorzugt liegen die Tenside (C) in einem Verhältnis zwischen (C) : (A/B) ≤ 2:1 und ≥1:6 vor, äusserst bevorzugt liegt (C) : (A/B) ≤ 3:1 und ≥1:10; jeweils bezogen auf Gewichtsprozent Aktivgehalt im gesamten Mittel. (Verhältnis von A:B hier beliebig).

**Weitere optionale Tenside und Emulgatoren (D)**

Unter Tensid werden in diesem Zusammenhang amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Micellen, lamellare Strukturen u.a. Im Zusammenhang dieser Erfindung, werden Emulgatoren unter den Begriff der Tenside gefasst, jedoch nicht umgekehrt.

Vorzugsweise sind die Tenside geeignet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

Seifen und Fettsäuren

In einer weiteren Ausführungsform, hat es sich als vorteilhaft erwiesen, wenn das Mittel als anionisches Tensid zusätzlich mindestens eine Seife bzw. deren korrespondierende Säure enthält. Seifen sind Alkali- oder Ammoniumsalze gesättigter oder ungesättigter Fettsäuren von 6 bis 24 Kohlenstoffatomen.

Überraschenderweise können die Mittel bestehend aus dem oder den Tensiden (A) und dem oder den Glycolipid-Biotensiden (B) bei neutralem oder alkalischem pH zusätzlich mit einer Seife kombiniert werden, bzw. bei saurem pH mit der korrespondierenden Fettsäure, so dass das Gesamtergebnis weiter verbessert wird, d.h. mit einer hohen Reinigungskraft auf Kohlenhydratflecken oder Farbstoffflecken. Es können auch Fettsäuren zugegeben werden, die beispielsweise in einem späteren Verfahrensschritt zur Herstellung des Reinigungs- und Pflegemittels dienen. Aus diesem Grund werden Fettsäuren im Zusammenhang mit dieser Erfindung als Tenside behandelt. Weiterhin kann die Zugabe von Fettsäuren oder Seife für andere Eigenschaften der Formulierung wie Konsistenz, Stabilität, Hautgefühl und nicht zuletzt den Kosten wünschenswert sein.

Besonders bevorzugt in dieser Erfindung sind Salze von Fettsäuren der Formel (X)

$$RCOOM \qquad (X)$$

M ist ein Alkali- oder Ammoniumkation oder H
R bzw. RCO wie in Formel (I) definiert und abgeleitet von C-18-Pflanzenölen. das bedeutet: R = gesättigte, ein- oder mehrfach ungesättigter Kohlenwasserstoffkette mit 5-23 Kohlenstoffatomen und RCO abgeleitet aus einem

Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt;
und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (X);
und wobei das Tensid (X) aus einem Gemisch unterschiedlicher Kettenlängen und Sättigungsgrade des Fettsäurerests RCO wie oben definiert besteht und abgeleitet ist von einem C-18-Pflanzenöl.

Beispielhafte Vertreter der erfindungsgemäss geeigneten Produkte sind:
Natrium Olivenölseifen, Kalium Rapsölseifen, Ammonium Diestelölseifen, Natrium Leinsamenölseifen, Natrium Sonnenblumenölseifen, Natrium Sojaölseifen, Leinsamenfettsäure, Olivenölfettsäuren und andere.
Erfindungmässig bevorzugt sind die Salze der Fettsäuren.
In dieser Ausführungsform bevorzugt sind Zubereitungen, die dadurch gekennzeichnet sind, dass sie - bezogen auf ihr Gewicht - 0.001 bis 50 Gew.-%, bevorzugter 0.01 bis 20 Gew.-% und besonders bevorzugt 0.1 bis 20 Gew.-% Seifen bzw. Fettsäuren und ganz besonders bevorzugt 0.5 bis 10 Gew.-% enthalten; Gewichtsprozent bezogen auf die Gesamtmenge der Zubereitung.

Weitere optionale Tenside

Weiterhin kann die Zubereitung zusätzlich mit weiteren dem Fachmann gut bekannten Tensiden kombiniert werden, wobei es erfindungsgemäss keine Einschränkungen auf den Ursprung, Kettenlänge oder Sättigungsgrad der gewählten Tenside gibt. Vorzugsweise jedoch werden Tenside verwendet, welche von C-18-Pflanzenölen abgeleitet sind.
Unter Tensid werden in diesem Zusammenhang amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Mizellen, lamellare Strukturen u.a. Vorzugsweise sind diejenigen Tenside (D) geeignet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

Weitere anionische Tenside

Geeignete optionale anionische Tenside, welche vom Fachmann frei mit der erfindungsgemässen Zubereitung kombiniert werden können, sind weitere Seifen, Fettalkoholcarboxylat, Alkylpolyglykolethercarboxylate, Acyllactylat, u.s.w. sowie deren Mischungen.
Daneben können zusätzlich optional die weniger bevorzugten Schwefeltenside, Phosphate oder Phosphonate verwendet werden. Beispiele sind Alkylbenzolsulfonate, Alkan-/Alkensulfonate, Alkylsulfate bzw. Fettalkoholsulfate, Alkylpolyglykolethersulfate mit 2 bis 6 Ethylenoxideinheiten (EO) im Etherteil, sowie Sulfosuccinate, Carbonsäureamidethersulfate, Sulfobernsteinsäuremono- und diAlkylester, $\alpha$-Olefinsulfonate, Alkylisethionat, Acylisethionat, Alkylsulfoacetate, sulfonierte Fettsäuren, sulfonierte Fettsäureester, wie sulfonierte Fettsäureglycerinester und sulfonierte Fettsäuremethylester, N-Acylaminosulfonsäuren, Alkylphosphate und Alkyletherphosphate, Phosphor- und Polyphosphorsäureester.
Überraschenderweise zeigen die erfindungsgemässen Zubereitungen eine vergleichbare Reinigungsleistung zu üblichen Mitteln mit Schwefeltensiden, auch ohne deren Verwendung. Aus Umweltschutzgründen und den zunehmenden Sulfatgehalten in Trinkwasser sowie der Konsumentenpräferenz von sulfatfreien Kosmetikprodukten ist eine bevorzugte Ausführungsvariante frei von allen Schwefeltensiden.
Ebenso kann auf die gewässerbelastenden Tenside auf Basis Phosphate und Phosphonate ohne Kompromisse an die Reinigungsleistung und Pflegeleistung verzichtet werden. Eine weitere bevorzugte Ausführungsform ist phosphat- und phosphonatfrei.

Weitere nichtionische Tenside

Als weitere optionale nichtionische Tenside welche vom Fachmann frei mit der erfindungsgemässen Zubereitung kombiniert werden können, eignen sich' beispielsweise Alkoholpolyglykolether d.h. ethoxylierte und/oder propoxylierte Alkohole mit 1-40 Ethylenoxid (EO) und/oder Propylenoxid (PO)- Einheiten, Aminoxide, Amidoamine, Amidopropyl dimethylamine, Polyethylenglykolmercaptane, Glykolipide, wie zum Beispiel Alkylpolyglykoside mit 1-10 Glykosideinheiten, Polyhydroxyfettsäureamide, Polyhydroxyfettsäureester, Carbonsäureester, Sorbitanester, sowie alkoxylierte Sorbitanester, Alkanolamin-Carbonsäure-Kondensate, N-Alkylpyrrolidone, Amidoalkyl-2-pyrrolidone.

Optionale amphotere Tenside

Weiterhin können optional amphotere Tenside, welche vom Fachmann frei mit der erfindungsgemässen Zubereitung kombiniert werden können, zusätzlich enthalten sein, wie beispielsweise N-Alkylbetaine, Alkylamidobetaine, Imidazoliniumbetaine, Aminoxide, und weniger bevorzugt Sulfobetaine, Phosphobetaine und Sultaine.

Optionale kationische Tenside

Weiterhin kann die Zubereitung optional zusätzlich kationische Tenside enthalten, beispielsweise primäre, sekundäre, tertiäre oder quartäre Alkylammoniumsalze der Formel (RI)(RII)(RIII)(RIV)$N^+X^-$, in der RI bis RVI unabhängig voneinander gleich- oder verschiedenartige organische Reste, verzweigte und unverzweigt, gesättigt oder ungesättigt, unsubstituiert, einfach oder mehrfach substituiert oder H, wobei mindestens einer der Reste RI - RIV kein H darstellt; und $X^-$ für ein Anion stehen.

Für weitere zusätzliche optionale Tenside, welche vom Fachmann frei mit der erfindungsgemässen Zubereitung kombiniert werden können, wird auf die einschlägige Fachliteratur wie z.B. Richard J. Farn, Chemistry and Technology of Surfactants, Blackwell Publishing, verwiesen.

**Emulgatoren**

Weiterhin können in der erfindungsgemässen Zubereitung als optionale Tenside (D) zusätzlich Verbindungen, die in der Literatur z.T. unter Emulgatoren geführt werden, enthalten sein.

Geeignet sind beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest; Ethoxylierte Analoga von Alkyl- und/oder Alkenyloligoglykoside mit 8 bis 24 Kohlenstoffatomen im Alk(en)ylrest; Anlagerungsprodukte von 1 bis 15 Mol Ethylenoxid an Ricinusöl und/oder gehärtetes Ricinusöl; Anlagerungsprodukte von 15 bis 60 Mol Ethylenoxid an Ricinusöl und/oder gehärtetes Ricinusöl; Addukte mit 1 bis 30 Mol Ethylenoxid von Partialestern von Glycerin und/oder Sorbitan mit ungesättigten, linearen oder gesättigten, verzweigten Fettsäuren mit 12 bis 24 Kohlenstoffatomen und/oder Hydroxycarbonsäuren mit 3 bis 18 Kohlenstoffatomen; alkoxylierte Sterine, also Steroide, die am C-Atom 3 des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können; niedrig ethoxylierte (1 - 4 EO) Alkanole und Carbonsäuren mit 8 - 24 C-Atomen, Polyethylenglycol (Molekulargewicht 400 bis 5000); Alkoxylierte Partialester von Polyglycerin (durchschnittlicher Eigenkondensationsgrad 2 bis 8), sowie Partialester von Trimethylolpropan, Pentaerythrit, mit gesättigten und/oder ungesättigten, linearen oder verzweigten Fettsäuren mit 12 bis 24 Kohlenstoffatomen und/oder Hydroxycarbonsäuren mit 3 bis 18 Kohlenstoffatomen; Mono-, Di- und Trialkylphosphate sowie Mono-, Di- und/oder Tri-PEG-alkylphosphate und deren Salze; Polysiloxan-Polyalkyl-Polyether-Copolymere bzw. entsprechende Derivate; Block-Copolymere z. B. Polyethylenglycol-30 Dipolyhydroxystearate; Polymeremulgatoren, z. B. Pemulen-Typen; Polyalkylenglycole; Wollwachsalkohole; Gemische derselben; Fettalkohole, Partialester von Polyglycerin (durchschnittlicher Eigenkondensationsgrad 2 bis 8), Pentaerythrit, Zuckeralkoholen (z. B. Sorbit), Alkylglucosiden sowie Polyglucosiden (z. B. Cellulose) mit gesättigten und/oder ungesättigten, linearen oder verzweigten Fettsäuren mit 12 bis 24 Kohlenstoffatomen und/oder Hydroxycarbonsäuren mit 3 bis 18 Kohlenstoffatomen; Polyglycerinester, Polyolester, umgesetzte Mono-, Di- und Triester von Trimethylolpropan oder Pentaerythrit mit C8-C24 Fettsäuren,

Alkyl- und/oder Alkenyloligoglykoside mit 8 bis 24 Kohlenstoffatomen im Alk(en)ylrest, Acylglutamine, Partialestern von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen, Glyceride wie Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozess noch geringe Mengen an Triglycerid enthalten können. Mischester aus Pentaerythrit, Fettsäuren, Citronensäure und Fettalkohol und/oder Mischester von Fettsäuren mit 6 bis 24 Kohlenstoffatomen, Methylglucose und Polyole, vorzugsweise Glycerin oder Polyglycerin, Glycerincarbonat, Gemische derselben. Besonders geeignete Beispiele sind Glyceryl Stearate citrat, Glyceryl stearate tartrate.

Dicarbonsäuren mit 12 bis 24 Kohlenstoffatomen, wie beispielsweise Azelainsäure oder Sebacinsäure

Weitere erfindungsgemäss geeignete Emulgatoren sind dem Fachmann allgemein bekannt und können aus der einschlägigen Literatur, beispielsweise Kirk-Othmer, "Encylcopedia of Chemical Technology", 5. Aufl. 2007 entnommen werden.

Im Folgenden werden einige bevorzugte Beispiele genannt:

Bevorzugte Emulgatoren sind lineare gesättigte Alkanole mit 12 - 30 Kohlenstoffatomen, insbesondere mit 16 - 22

Kohlenstoffatomen. Typische Beispiele sind Capronalkohol, Gaprylalkohol, 2-Ethylhexylalkohol, Caprinalkohol, Laurylalkohol, Isotride- cylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachidyl- alkohol, Gadoleylalkohol, Behenylalkohol, Erucylalkohol und Brassidylalkohol sowie deren Gemische dieser Alkohole, wie sie bei der technischen Hydrierung von pflanzlichen und tierischen Fettsäuren erhältlich sind.

Erfindungsgemäss besonders bevorzugt sind Fettalkohole abgeleitet aus C18-Pflanzen, insbesondere Mischungen aus Fettalkohlen welche der natürlichen Zusammensetzung des Pflanzenöls entsprechen oder der Mischung, welche durch Umsetzung des Pflanzenöls zum Fettsäure- bzw. Fettsäureestergemisches mit anschliessender Reduktion zum Fettalkoholgemisch erhalten wird.

Vorzugsweise sind die Tenside (D) ausgewählt aus Seife bzw. Fettsäuren, Polyhydroxyfettsäureamide, Polyhydroxyfettsäureester, sowie Glyceryl- und Polyglycerylester mit 1-20 Glycerineinheiten, Carbonsäureester, Sorbitanester, sowie alkoxylierte Sorbitanester, acylierte Aminosäuren oder Peptide, N-Alkylbetaine, Amidoaminen, Alkylamidobetaine, Imidazoliniumbetaine, Aminoxide, Fettalkoholen. Die bevorzugte erfindungsgemäße Zubereitungen können ein oder mehrere zusätzliche Tenside (D) in einer Gesamtmenge von 0.05-50 Gew.-%, vorzugsweise 0.2-20 Gew.-%, insbesondere 0.5-10 Gew.-% enthalten, bezogen auf die Gesamtmenge der Zubereitung.

Besonders bevorzugt enthalten die Zubereitungen Tenside (D), die abgeleitet sind von C-18-Pflanzenölen mit den lipophilen Resten R, stammend aus Fettsäuren RCOOH, wie unter (I) definiert. Die Reste R liegen bevorzugt als Gemisch gemäss der Fettsäureverteilung im nativen Öl vor oder wie sie bei der Umsetzung von nativen Ölen entstehen.

<u>Weitere Biotenside / Bioemulgatoren</u> (E)

In der erfindungsgemässen Zubereitung können zusätzlich weitere optionale Biotenside (E) enthalten sein, insbesondere Lipopeptide, welche vorzugsweise aus einem Cycloheptapeptid, mit gesättigten, ungesättigten, substituierten oder unsubstituierten Fettsäurereste von Kettenlängen von 2-30 Kohlenstoffatomen bestehen, welche an die Aminosäurereste gebunden sind, beispielsweise Surfactin, Iturin, Fengycin.

Bioemulgatoren (E) werden von Mikroorganismen produziert. Bioemulgatoren gehören den Biotensiden an, werden aber in der Literatur als höhermolekulare Verbindungen als die klassischen Glycolipid- Biotenside verstanden (Chibuzo Uzoigwe et al., Front. Microbiol. 2015; 6; 245), welche aus komplexen Mischungen von Heteropolysacchariden, Lipopolysacchariden, Lipoproteinen und Proteinen bestehen. Weitere Beispiele für Bioemulgatoren (E) sind Emulsan, produziert durch Acinetobacter calcoaceticus, Alasan, produziert durch A. redioresistens, oder mannoprotein bioemulsifiers von Kluyveromyces marxianus, Saccharomyces cerevisiae, oder Acinetobacter sp.

Desweiteren werden unter Bioemulgatoren (E) Biotenside verstanden, die dadurch gekennzeichnet sind, dass das Biotensid-Glycolipide (B) oder der Bioemulgatoren (E) chemisch oder enzymatisch so derivatisiert wurde, dass die Zuordnung unter (B) nicht mehr möglich ist, wie beispielsweise Amidierung, Glycosylierung, Deglycosilierung, Veresterung, Oxidation der Hydroxylgruppen oder C,C-Doppelbindungen, Additionen an die C,C-Doppelbindungen (z.B. nukleophil oder Cylcoadditionen), und andere.

Ein Beispiel für eine Modifizierung ist das Sophorolipid der Formel (III) mit $R^9 =$ - $COO(CH_2)_m$ Z mit m= 2-8, Z = OH, $CH_3$, $N(CH_3)_2$, $N(CH_3)_3^+$, $NH_2$, $NH_3^+$, (1-pyrrolinyl), (2-imidazolyl), oder -$CONH(CH_2)_m$Z mit m= 2-8, Z = $NH_2$, NH $(CH_2)_4NH(CH_2)_3$ $NH_2$, (1-Imidazole), sowie, deren Ammoniumsalze

Die bevorzugte erfindungsgemäße Zubereitungen können ein oder mehrere zusätzliche Bioemulgatoren (E) in einer Gesamtmenge von 0.05-50 Gew.-%, vorzugsweise 0.2-20 Gew.-%, insbesondere 0.5-10 Gew.-% enthalten, bezogen auf die Gesamtmenge der Zubereitung.

Besonders bevorzugt enthalten die Zubereitungen Tenside und Emulgatoren (E), die abgeleitet sind von C-18-Pflanzenölen mit den lipophilen Resten R, stammend aus Fettsäuren RCOOH, wie unter (I) definiert. Die Reste R liegen bevorzugt als Gemisch gemäss der Fettsäureverteilung im nativen Öl vor oder wie sie bei der Umsetzung von nativen Ölen entstehen.

**Gesamt-Tensidzusammensetzung**

Bevorzugt enthält die Zubereitung einen Anteil an Tensiden, bestehend aus Tensid (A), Tensid (C) und gegebenenfalls den Tensiden (D) und (E), mit der Vorgabe, dass. das oder die Tenside (D) und (E) von einem C-18-Pflanzenöl abgeleitet sind, und Biotensid-Glycolipid (B), welcher in Summe ≥ 30%, bevorzugt ≥ 60 %, besonders bevorzugt ≥ 95 % und äusserst bevorzugt ≥ 99 % beträgt, bezogen auf den Gesamtgehalt an Tensiden in Gew.-% in dem Mittel.

Dieser Anteil in % stellt den Anteil an Tensiden in der Zubereitung dar, welcher von C-18-Pflanzen abgeleitet ist, und wird bestimmt nach der Formel:

$$\text{Anteil} = [(A) + (B) + (C) + (D_{C18}) + (E_{C18})] / [(A) + (B) + (C) + (D) + (E)]$$

Wobei

(A), (B), u.s.w. jeweils die Menge an dem oder den Tensiden (A), (B), u.s.w. angegeben, bezogen auf Gew.-% bezogen auf die Gesamtzusammensetzung,

wobei (C), (D) und (E) jeweils unabhängig voneinander Null sein können,

wobei (D) und (E) weitere optionale Tenside (RX mit X = hydrophiler Rest) wie oben definiert darstellen, mit der Vorgabe, dass sie die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

(D) und (E) können entweder abgeleitet sind aus C-18-Pflanzen (Hier genannt $D_{C18,}$ $E_{C18}$) - mit R stammend aus den Fettsäuren RCOOH mit R, bzw. RCO definiert wie unter Formel (I) - oder abgeleitet sein von anderen Ölen oder Fetten wie beispielsweise Erdöl, Palmkernöl, Kokosöl, tierischen Fetten, Rizinusöl oder Silikontenside u.a. (hier genannt $D_{Rest}$ bzw. $E_{Rast}$). D.h. (E) = ($E_{C18}$ + $E_{Rest}$ ).bzw. (D) = ($Dc_{18}$ + $D_{Rest}$ ).

[(A) + (B) + (C) +(D) + (E)] stellt den Gesamtgehalt an Tensiden in Gew.-% in der Zubereitung dar.

**Weitere Zusatzstoffe & Eigenschaften der kosmetischen Zubereitung**

**Chelatbildner**

Chelatbildner erhöhen die Stabilität der Zubereitungen, können jedoch auch gegen Zahnstein wirksam sein.

Überraschenderweise zeigen die Chelatbildner in Kombination mit den erfindungsgemässen Zubereitungen eine unverändert hohe bis verbesserte Reinigungsleistung auf Kohlenhydrate, Farb- und Pigmentanschmutzungen und beeinträchtigen somit die synergistische Wirkung zwischen den Biotensid-Glycolipiden und Tensid (A) nicht. Dies ist insofern erstaunlich, da die Chelatbildner aufgrund Ihrer Nukleophilie mit dem hydrophilen Teil des Biotensid-Glycolipid strukturell in Konkurrenz treten.
Erstaunlicherweise erhöht die Zugabe von Chelatbildner zu den erfindungsgemässen Zubereitungen deutlich die Reinigungskraft für Makeup auf der Haut und erhöht die Schaumbildung der Zubereitung und die Schaumstabilität erheblich. Weiterhin wurde festgestellt, dass die Zugabe von Chelatbildnern die Zubereitungen in einer nicht vorhersehbaren Weise klarstellten. Es sind dadurch auch transparente Formulierungen möglich.
In den Ausführungsbeispielen sind exemplarische Zubereitungen mit Chelatbildnern offenbart.

Erfindungsgemäss geeignet sind alle in Reinigungs- und Pflegemittel üblichen Chelatbildner, z.B. aus den Gruppen der Phosphate und Phosphonate, Schichtsilikate, Zeolithe, Carbonate und Polycarboxylate, Aminopolycarbonsäuren, wie Aminoessigsäuren und Polyaminoessigsäuren sowie deren Salze, Hydroxycarbonsäuren und deren Salze, Polyglycoside und-gluconsäuren und deren Salze.

Vetreter geeigneter Chelatbildner sind beispielsweise die folgenden gemäss INCI bezeichneten Verbindungen: Aminotrimethylene Phosphonsäure, Beta-Alanine Acetessigsäure, Calcium Disodium EDTA, Chitosan, Zitronensäure und dessen Salze und Hydrate, Cyclodextrin, Cyclohexanediamin Tetraessigsäure, Diammonium Citrat, Diammonium EDTA, Diethylenetriaminepentaacetic Acid, Diethylenetriamine Pentamethylene Phosphorsäure, Dikalium EDTA, Dinatrium Azacycloheptane Diphosphonat, Dinatrium EDTA, Dinatrium Pyrophosphate, EDTA, Ethylenediamine- *N,N'*-disuccinic acid (EDDS), Etidronsäure, Galactarsäure, β-Glucan, Gluconsäure, Glucuronsäure, Glucoheptonsäure, HEDTA, Hydroxypropyl Cyclodextrin, Methyl Cyclodextrin, Pentapotassium Triphosphat, Pentasodium Aminotrimethylene Phosphonat, Phosphonobutane tricarbonsäure (PBTC), Pentasodium Ethylenediamine Tetramethylene Phosphonat, Pentasodium Pentetate, Pentasodium Triphosphate, Pentetic Acid, (DTPA), Phytic Acid, Potassium Citrate, Potassium EDTMP, Kalium Gluconate, Kalium Polyphosphate, Kalium Trisphosphonomethylamine Oxide, Ribonsäure, Natrium Chitosan Methylene Phosphonate, Natriumcitrat, Natrium Diethylenetriamine Pentamethylene Phosphonate, Natrium Dihydroxyethylglycinate, Natrium EDTMP, Natrium Glucoheptate, Natrium Gluconate, Natrium Glycereth-1 Polyphosphate, Natrium Hexametaphosphate, Natrium Metaphosphate, Natrium Metasilicate, Natrium Phytate, Natrium Polydimethylglycinophenolsulfonate, Natrium Trimetaphosphate, TEA-EDTA, TEA Polyphosphate, Tetrahydroxyethyl Ethylenediamin, Tetrahydroxypropyl Ethylenediamin, Tetrakalium Etidronat, Tetranatrium Iminodisuccinat (IDS), Tetrakalium Pyrophosphat, Tetranatrium EDTA, Tetranatrium Etidronat, Tetranatrium Pyrophosphat, Trikalium EDTA, Trikalium Dicarboxymethyl Alaninat, Trinatrium EDTA, Trinatrium HEDTA, Trinatrium Methylglycin dieacetic acid (MGDA- $Na_3$), Trinatrium NTA und Trinatrium Phosphate, Phytinsäure, Plfanzenextrakte wie z.B. Lupinus Albus Seed Extract, Carnosine, Bambusa Arundinacea Leaf Extract, Citrus Paradisi (Grapefruit) Peel Extract, Sambucus Nigra Extract; oder im Falle von Säuren, deren Salze. Diese Chelatbildner können vom Fachmann frei mit anderen hier genannten Inhaltstoffen kombiniert werden.

In einer bevorzugten Ausführungsform enthalten die erfindungsgemässen Zubereitungen Chelatbildner, die biologisch abbaubar sind. Bevorzugt enthalten die erfindungsgemässen Zubereitungen daher keine Phosphate, keine Phosphonate, kein EDTA und keine Polycarboxylate.

Bevorzugt in dieser Erfindung sind daher folgende Komplexbildner auf Basis erneuerbarer Rohstoffe, bzw. mit einer sehr guten biologischen Abbaubarkeit: Beta-Alanine, Beta-Glucan, Chitosan, Diacetic acid, Cyclodextrin, Ethylenediamine- *N,N'*-disuccinic acid (EDDS), Galactarsäure, Gluconsäure, Glucuronsäure, Glucoheptonsäure, Methylcyclodextrin, Hydroxypropyl cyclodextrin, Phytinsäure, Polyasparaginsäure, Natrium Carbonat, Carboxy methyl inulin und Natrium Carboxymethyl inulin (NaCMI), Natrium Dihydroxyethylglycinat, Natrium Iminodisuccinat, Natrium Lignosulfate, Tetranatrium Iminodisuccinat (IDS, Tetranatrium Glutamate Diacetat (GLDA, I-glutamic acid, N,N-di (acetic acid), tetrasodium salt), Trinatrium Methylglycin dieacetic acid (MGDA- $Na_3$), Zitronensäure, Milchsäure und jeweils deren Salze, Plfanzenextrakte wie z.B. Lupinus Albus Seed Extract, Carnosine, Bambusa Arundinacea Leaf Extract, Citrus Paradisi (Grapefruit) Peel Extract, Sambucus Nigra Extract, oder im Falle von Säuren, deren Salze.

Äusserst bevorzugt sind die Chelatbildner Tetranatrium Glutamate Diacetat (GLDA, I-glutamic acid, N,N-di (acetic acid), tetrasodium salt), Tetranatrium Iminodisuccinat (IDS), Ethylenediamine- *N,N'*-disuccinic acid (EDDS), Trinatrium Methylglycin dieacetic acid (MGDA- $Na_3$), Gluconsäure, Glucuronsäure, Glucoheponsäure, Polyasparaginsäure, sowie deren Salze.

Bevorzugte erfindungsgemäße Zubereitungen enthalten mindestens einen Komplexbildner in einer Gesamtmenge von 0,1-20 Gew.-%, vorzugsweise 0,2-15 Gew.-%, insbesondere 0,5-10 Gew.-%, bezogen auf die Gesamtmenge der Zubereitung.

In den Ausführungsbeispielen werden examplarisch Zubereitungen mit Tetranatrium GLDA, MGDA- $Na_3$, IDS, Chitosan, Natrium carbonat, Beta-Glucan, Citrus Paradisi (Grapefruit) Peel Extract und Natrium Gluconat als Kompexbildner offenbart.

### Abrasiva und Poliermittel

Die erfindungsgemässen Zubereitungen zeigen eine gute Reinigungswirkung, so dass der Zusatz von Abrasiva entbehrlich ist. Nichtsdestoweniger können die Zubereitungen, insbesondere im Bereich der Mund- und Zahnpflege oder im Bereich der Grobreiniger gegebenenfalls für bestimmte Reinigungsanwendungen Abrasiva als Komponenten enthalten. Hierzu gehören Kunststoffreibemittel auf der Basis von Polyethylen oder Polyurethan, organische Polymere, mineralische Reibemittel wie Kieselsäuren z.B. Gelkieselsäuren, Hydrogelkieselsäuren und Fällungskieselsäuren, Aluminiumhydroxid, Aluminiumoxid, Magnesiumsulfat, Natriumaluminiumsilikate, Calciumcarbonat, Kaolin, Sand, Kreide, Calciumpyrophosphat, Dicalcium-phosphatdihydrat und andere sowie Reibemittel auf pflanzlicher Basis wie Cellulosederivate, Holzmehl oder Kern- und Schalenmehle, sowie deren Gemische.

Insbesondere bevorzugt sind für die Verwendung als Grobreinigung sind Reibemittel auf der Basis von natürlichen Kern- und/oder Schalenmehlen, insbesondere Walnussschalen, Mandelschalen, Hasselnussschalen, Olivenkern-, Aprikosenkern- und Kirschkernmehl oder Perlen aus Wachsen (z.B. Jojobawachse).

Für die Verwendung in der Zahnpflege bevorzugt geeignete Poliermittelkomponenten sind calciumarm wie Kieselsäuren, Aluminiumhydroxid, Aluminiumoxid, Natriumaluminiumsilikate, organische Polymere oder Gemische solcher Reibkörper.

Die Konzentration der Reibemittel kann bis zu 50 Gew.-% betragen, bevorzugt 0 - 30 Gew.-%, bezogen auf die Gesamtmenge der Zubereitung.

### Lösungsmittel

Die erfindungsgemässe Zubereitung kann alle in Kosmetik üblichen Lösungsmittel enthalten. Sie dienen der Stabilisierung der Formulierung, der Solubilisierung von schlecht löslichen Inhaltsstoffen, dem Feuchthalten der Haut und daraus resultierenden verbesserten sensorischen Eigenschaften und der Erhöhung der Reinigungsleistung insbesondere auf Fett und Öl.

Überraschenderweise wird in den erfindungsgemässen Zubereitungen durch Zugabe eines milden organischen Lösungsmittels die Synergie von Tensid (A) mit den Biotensid-Glycolipiden (B) nicht beeinträchtigt. Es wird sogar eine Verbesserung bei einzelnen Verunreinigungen festgestellt, exemplarisch in den Ausführungsbeispielen an Lidschatten offenbart. Hierbei wurde ein Teil des Wassers durch ein mildes organisches Lösungsmittel ersetzt ohne Beeinträchtigung der synergistischen Wirkung von Tensid (A) mit den Biotensid-Glycolipiden (B). Dies ist insofern nicht selbstverständlich, als dass die Interaktionen Tensid- Wasser stark durch Wasserstoffbrückenbindung gesteuert sind, die durch die Zugabe eines Lösungsmittels wesentlich verändert wird. Bekanntermassen führt dies zu veränderten Solubilisierungs- bzw. Dispergiervermögen, deren Richtung und Ausprägung jedoch a priori nicht vorhergesagt werden kann.

Im Falle der Biotensid-Glycolipide (B) mit Tensid (A) zeigt sich eine verbesserte Reinigung für pigmentäre Verunreinigungen, wie hier am Lidschatten offenbart. Weitere Tests haben diesen Effekt auch auf anderen Substraten

bestätigt.

Gegenstand der Erfindung ist daher die Zubereitung gemäss einem der vorgehenden Ansprüche zusätzlich enthaltend ein oder mehrere milde organische Lösungsmittel.

In einer bevorzugten flüssigen oder gelförmigen Ausführungsform enthält die Zubereitung Wasser als Lösungsmittel, wobei mehr als 5 Gew.-%, bevorzugt mehr als 15 Gew.-% und besonders bevorzugt mehr als 25 Gew.-% Wasser enthält, jeweils bezogen auf den Aktivgehalt in der Gesamtmenge der Zubereitung. Besonders bevorzugte Zubereitungen enthalten - bezogen auf ihr Gewicht- 5 bis 99.5 Gew.-%, bevorzugt 15 bis 90 Gew.-%, besonders bevorzugt 25 bis 75 Gew.-% Wasser.

Alternativ kann es sich um wasserarme oder wasserfreie Zubereitungen handeln, wobei der Gehalt an Wasser in einer bevorzugten Ausführungsform weniger als 10 Gew.-%, besonders bevorzugt weniger als 8 Gew.-% enthält, jeweils. bezogen auf die Gesamtmenge der Zubereitung.

In einer weiteren flüssigen oder gelförmigen Ausführungsform ist die Zubereitung wasserfrei, wobei die Zubereitung ein mildes organisches Lösungsmittel als Hauptlösungsmittel enthält. Dabei ist es bevorzugt, dass das Mittel 5 bis 99.5 Gew.-%, bevorzugt 10 bis 90 Gew.-%, besonders bevorzugt 25 bis 75 Gew.-% Lösungsmittel enthält, bezogen auf die Gesamtmenge der Zubereitung.

Beispielhafte geeignete organische Lösungsmittel sind die folgenden gemäss INCI benannten Verbindungen: Alcohol (Ethanol), Alkohole, Buteth-3, Butoxydiglycol, Butoxyethanol, Butoxyisopropanol, Butoxypropanol, n-Butyl Alcohol, t-Butyl Alcohol, Butyl-3-hydroxybutyrate, Butylene Glycol, Butyloctanol, C1-C6-Alkane, C7-C15-Alkane, Diethylene Glycol, Diethylenglycol monobutylether, Dimethoxydiglycol, Dimethyl Ether, Dimethyl 2-methylglutarat, Dipropylene Glycol, Dipropylenglycol Phenylether, Ethyllactat, 2-Ethyllactat, Ethyl levulinate glycerol ketal, Ethyl levulinate propylene glycol ketal, Ethyl levulinate ethylene glycol ketal, Ethoxydiglycol, Ethoxyethanol, Ethyl Hexanediol, Fettsäuremethylester z.B. auf Basis C18-Pflanzen, Gammalaverolacton, Glycol, Glycerin, Hexanediol, 1,2,6-Hexanetriol, Hexyl Alcohol, Hexylene Glycol, Isobutoxypropanol, Isopentyldiol, Isopropyl Alcohol (iso-Propanol), Lävulinsäure ester, 3-Methoxybutanol, Methoxydiglycol, Methoxyethanol, Methoxyisopropanol, Methoxymethylbutanol, Methoxy PEG-10, Methylal, Methyl Alcohol, Methyl-9-dodecenoate, Methyl Hexyl Ether, Methylpropanediol, 2-Methyl THF, Neopentyl Glycol, N,N-Dimethyl-9-decenamide, Polyole, PEG-4, PEG-6, PEG-7, PEG-8, PEG-9, PEG-6 Methyl Ether, Pentylene Glycol, PPG-7, PPG-2-Buteth-3, PPG-2 Butyl Ether, PPG-3 Butyl Ether, PPG-2 Methyl Ether, PPG-3 Methyl Ether, PPG-2 Propyl Ether, 1,2-Propanediol, 1,3-Propandiol, Propyl Alcohol (n-Propanol), Propylene Glycol, Propylene Glycol Butyl Ether, Propylene Glycol Propyl Ether, Terpene, z.B. Limonen, Thymol, u.a. insbesondere natürlichen Ursprungs wie Zitronenöl, Lavendelöl, Thymianöl, u.a., Tetrahydrofurfuryl Alcohol, Trimethylhexanol. Erfindungsgemäss können diese Lösungsmittel in einer dem Fachmann durchaus bekannten Art und Weise frei mit anderen Inhaltsstoffen kombiniert werden.

In einer bevorzugten Ausführungsform, werden Lösungsmittel aus der Gruppe Lösungsmittel, die aus pflanzlichen Rohstoffen gewonnen werden und biologisch abbaubar sind, verwendet. Erfindungsgemäss bevorzugte milde organische Lösungsmittel sind daher ausgewählt aus der Gruppe Ethanol, C1-C6-Alkane, C7-C15-Alkane, Dimethyl 2-methylglutarat, Ethyllactat, 2-Ethyllactat, Ethyl levulinate glycerol ketal, Ethyl levulinate propylene glycol ketal, Ethyl levulinate ethylene glycol ketal, Fettsäuremethylester auf Basis C18-Pflanzen, Gammalaverolacton, Glycerin, 2-Methyl THF, Polyole, Pentylene Glycol, 1,2-Propanediol, 1,3-Propandiol, Pentylenglycol, Fettsäurealkylester von C-18-Pflanzen, insbesondere Rapsmethylester, Sonnenblumenmethylester, Sojamethylester, Terpene, z.B. D-Limonen, Tetrahydrofurfuryl Alcohol.

Ganz besonders bevorzugt ist die Ausführungsform enthaltend Ethanol, Glycerin, 1,2-Propanediol, 1,3-Propandiol, Pentylenglycol, oder einen Fettsäurealkylester von C-18-Pflanzen, insbesondere Rapsmethylester und Sojamethylester als mildes organisches Lösungsmittel.

Das Lösungsmittel kann dabei in der wässrigen, der wasserarmen sowie der wasserfreien Ausführungsform enhalten sein, dabei ist es bevorzugt, dass die Zubereitung 0.2- 99.5 Gew.-% Lösungsmittel, besonders bevorzugt 0.5 -98 Gew.-% enthält, wobei die Konzentration jeweils bezogen ist auf den Aktivgehalt in der gesamten Zubereitung.

**pH- Stellmittel und Puffersubstanzen**

Der pH-Wert der erfindungsgemässen Zubereitung kann mittels üblicher pH-Regulatoren eingestellt werden, wobei je nach Anwendung dem Fachmann bekannte, unterschiedliche pH-Bereiche von sauer (pH 1-4) zu neutral (pH 5-7) bis hin zu basisch (pH 8-11) eingestellt werden.

pH-Stellmittel und Puffersubstanzen, deren Auswahl dem Fachmann keine Mühe . bereitet, sind beispielsweise Carbonsäuren, Mineralsäuren, Ammoniak und Amine, z.B. Natriumbicarbonat, Natriumeitrat, Natriumbenzoat, Zitronensäure, Milchsäure, Glycolsäure, Phosphorsäure oder saure Salze, z.B. $NaH_2PO4$, Alkali- oder Erdalkalihydroxide oder organische Amine, wie Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin. Auch die Verwendung von Omega-Aminosäuren wie Omega-Aminocapronsäure als Alkalisierungsmittel ist möglich.

Die Zubereitung ist über einen weiten pH-Bereich stabil, so werden unterschiedliche Ausführungsformen ermöglicht, wie zum Beispiel Haarfärbebereich zwischen 5 und 11, Rasur/ Haarentfernung zwischen 3 und 10, Seifen und Gesichtswasser pH 5-9, insbesondere ein hautneutraler pH von 5.5.
Vorzugsweise hat die Zubereitung einen pH-Wert zwischen 0-14,besonders bevorzugt zwischen 3 und 11.

**Antioxidantien**

Bevorzugt werden der kosmetischen Zubereitung Antioxidantien zugesetzt, einerseits um die ungesättigten Kohlenwasserstoffketten der erfinderischen Zubereitung zu schützen und andererseits, um bei der Anwendung schädliche oxidative Einflüsse auf Haut bzw. Haare zu mindern. Beispielsweise werden die Antioxidantien ausgewählt aus der Gruppe bestehend aus Aminosäuren (z.B. Glycin, Histidin, Tyrosin, Tryptophan) und deren Derivaten, Imidazole (z.B. Urocaninsäure) und deren Derivaten, Peptide wie D,L-Carnosin, D-Carnosin, L-Carnosin und deren Derivaten (z.B. Anserin), Carotinoide, Carotine und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, [gamma]-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z.B. [alpha]-Hydroxyfettsäuren, Palmitinsäure, Phytinsäure, Lactoferrin), [alpha]-Hydroxysäuren (z.B. Citronensäure, Milchsäure, Apfelsäure), Numinsäure, Gallensäure, Gallenextrakte, Bilirubin, Biliverdin, EDTA, EGTA und deren Derivate, ungesättigte Fettsäuren und deren Derivate (z.B. [gamma]-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, [alpha]-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid), Superoxid-Dismutase und die erfindungsgemäss geeigneten Derivate (Salze, Ester, Ether, Zucker, Nukleotide, Nukleoside, Peptide und Lipide) dieser genannten Stoffe.

Erfindungsgemäss bevorzugt sind Antioxidantien, die auf Rohstoffen aus Pflanzen, bevorzugt C-18-Pflanzen beruhen, wie beispielsweise Antioxidantien aus den Gruppen der Aminosäuren, Peptide, Cartinoide, Chelatoren, Pflanzenextrakten und Hydroxysäuren, sowie Mischungen derselben.

Die Menge der Antioxidantien (eine oder mehrere Verbindungen) in den Zubereitungen beträgt vorzugsweise 0.001 bis 30 Gew.-%, besonders bevorzugt 0.05-20 Gew.-%, insbesondere 1-10 Gew.-%, bezogen auf die Gesamtmenge der Zubereitung. Diese Antioxidatien können vom Fachmann mit anderen hier genannten Inhaltstoffen kombiniert werden.

**Konditionierungsmittel**

Die erfindungsgemässe Zubereiung kann übliche, dem Fachmann bekannte Konditionierungsmittel enthalten, wie beispielsweise monographiert in dem "International Cosmetic Ingredient Dictionary and Handbook", 14. Auflage, K. Schrader, oder den "Fundamentals and Formulations of Cosmetics", 2. Auflage (Hüthig Verlag, Heidelberg, 1989), S. 782-815.

**Desodorierende Wirkstoffe**

Das Mittel kann Deodorant-Wirkstoffe enthalten. Hierunter werden Geruchsabsorber, desodorierend wirkende Ionenaustauscher, keimhemmende Mittel, präbiotisch wirksame Komponenten sowie Enzyminhibitoren oder Kombinationen der genannten Wirkstoffe verstanden.

Als Geruchsabsorber dienen Silicate, die auch gleichzeitig die Viskosität der erfindungsgemäßen Mittel vorteilhaft unterstützen können. Hierzu zählen Schichtsilicate, insbesondere Montmorillonit,. Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.

Sie werden in einer Menge von 0.1 - 10 Gew.-%, vorzugsweise 0.5 - 7 Gew.-% und insbesondere 1 — 5 Gew.-%, jeweils bezogen auf die Gesamtmenge der Zubereitung eingesetzt.

**Keimhemmende, fungizide und antimikrobielle Wirkstoffe**

Optional kann die erfindungsgemässe Zubereitung Wirkstoffe gegen Mikroorganismen wie Pilze oder Bakterien

enthalten um Geruch, Schuppenbildung, Karies, Akne u.a. zu unterbinden.

Als keimhemmende oder antimikrobielle Wirkstoffe erfindungsgemäß einsetzbar sind Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, $\alpha$-Monoalkylglycerinether mit einem verzweigten oder linearen gesättigten oder ungesättigten, gegebenenfalls hydroxylierten $C_6$ — $C_{22}$-Alkylrest, besonders bevorzugt $\alpha$-(2-Ethylhexyl)glycerinether, Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Wirkstoffe sind ausgewählt aus sogenannten präbiotisch wirksamen Komponenten, worunter erfindungsgemäß solche Komponenten zu verstehen sind, die nur oder zumindest überwiegend die unerwünschten Keime der Hautmikroflora hemmen, nicht aber die erwünschten, das heißt, diejenigen, die zu einer gesunden Hautmikroflora gehören. Dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus Picea sp., Paullinia sp., Panax sp., Lamium album oder Ribes nigrum sowie Mischungen dieser Substanzen.

Weitere bevorzugte Wirkstoffe sind ausgewählt aus den keimhemmend wirkenden Parfümölen bzw. ätherischen Ölen.

Weiterhin kann das Mittel Enzyminhibitoren enthalten, die die für die Schweißzersetzung verantwortlichen Enzyme, insbesondere die Arylsulfatase, $\beta$-Glucuronidase, Aminoacylase, die esterspaltenden Lipasen und die Lipoxigenase, hemmen, z. B. Trialkylcitronensäureester, insbesondere Triethylcitrat, oder Zinkglycinat.

Die Menge der Deodorant-Wirkstoffe in den erfindungsgemäßen Zusammensetzungen beträgt 0,1 — 10 Gew.-%, vorzugsweise 0,2 — 7 Gew.-%, insbesondere 0,3 - 5 Gew.-% und außerordentlich bevorzugt 0,4 — 1,0 Gew.-%, bezogen auf die Gesamtmenge der Zubereitung.

**Antitranspirant-Wirkstoffe**

Optional können auch Antitranspirant-Wirkstoffe enthalten sein wie wasserlösliche adstringierende anorganische und organische Salze des Aluminiums, Zirkoniums und Zinks bzw. beliebige Mischungen dieser Salze. Hierzu zählen Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Atuminium-Zirkonium-Chiorohydrat- Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat ($KAl(SO_4)_2 \cdot 12\,H_2O$, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat.

Die Menge der Antitranspirant-Wirkstoffe in den erfindungsgemäßen Zusammensetzungen kann bis zu 10 Gew.-% betragen, vorzugsweise 0.2 — 7 Gew.-%, insbesondere 0.3 — 5 Gew.-% und außerordentlich bevorzugt 0.4 — 1.0 Gew.-%, bezogen auf die Gesamtmenge der Zubereitung.

Eine bevorzugte Ausführungsvariante enthält keine Antitranspirant-Wirkstoffe.

**Konservierungsmittel**

Die erfindungsgemässe Zubereitung kann alle in Kosmetika üblichen Konservierungsmittel enthalten. Diese haben teilweise auch antimikrobielle Eigenschaften.

Erfindungsgemäss geeignet sind beispielsweise Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff- und Stickstoff-Acetale sowie -Formale, Benzamidine, Isothiazole und deren Derivate wie Isothiazolinone, Phtalimidderivate, Pyridinderivate, oberflächenaktive Verbindungen, Guanidine, antimikrobielle amphoterer Verbindungen, Chinoline, 1,2-Dibrom-2,4-dicyanobutan, lodo-2-propynyl-butyl-carbamat, Iod, lodophore und Peroxide. Derartige Stoffe können z. B. ausgewählt sein aus p- Hydroxybenzoesäuremethyl-, -ethyl- oder propylester,

Natriumsorbat, Natriumbenzoat, Bromchlorophen, Triclosan, Phenyl- Salicylsäureester, Biguanide, z. B. Chlorhexidin und Thymol.

Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z. B. Methyl-, Ethyl-, Propyl-, Isopropyl-, Butyl-, Isobutyl-, Isodecyl-, Phenyl-, Phenoxyethyl- und Benzylparaben, Hexamidinparaben und - diparaben, Natrium- und Kaliumparaben, Natrium- und Kaliummethylparaben, Kaliumbutylparaben, Natrium- und Kaliumpropylparaben), Alkohole und deren Salze (z.B. Ethanol, Propanol, Isopropanol, Benzylalkohol, Phenethylalkohol, Phenol, Kaliumphenolat, Phenoxyethanol, Phenoxyisopropanol, o-Phenylphenol), Guajacol und dessen Derivate, Chlorhexidin und dessen Derivate (z. B. Chlorhexidindiacetat, -digluconat, und -dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylharnstoff, Imidazolidinylharnstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquaternium-42, Quaternium-8, Quaternium-14, Quaternium-15), Carbamate (z. B. Iodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Thymol, Triclosan, Zinkpyrithion sowie diverse Peptid-Antibiotika (z. B. Nisin).

Erfindungsgemäss bevorzugt ist eine milde Konservierung der Zubereitung auf Basis von Wirkstoffen, ausgewählt aus Ethanol, Benzylalkohol, Dehydroessigsäure und deren Salzen, pflanzlichen organischen Säuren, Glycerin, Zitronensäure, Milchsäure, Benzoesäure, Salicylsäure, Kaliumsorbat.

Die hier genannten Inhaltstoffe können vom Fachmann mit anderen kosmetischen Inhaltstoffen kombiniert werden.

Die Menge der Konservierungsmittel in den erfindungsgemäßen Zusammensetzungen beträgt 0.001 — 10 Gew.-%, vorzugsweise 0.01 - 5 Gew.-% und insbesondere 0.1 - 3 Gew.-%, bezogen auf die Gesamtmenge der Zubereitung. In einer bevorzugten Ausführungsvariante ist die Zubereitung konservierungsmittelfrei.

**UV-Filter**

Es ist auch möglich und vorteilhaft im Sinne der vorliegenden Erfindung, einen Gehalt an UV-Schutzsubstanzen zu verwenden. Die erfindungsgemässe Zubereitung eignet sich insbesondere, da die Pigmente in der Kombination der Tenside wie offenbart ausserordentlich gut dispergiert werden. Neben dem Effekt, dass dadurch Pigmente gut von einem Substrat gelöst werden können, hat dies gleichzeitig den Effekt, dass Zubereitungen mit Farbe oder Pigmenten stabilisiert werden. Beispiele für pigmentäre UV-Filter sind Zinkoxid, Titandioxid, Methylene Bis-Benzotriazolyl Tetramethylbutylphenol, u.a.

Vorteilhaft können daher erfindungsgemässe Zubereitungen Substanzen enthalten, die UV-Strahlung im UVA und UVB-Bereich absorbieren, wobei die Gesamtmenge der Filtersubstanzen an der gesamten Zubereitung vorzugsweise zwischen 0.1 Gew.-% bis 30 Gew.-% betragen kann. Bevorzugt sind handelsübliche wasser- oder öllösliche UV-Filter. Üblicherweise werden Kombinationen von UV-Filtern eingesetzt, die vom Fachmann mit anderen hier genannten Inhaltstoffen kombiniert werden können.

**Antischuppenmittel**

Das kosmetische Mittel kann Komponenten enthalten, die gegen Kopfschuppen wirksam sind. Beispiele hierfür sind Zinkpyrithion, Selendisulfid, Piroctone Olamin, Climbazol oder auch Pflanzenextrakte wie Vitis Vinifera Seed Extract, Thymus Serpyllum Extrakt, Rosmarinus officialis Leaf Extrakt, Leuconostoc / Radish Root Ferment Filtrate, Leptospermone/ Isoleptospermone/ Flavesone, Leptospermum Scoparoi, Branch/ Leaf Oil, Fragaria ananassa (Strawberry) Seed oil, Adianthum Capillus Veneris Leaf Extrakt, Brennessel Extrakt, Wacholder Extrakt, Thymian Extrakt und andere.

In den erfindungsmässigen Zubereitungen mit Anti-Schuppen-Wirkstoff werden diese in Konzentrationen bis zu 7 Gew.-%, bevorzugt 0.1- 4 Gew.-%, besonders bevorzugt 0.1- 2 Gew.-% eingesetzt, jeweils bezogen auf die Gesamtmenge der Zubereitung.

**Farb-, Duft- und Aromastoffe**

Um den ästhetischen bzw. organoleptischen Eindruck der erfindungsgemässen Zubereitungen zu verbessern, können alle in Kosmetika üblichen Duftstoffe zugesetzt werden.
Bevorzugte Duftstoffe, deren Auswahl dem Fachmann keinerlei Schwierigkeit bereitet, besitzen eine hohe Lagerstabilität

und Unempfindlichkeit gegenüber den übrigen Inhaltsstoffen der kosmetischen Zubereitung.

Als Parfümöle können einzelne Duftstoffverbindungen, wie z.B. Ester, Ether, Aldehyde, Ketone, Alkohole und Kohlenwasserstoffe verwendet werden.

Duftstoffverbindungen auf Basis von Estern sind z.B. Phenoxyethylisobutyrat, Benzylacetat, p-tert.-Butylcyclohexylacetat, Dimethylbenzylcarbinylacetat, Linalylacetat, Phenylethylacetat, Linalylbenzoat, Ethylmethylphenylglycinat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. die Ionone $\alpha$-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet.

Geeignete Parfümöle können auch natürliche Duftstoffgemische aus pflanzlichen oder tierischen Quellen sein, z.B. Pinien-, Citrus-, Jasmin-, Lilien-, Rosen- oder Ylang-Ylang-Öl. Auch ätherische Öle eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Laudanumöl.

Besonders bevorzugt werden Farbstoffe und Duftstoffe, welche in der Literatur nicht als Allergene beschrieben werden und auf natürlichen Rohstoffen basieren, wie zum Beispiel Extrakte aus Pflanzen.

Als Aromaöle kommen alle gebräuchlichen natürlichen und synthetischen Aromen in Frage. Bevorzugt in der Ausführungsform der Zahnpflegeprodukte sind Aromaöle aus der Gruppe Pfefferminzöl, Krauseminzöl, Anisöl, Kümmelöl, Eukalyptusöl, Fenchelöl, Zimtöl, Geraniumöl, Salbeiöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

In einer weiteren bevorzugten Ausführungsform der Erfindung, werden keinerlei Duftstoffe zugesetzt, beispielsweise für Anwendungen bei Konsumenten mit Allergien und/oder sensibler Haut.

Die erfindungsgemäßen Zubereitungen enthalten in der parfümierten Ausführungsvariante die Duftstoffkomponente/n in Mengen von bis zu 5 Gew.-%, bevorzugt 0.1 - 3 Gew.-%, besonders bevorzugt 0.2 - 2 Gew.-%; jeweils bezogen auf die Gesamtmenge der Zubereitung; in der unparfümierten Ausführungsform sind keinerlei Duftsstoffe enthalten.

**Wirkstoffe**

*Anti-Karies*

Eine weitere bevorzugte Gruppe von Inhaltsstoffen, die in den erfindungsgemäßen Mitteln enthalten sein kann, sind Antikaries-Wirkstoffe. Diese können beispielsweise aus organischen oder anorganischen Fluoriden ausgewählt sein, z. B. aus Natriumfluorid, Kaliumfluorid, Natriummonofluorphosphat, Natriumfluorosilikat, Zinkfluorid und Zinn-(II)-fluorid. Bevorzugt sollte eine Menge von 0.01 - 0.5 Gew.-% Fluor in Form der genannten Verbindungen enthalten sein.

*Wundheilende und entzündungshemmende Wirkstoffe*

Die kosmetische Zubereitung kann weitere Wirkstoffe z. B. gegen Zahnfleischentzündungen, Hautentzündungen, insbesondere bei erkrankter oder beanspruchter Haut wie z.B. während der Rasur enthalten. Derartige Stoffe können z. B. ausgewählt sein aus Acetylsalicylsäurederivate, Allantoin, Aloe Vera, Azulen, Harnstoff, Kamillenextrakten, Tocopherol, Panthenol, Pantothensäure Bisabolol, Propolis , Rhodanid, Retinol, Salbeiextrakten. Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Weiterhin können halogenierte Diphenylether, z.B. 2,4-Dichlor-2-hydroxydiphenylether, 4,4-Dichlor-2-hydroxydiphenylether, 2,4,4-Tribrom-2-hydroxydiphenylether und 2,4,4-Trichlor-2-hydroxydiphenylether (Triclosan) verwendet werden. Eingesetzt werden können zahlreiche Pflanzenextrakte, wie einige Beispiele stellvertretend für die Klasse der Pflanzenextrakte in den Ausführungsbeispielen offenbart.

Diese Wirkstoffe werden bevorzugt in Mengen von 0.01- 5 Gew.-% in die erfindungsgemäßen Zubereitungen eingesetzt, Gew.-% bezogen auf die Gesamtmenge der Zubereitung.

*Anti-Akne Wirkstoffe*

Das Mittel kann weiterhin übliche Wirkstoffe gegen Akne enthalten, wie beispielsweise Azelainsäure, Antibiotika, Retinoide, Zinc Oxid, $\alpha$-Hydroxysäuren, Linolsäure, pflanzliche Extrakte wie z.B. Kamillenextrakte, Teebaumöl, Grüner Tee und andere.
Sie werden bevorzugt in Mengen von 0.01- 5 Gew.-% in die erfindungsgemäßen Zubereitungen eingesetzt, Gew.-% bezogen auf die Gesamtmenge der Zubereitung.

*Weitere Wirkstoffe*

Weiterhin können andere Wirkstoffe in dem kosmetischen Mittel enthalten sein, wie beispielsweise Anti-Ageing-Wirkstoffe, Peptide, UV-Absorber, Vitamine & Mineralstoffe (z.B. Ascorbinsäure, Biotin, Tocopherol oder Rutin, Niacin), Mineralsalze wie z.B. Mangan-, Zink- oder Magnesiumsalze, weitere pflanzliche Extrakte.
Sie werden bevorzugt in Mengen von 0.01- 5 Gew.-% in die erfindungsgemäßen Zubereitungen eingesetzt, Gew.-% bezogen auf die Gesamtmenge der Zubereitung.

**Haarfarben**

Die erfindungsgemässen Zubereitungen können auch Farbstoffe zur direkten oder oxidativen Färbung von Haaren enthalten. Die Färbezubereitungen sollen das Haar schützen und möglichst wenig schädigen. Auf Basis der erfindungsgemässen Zubereitungen können sowohl Haarfärbemittel als auch Tönungsshampoos hergestellt werden.
In einer weiteren bevorzugten erfindungsgemäßen Ausführungsform enthält die Zubereitung mindestens ein Farbstoffvorprodukt und/oder mindestens einen Farbstoff zur Färbung keratinischer Fasern, insbesondere zur Färbung menschlicher Haare.
Die Zubereitung eignet sich hierfür insbesondere, da die Pigmente und Farben in der Kombination der Tenside wie offenbart ausserordentlich gut gelöst bzw. dispergiert werden. Der Überschuss an Farbstoffen und Pigmenten, welche nach dem Färbevorgang noch auf Haar und Kopfhaut verblieben sind, können dank der erfindungsgemässen Zubereitung in einem Schritt ausgewaschen werden ohne dass ein zusätzliches Nachwaschen zum Ausspülen der Farbreste notwendig ist.
Als Farbstoff(vorprodukt)e können

- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,

sowie Mischungen von Vertretern einer oder mehrerer dieser Gruppen eingesetzt werden.
Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxa decan sowie 4,5-Diaminopyrazol-Derivate wie z.B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.
Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind

- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,

- o-Aminophenol und dessen Derivate,

- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,

- o-Diaminobenzol und dessen Derivate wie beispielsweise 3,4-Diaminobenzoesäure und 2,3-Diamino-1-methylbenzol,

- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,

- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,

- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,

- Morpholinderivate wie beispielsweise 6-Hydroxybenzomorpholin und 6-Aminobenzomorpholin,

- Chinoxalinderivate wie beispielsweise 6-Methyl-1,2,3,4-tetrahydrochinoxalin,

- Pyrazolderivate wie beispielsweise 1-Phenyl-3-methylpyrazol-5-on,

- Indolderivate wie beispielsweise 4-Hydroxyindol, 6-Hydroxyindol und 7-Hydroxyindol,

- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.
Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.
Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.
Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z.B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können sowohl als freie Basen als auch in Form ihrer physiologisch verträglichen Salze mit anorganischen oder organischen Säuren, z.B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere alpha-Aminocarbonsäuren und Omega-Aminocarbonsäuren. Unter den alpha-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise über pH-Stellmittel schwach sauer bis alkalisch, d.h. auf pH-Werte im Bereich von etwa 5 bis 11, eingestellt.

Erfolgt die Ausbildung der eigentlichen Haarfarben im Rahmen eines oxidativen Prozesses, so können übliche Oxidationsmittel verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen. Bevorzugt sind dabei Oxidasen wie Tyrosinase, Ascorbatoxidase und Laccase aber auch Glucoseoxidase, Uricase oder Pyruvatoxidase.

Zweckmäßigerweise wird die Zubereitung des Oxidationsmittels unmittelbar vor dem Färben der Haare mit der erfindungsgemässen Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Nach einer Einwirkungszeit von ca. 5 bis 45, insbesondere 15 bis 30, Minuten wird das Haarfärbemittel durch Ausspülen von dem zu färbenden Haar entfernt.

Weiterer Vorteil der erfindungsgemässen Zubereitung ist, dass das Nachwaschen mit einem Shampoo entfallen kann, da die Farbstoffzubereitung bereits tensidhaltig ist.

Besonders bevorzugte erfindungsgemäße Ausführungsformen sind dadurch gekennzeichnet, dass die Zubereitung nicht gleichzeitig Oxidationsmittel und (davon verschiedene) Reduktionsmittel enthält.

**Bindemittel/ Konsistenzgeber**

Die erfindungsgemässe Zubereitung kann als Bindemittel bzw. Konsistenzregler dienen z. B. natürliche und/oder synthetische wasserlösliche Polymere wie Alginate, Carrageenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere Polyvinylalkohol, Polyvinylpyrrolidon, Polyethylenglycole.

Sie werden bevorzugt in Mengen von 0,01- 10 Gew.-% in die erfindungsgemäßen Zubereitungen eingesetzt, Gew.-% bezogen auf die Gesamtmenge der Zubereitung.

**Viskosität**

Die erfindungsgemässe Zubereitung kann alle in der Kosmetik üblichen, dem Fachmann bekannten, Viskositätsregulatoren enthalten und können mit anderen kosmetischen Inhaltsstoffen in der erfindungsgemässen Zubereitung kombiniert werden. Die Viskosität wird je nach Anwendung auf die dem Fachmann bekannten und im Markt üblichen Werte eingestellt.

Geeignete Viskositätsregulatoren sind beispielsweise organische abgewandelte Naturstoffe (Carboxymethylcellulose und andere Celluloseether, Hydroxyethyl- und - propylcellulose und dergleichen, Kernmehlether), organische vollsynthetische Verdickungsmittel (Polyacryl- und Polymethacryl-Verbindungen, Vinylpolymere, Polycarbonsäuren, Polyether, Polyimine, Polyamide) und anorganische Verdickungsmittel (Polykieselsäuren, Schichtsilikate, Tonmineralien wie Montmorillonite, Zeolithe, Kieselsäuren), sowie organische natürliche Verdickungsmittel (Agar-Agar, Carrageen, Xanthan, Traganth, Gummi arabicum, Alginate, Pektine, Polyosen, Guar-Mehl, Johannisbrotbaumkernmehl, Stärke, Dextrine, Gelatine, Casein) und Mischungen derselben.

Bevorzugte Viskositätsregulatoren sind natürliche organische Verdickungsmittel aus pflanzlichen Rohstoffen - einschliesslich Algen - beispielsweise Polysaccharide wie Pektine oder Stärke. Des Weiteren bevorzugt sind biotechnologisch hergestellte Verdickungsmittel mithilfe von nicht genmodifizierten Organismen (non GMO), wie beispielsweise Xanthan. Bevorzugt sind zudem mineralische Verdickungsmittel wie Polykieselsäuren, Schichtsilikate, Tonmineralien wie Montmorillonite, Zeolithe, Kieselsäuren.

Bevorzugt werden in den kosmetischen Zubereitungen bis zu 5 Gew.-% der Verdickungsmittel eingesetzt, besonders bevorzugt 0.01- 3 Gew.-%, bezogen auf die Gesamtmenge der Zubereitung.

**Schaum**

Generell zeigen die erfindungsgemässen Zubereitungen eine geringere Schaumbildung als herkömmliche Mittel auf Basis kurzkettiger Tenside. Die geringe Schaumbildung ist für viele Verwendungen wünschenswert, wie zum Beispiel die Gesichtspflege.

Eine besonders bevorzugte Ausführungsform, bei der Schaumbildung auftritt, ist die Zugabe des alkoxylierten Tensids (C) oder die Zugabe eines Chelatbildners, wie oben ausgeführt.

Weiterhin können den erfindungsgemässen Zubereitungen zusätzliche Schaumbildner zugegeben werden. Die Schaumbildung bei Dusch- und Waschzubereitungen wird durch die Wahl der Tenside, wie z.B. Tensid (C) beeinflusst. Geeignet sind ausserdem bekannte Kombinationen mit stark schäumenden Tensiden, beispielsweise Aminosäurederivate von Fettsäuren, wie Glycinate, Alanate, Taurate, oder Sarcosinate. Geeignet für eine gute Schaumbildung sind zudem amphotere Tenside, wie zum Beispiel Betaine, Imidazolcarboxylate oder Alkenylaminopropionsäuren.

Eine weitere bevorzugte Ausführungsform der erfindungsgemässen kosmetischen Zubereitung enthält Saponine als Schaumbildner. Saponine sind natürliche Glykoside, die als Pflanzeninhaltsstoffe weit verbreitet sind. Geeignet sind beispielsweise Saponine aus der indischen Waschnuss (Sapindus mukorossi), Koreanischen Ginsengs (Panax ginseng), Agavengewächsen, Inka-Gurke (Cyclanthera pedata), Süssholz *(Glycyrrhiza glabra)* und Seifenrinde (*Quillaja saponaria* Molina) und Mischungen derselben.

Besonders bevorzugt werden Saponine, die aufgrund ihres Vorkommens keine langen Transportwege erfordern, in der erfindungsgemässen Zubereitung eingesetzt. Hierbei handelt es sich um Saponine aus folgenden bevorzugten Pflanzen: Efeu (Hedera), Schlüsselblume (Primula veris), Vogelmiere (Stellaria media), Wald-Sanickel (Sanicula europaea), Dornige Hauhechel (Ononis spinosa), Hülsenfrüchten (Leguminosae), Spinat (Spinacia), Spargel (Asparagaceae), Hafer (Avena), (Ononis spinosa), Schattenblümchen (Maianthemum bifolium), Seifenkraut (Saponaria officinalis), Walnuss (Aesculus hippocastanum), Acker-Gauchheil (Anagallis arvensis), Gelber Hohlzahn (Galeopsis segetum), Karthäuser-Nelke (Dianthus carthusianorum), Ackerschachtelhalm (Equisetum arvense) und Mischungen derselben.

Die Menge an Saponinen beträgt üblicherweise bis zu 5 Gew.-%, vorzugsweise 0.001 bis 3 Gew.-%, insbesondere 0.01 bis 2 Gew.-%, bezogen auf die Gesamtmenge der Zubereitung.

**Ölkörper**

Die erfindungsgemässe Zubereitung kann übliche, dem Fachmann bekannte Ölkörper enthalten. Zu Ölkörpern zählen wasserunlösliche, organische Verbindungen, wobei die Wasserunlöslichkeit erfindungsgemäß als eine Löslichkeit von weniger als 10 Gew.-% bei 20° C verstanden wird.

Zu den erfindungsgemäß einsetzbaren Ölkörpern zählen beispielsweise Glyceride, Kohlenwasserstoffe, Silikonöle, Dialkylether, Dialkylcarbonate, Wachsester, etc. Auch beliebige Gemische von Ölkörpern sind erfindungsgemäß einsetzbar. Glyceride sind Fettsäureester des Glycerins, die natürlicher (tierischer, mikrobieller, algenbasierter und pflanzlicher) oder synthetischer Herkunft sein können. Man unterscheidet zwischen Mono-, Di- und Triglyceriden. Es handelt sich um bekannte Stoffe, die nach einschlägigen Verfahren der präparativen organischen Chemie hergestellt werden können. Synthetisch hergestellte Glyceride sind üblicherweise Mischungen von Mono-, Di- und Triglyceriden, die durch Umesterung der entsprechenden Triglyceride mit Glycerin oder durch gezielte Veresterung von Fettsäuren erhalten werden. Triglyceride oder Glycerid-Gemische mit einem hohen Triglycerid-Anteil (> 90 Gew.-%) sind bevorzugt. Als Fettsäure sind erfindungsgemäß C6-C30-Fettsäuren geeignet. Die Fettsäuren können verzweigt oder unverzweigt, hydroxyliert oder nicht-hydroxyliert, gesättigt oder ungesättigt sein. Erfindungsgemäß bevorzugt ist die Verwendung Glyceride pflanzlicher Herkunft, besonders bevorzugt sind Glyceride der C-18-Pflanzen, z.B. Sojaöl, Baumwollsaatöl, Sonnenblumenöl, Leinöl, Mandelöl, Maisöl, Olivenöl, Rapsöl, Sesamöl, Distelöl, Weizenkeimöl, Pfirsichkernöl und dergleichen. Exemplarisch sind einige Zubereitungen mit Ölkörpern in den Ausführungsbeispielen offenbart.

Zu den erfindungsgemäß einsetzbaren Ölkörpern zählen auch natürliche und synthetische, aliphatische und/oder naphthenische Kohlenwasserstoffe, wie z. B. Squalan, Squalen, Paraffinöle, Isohexadecan, Isoeicosan oder Polydecene sowie Dialkycyclohexane.

Erfindungsgemäß sind als Ölkörper auch Siliconöle geeignet. Zu diesen zählen z. B. Dialkyl- und Alkyl- arylsiloxane, wie beispielsweise Cyclomethicone, Dimethylpolysiloxan und Methylphenylpolysiloxan, sowie deren alkoxylierte und quaternisierte Analoga. Geeignete nicht-flüchtige Siliconöle, wie z. B. Polyalkylsiloxane, Polyalkylarylsiloxane und Polyethersiloxan-Copolymere sind in Cosmetics: Science and Technology, Hrsg.: M. Balsam und E. Sagarin, Bd. 1, 1972, S. 27-104 zu entnehmen.
Siliconöle sind aus Nachhaltigkeitsbetrachtungen in den erfindungsgemässen Zubereitungen nicht bevorzugt.

Geeignete und erfindungsgemäß bevorzugte Öle sind weiterhin verzweigte gesättigte oder ungesättigte Fettalkohole mit 6 — 30 Kohlenstoffatomen. Diese Alkohole werden häufig auch als Guerbet-Alkohole bezeichnet, da sie nach der Guerbet-Reaktion erhältlich sind. Beispiele bevorzugter Alkoholöle sind Hexyldecanol, Octyldodecanol, 2-Ethylhexylalkohol und die Handelsprodukte Guerbitol® 18, Isofol® 12, Isofol® 16, Isofol® 24, Isofol® 36, Isocarb® 12, Isocarb® 16 oder Isocarb® 24.

Weitere geeignete Ölkomponenten sind Mischungen aus Guerbetalkoholen und Guerbetalkoholestern, z.B. Hexyldecanol und Hexyldecyllaurat.

Als Ölkörper kommen auch Ester von linearen, gesättigten oder ungesättigten C6-C30-Fettsäuren mit linearen oder verzweigten, gesättigten oder ungesättigten C6-C30-Fettalkoholen bzw. Ester von verzweigten C6-C30-Carbonsäuren mit linearen oder verzweigten, gesättigten oder ungesättigten C6-$C_{30}$-Feftalkoholen, welche hydroxyliert sein können (sogenannte Wachsester) in Frage.

Unter den Wachsestern seien expemplarisch folgende Vertreter genannt: Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearyl- myristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearyl- behenat, Isostearyloleat, Oleylmyristat, Oleylpalmitat, Oleylstearat, Oleylisostearat, Oleyloleat, Oleylbe- henat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Vorzugsweise werden ein- oder mehrfach ungesättigte Wachsester, wie beispielsweise Oleyloleat und Oleylerucat eingesetzt. Insbesondere bevorzugt sind diese Wachsester abgeleitet aus C18-Pflanzen. Daneben eignen sich auch Ester von linearen C6-C22-Fettsäuren mit verzweigten Alkoholen, insbesondere 2-Ethylhexanol, Ester von C2-C38-Alkylhydroxycarbonsäuren mit linearen oder verzweigten C6-C22-Fettalkoholen, Ester von linearen und/oder verzweigten Fettsäuren mit mehrwertigen Alkoholen (wie z. B. Propylenglycol, Dimerdiol oder Trimertriol) und/oder Guerbetalkoholen, sowie Ester von C6-C22-Fettalkoholen und/oder Guerbet- alkoholen mit aromatischen Carbonsäuren, insbesondere Benzoesäure, Ester von C2-C12- Dicarbonsäuren mit linearen oder verzweigten Alkoholen mit 1 bis 22 Kohlenstoffatomen (z. B. Dioctyl Malate) oder Polyolen mit 2 bis 10 Kohlenstoffatomen und 2 bis 6 Hydroxylgruppen.
Weitere beispielhafte Vertreter sind: Hexyldecylstearat , Hexyldecyllaurat, Isodecylneopentanoat, Isononylisononanoat, 2-Ethylhexylpalmitat und 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Ethylenglycoldioleat und -dipalmitat, Linolylmyristat, Linolylpalmitat, Linoleylmyristat, Linoleylpalmitat, Isostearyloleat, Isostearylbehenat, Isostearyloleat, , Linoylstearat, Linoleylstearat, Oleyloleat, Oleyllinolat, Oleyllinolenat, Oleylrizinat, Oleylerucat, Linolyloleat Linolyllinolat, Linolylrizinat, Linolylerucat, Linolenyloleat, Linolenyllinolat, Linolenyllinenolat, Linolenylrizinat, Linolenylerucat, Erucyllinolat, Erucyllinolenat, Erucylrizinat, wobei die Linolenderivate alpha-, sowie gamma-Linolenderivate einschliessen.

Als Ölkörper geeignet sind lineare oder verzweigte, symmetrische oder unsymmetrische, gesättigte oder ungesättigte Di-n-alkyl(en)ether mit 12 bis 24 C-Atomen pro Alkyl(en)gruppe, wie z. B. Di-n-octylether, Di-(2-ethylhexyl)-ether, Laurylmethylether oder Octylbutylether, Didodecylether oder Dibehenylether, wobei bevorzugt Ether abgeleitet aus C18-Pflanzen verwendet werden.

Besonders bevorzugt sind als Ölkörper lineare oder verzweigte, gesättigte oder ungesättigte C6-C40-Fettalkoholcarbonate geeignet. Diese Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in Chem.Rev. 96, 951 (1996). Typische Beispiele für Dialkyl(en)carbonate sind vollständige oder partielle Umesterungsprodukte von Dimethyl- und/oder Diethylcarbonat mit Capronalkohol, Caprylalkohol, 2-Ethylhexylalkohol, Caprinalkohol, Laurylalkohol, Isotride- cylalkohol, Myristylalkohol, Cetylalkohol, Palmoleylalkohol, Stearylalkohol, Isostearylalkohol, Oleylalko- hol, Elaidylalkohol, Petroselinylalkohol, Linolylalkohol, Linolenylalkohol, Elaeostearylalkohol, Arachidyl- alkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen anfallen. Bevorzugt geeignet Dialkylcarbonate abgeleitet von C-18-Pflanzenölen.

Weitere erfindungsgemäß geeignete Öle sind ausgewählt aus den Dicarbonsäureestern von linearen oder verzweigten $C_2$-$C_{10}$-Alkanolen, insbesondere Diisopropyladipat, Di-n-butyladipat, Di-(2-ethylhexyl)adipat, Dioctyladipat, Diethyl-/Din-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weiterhin geeignet sind Ölkörper aus Ester von linearen oder verzweigten, gesättigten oder ungesättigten Fettalkoholen mit 2-30 Kohlenstoffatomen mit linearen, oder verzweigten gesättigten oder ungesättigten Fettsäuren mit 2 — 30 Kohlenstoffatomen, die hydroxyliert sein können.

Weitere Öle, sind ausgewählt aus den Anlagerungsprodukten von Ethylenoxid und/oder Propylenoxid an ein- oder mehrwertige $C_{3-20}$-Alkanole wie Butanol, Butandiol, Myristylalkohol und Stearylalkohol, z. B. PPG-14-Butylether, PPG-9-Butylether, PPG-10-Butandiol, PPG-3-Myristylether und PPG-15-Stearylether.

Vorzugsweise werden Ölkörper eingesetzt, die auf C-18-Pflanzen basieren, wie beispielsweise Pflanzenöle und entsprechende Mono-/Di- /Triglyceridmischungen auf Basis von gesättigten und ungesättigten C6-C24-Fettsäuren, Ester von C6-C24-Fettalkoholen und/oder Fettalkohole mit Carbonsäuren oder Polyolen, wie zum Beispiel Zucker mit 2 bis 10 Kohlenstoffatomen und 2 bis 6 Hydroxylgruppen.

In einer bevorzugten Ausführungsform werden keine Silikone eingesetzt.

*Öle, Fette und Wachse*

Typische Beispiele für Fette und Öle sind Glyceride, d. h. feste oder flüssige pflanzliche oder tierische Produkte, die im Wesentlichen aus gemischten Glycerinestern höherer Fettsäuren bestehen. Als Wachse kommen u. a. natürliche Wachse, wie z. B. Bienenwachs, Bürzelfett, Candelillawachs, Carnaubawachs, Ceresin, Espartograswachs, Guarumawachs, Japanwachs, Korkwachs, Lanolin (Wollwachs), Mikrowachse, Montanwachs, Ozokerit (Erdwachs), Petrolatum, Paraffinwachse, Ouricourywachs, Reiskeimölwachs, Schellackwachs, Sonnenblumenwachs, Fruchtwachse wie Orangenwachse, Zitronenwachse, Grapefruitwachse, Zuckerrohrwachs, chemisch modifizierte Wachse (Hartwachse), wie z. B. Montanesterwachse, Sasolwachse, hydrierte Jojobawachse, hydriertes Rinzinusöl sowie synthetische Wachse, wie z. B. Polyalkylenwachse und Polyethylenglycolwachse in Frage.

Neben den Fetten kommen als Zusatzstoffe auch fettähnliche Substanzen, wie Lecithine und Phospholipide in Frage. Als Beispiele für natürliche Lecithine seien die Kephaline genannt. Daneben kommen auch Sphingosine bzw. Sphingolipide in Frage. Desweiteren sind auch Mischungen der genannten Öle und Wachse möglich. Erfinderisch besonders bevorzugt eingesetzt werden Pflanzenöle, bzw. Triglyceride, wie sie aus den Pflanzen der gemässigten Zonen isoliert werden können. Ganz besonders bevorzugt werden Öle der Pflanzen und Pflanzenfamilien wie sie in der Anmeldung im Abschnitt "C18-Pflanzen" beschrieben wurden.

Die Ölkörper können in den erfindungsgemässen Zubereitungen in einer Menge von 0.2 bis 80 Gew.-%, bevorzugt Mengen von 0.2 bis 70 Gew.-%, bezogen auf die Gesamtmenge der Zubereitung.

*Perlglanzwachse*

Als Perlglanzwachse kommen beispielsweise in Frage: Alkylenglycolester, speziell Ethylenglycoldistearat; Fettsäurealkanolamide, Partialglyceride, Ester von mehrwertigen, gegebenenfalls hydroxysubstituierte Carbonsäuren mit Fettalkoholen mit 6 bis 24 Kohlenstoffatomen, Fettstoffe, wie beispielsweise Fettalkohole, Fettketone, Fettaldehyde, Fettether und Fettcarbonate, die in Summe mindestens 24 Kohlenstoffatome aufweisen; Fettsäuren wie Stearinsäure, Hydroxystearinsäure oder Behensäure, Ringöffnungsprodukte von Olefinepoxiden mit 12 bis 24 Kohlenstoffatomen mit Fettalkoholen mit 12 bis 24 Kohlenstoffatomen und/oder Polyolen mit 2 bis 15 Kohlenstoffatomen und 2 bis 10 Hydroxylgruppen sowie deren Mischungen.

Erfindungsgemäss bevorzugt sind Perlglanzwachse basierend auf C18-Pflanzen.

Die Einsatzmenge der Perlglanzwachse kann - bezogen auf die Gesamtmenge der Zubereitung - bei 0.1 bis 5, vorzugsweise 0.5 bis 3 und insbesondere 1 bis 1.5 Gew.-% liegen.

**Weitere Inhaltsstoffe**

Neben den bisher genannten Komponenten können die erfindungsgemässen Zubereitungen weitere übliche Inhaltsstoffe für Kosmetika enthalten, die dem Fachmann durchaus bekannt sind.

Die erfindungsgemässen kosmetischen und dermatologischen Zubereitungen können dementsprechend ferner kosmetische Hilfsstoffe enthalten, wie sie üblicherweise in solchen Zubereitungen verwendet werden; beispielsweise, Desensibilisierende Zusätze (z.B. Hydroxylapatit, Arginin mit Calciumcarbonat, Zinn-, Kalium-, Strontiumchlorid, Kaliumnitrat u.a.), Konsistenzgeber, kationische Polymere, Filmbildner, Überfettungsmittel, Stabilisatoren, biogene Wirkstoffe, Trübungsmittel, ferner Eiweissderivate wie Gelatine, Collagenhydrolysate, Aminosäuren, Oligo- oder Polypeptide auf natürlicher und synthetischer Basis, DNA- oder RNA-Oligonucleotide, Desoxyzucker oder Desoxyzucker-Bausteine enthaltende Polysaccharide, Kreatin, Xanthin-Derivate, z.B. Coffein, Theophyllin, Theobromin, Aminophyllin, Eigelb, Honig, Lanolin und Lanolinderivate, Stoffe mit keratolytischer und keratoplastischer Wirkung, Enzyme und Trägersubstanzen, Pflanzenextrakte, Vitamine, Provitamine und deren Ester, Antiageing- Mittel, Füllstoffe, Pigmente, , Suspendiermittel, Puffergemische, oberflächenaktive Substanzen, weichmachende, anfeuchtende und/oder feuchthaltende Substanzen, entzündungshemmende Substanzen, Meristem Wirkstoffe, Flavonoide und'flavonoid-reiche Pflanzenextrakte, Isoflavonoide und isoflavonoid-reiche Pflanzenextrakte, Ubichinon und ubichinonreiche Pflanzenextrakte, Silymarin, selbstbräunende Wirkstoffe, haarentfernende Wirkstoffe und haarwachstumanregende bzw. haarwuchsregulierende Wirkstoffe, sebumregulierende Wirkstoffe, hautaufhellende Wirkstoffe oder Weissmacher in Zahnpasten (z.B. Phosphate oder Papain), Lichtschutzmittel, Insektenrepellentien, Bakterizide, Salze, antimikrobiell, proteolytische oder wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte, sowie Gemische derselben.

Diese sind vorzugsweise in einer Menge von 0,001 - 20 Gew.-% in der Zubereitung enthalten.

## Verfahren

Ein weiterer Gegenstand der Erfindung richtet sich auf ein Verfahren zur Reinigung und/oder Pflege. Verfahren zur Reinigung und Pflege zeichnen sich im allgemeinen dadurch aus, dass in mehreren Verfahrensschritten verschieden aktive Substanzen auf den Körper, bzw. das Körperteil oder auf ein Substrat, z.B. ein Textil, ein Tuch, eine Bürste etc. aufgebracht und nach der Einwirkzeit abgewaschen werden, oder dass der Körper, der Mund, die Zähne, die Haare, das Fell oder die Haut in sonstiger Weise mit der erfindungsgemässen Zubereitung behandelt wird.

Das Reinigungs- und/oder Pflegeverfahren umfassend die Verfahrensschritte:

a) Bereitstellen einer Zubereitung umfassend ein Reinigungs- oder Pflegemittel gemäss den vorhergehenden Beschreibungen

b) In Kontakt bringen von Körper, Zähne, Zahnfleisch, Haaren oder Fell mit dem Reinigungs- oder Pflegemittel gemäss a)

In einem weiteren Verfahren wird das Mittel auf feste Substrate, wie Tücher aus Textil, Verbundstoff, non woven, Vlies, Papier, Watte oder Filz u.a. aufgebracht. Diese werden mit der Zubereitung durch ein Press-, Tauch-, Abstreif- oder Sprühverfahren imprägniert.

Alle Sachverhalte, Gegenstände und Ausführungsformen, die für die Zubereitung beschrieben sind, sind auch auf das Reinigungs-und Pflegeverfahren sowie deren Verwendung anwendbar und umgekehrt.

## Verwendungen

Gegenstand dieser Anmeldung ist die nicht-therapeutische Verwendung der erfindungsgemässen Zubereitungen als oder zur Herstellung von Reinigungs- und Pflegemitteln für Körper, Mund und Zähne, Haut und Haare sowie zur Tierpflege.

Wie oben beschrieben, betrifft die Erfindung insbesondere die Verwendung der Zubereitung zur Entfernung von Farb- oder Kohlenhydratverschmutzungen.

In einer bevorzugten Ausführungsform wird die Zubereitung in bei saurem pH zwischen 0 und 7, bevorzugt zwischen 1 und 6 eingesetzt und ganz besonders bevorzugt bei einem pH zwischen 2 und 5.5. Überraschenderweise zeigt die erfindungsgemässe Zubereitung eine ausgesprochen gute Eignung im Sauren. Das erfindungsgemässe Mittel eignet sich dadurch für die Verwendung in Haarentfernern, medizinischen Produkten wie zum Beispiel gegen Schuppenflechte oder Neurodermitis, insbesondere zu deren Prävention, weiterhin für Haarfärbeprodukte und für die Verwendung im Intimbereich.

In einer weiteren bevorzugen Ausführungsform wird die Zubereitung bei neutralem pH zwischen 5 und 8 eingesetzt, z.B. wenn ein hautneutraler pH wünschenswert ist, wie beispielsweise in einer Handseife, der Gesichtpflege, Gesichtswasser, AugenMakeup-Entferner, Makeup Entferner, Shampoo, Duschzubereitungen, Kontaktlinsenreinigung und andere.

In einer anderen bevorzugten Ausführungsform wird das Mittel bei alkalischem pH zwischen 7 und 14 eingesetzt, bevorzugt zwischen 8 und 12 eingesetzt. Typische Beispiele für die Verwendungen bei alkalischem pH sind Rasierseife, Haarfärbemittel, Hautreinigung auf Seifenbasis.

Die erfindungsgemässen Zubereitungen werden als oder für die Herstellung von Reinigungs- und Pflegemitteln verwendet, bevorzugt als kosmetisches, nichttherapeutisches dermatologisches oder medizinisches Reinigungs- und/oder Pflegemittel, welches direkt oder indirekt mit dem menschlichen Körper in Berührung kommt. Dies sind beispielsweise: Duschzubereitungen, Badezusätze, Handseifen, auch zur Grobreinigung, Handpasten, Intimreinigung, Reinigung des Augenbereichs, Kontaktlinsenreinigung, Makeup-Entferner, Augenmakeup-Entferner, Körperreinigung, Mund- und Zahnpflege, Haarshampoos, konditionierende Shampoos, Antischuppen-Shampoo, Babyshampoo, Reinigungs- und Pflegeprodukte bei Schuppenflechte, Neurodermitis, Akne; insbesondere für die Prävention, Körper-, bzw. Gesichtstonic, Gesichtslotion, Feuchttücher, auch mit pflegenden oder anderen Wirkstoffen, z.B. desodorierend, antiageing u.a., Haarentfernung und Rasierprodukte, Haar-Tonicen, Haarfärbeshampoos, Haarfärbemittel und Haarstyling-Zubereitungen, Mundpflegeprodukt (Mundhygieneprodukt), insbesondere in Form von Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Mundspray.

Eingeschlossen ist erfindungsgemäss die Tierpflege und -reinigung.

Im Sinne dieser Anmeldung kann das Mittel als Flüssigkeit, Lösung oder Dispersion, Emulsion, Lotion, Gel, Spray oder Schaum verwendet werden. So können sie z. B. eine Lösung, eine Emulsion (O/W), oder eine multiple Emulsion, beispielsweise vom Typ Wasser-in-Öl-in-Wasser (W/O/W), ein Gel, eine Hydrodispersion, eine lamellare Phase, eine flüssige isotrope Lösungsphase oder eine micellare Phase, darstellen; bevorzugt ist die flüssige oder gelförmige Ausführungsform mit Wasser oder einem organischen Lösungsmittel, ganz besonders bevorzugt ist die wässrige Ausführungsform.

Die Zubereitungen eignen sich hierbei sowohl zur verdünnten Anwendung, als auch zur direkten Applikation als auch zur Anwendung über ein Hilfsmittel.

Sie können als auf der Haut und dem Haar verbleibende ("leave-on") oder abzuspülende ("rinse-off") Produkte formuliert werden.

In einer besonders bevorzugten Ausführungsform handelt es sich bei den erfindungsgemässen Zubereitungen um Rinse-off-Formulierungen. Moderne Rinse-off Produkte haben häufig einen hohen Anteil an konditionierenden Wirkstoffen, die auch aus Ölanteilen bestehen können. Folglich können diese Zubereitungen als Emulsionen vorliegen.

Weitere Aspekte der vorliegenden Erfindung ergeben sich aus den nachfolgenden Beispielen sowie den beigefügten Patentansprüchen.

**Ausführungsbeispiele:**

In den Beispielen sind die Tenside wie folgt bezeichnet. Alle Konzentrationsangaben beziehen sich auf den Aktivgehalt der Inhaltsstoffe in der gesamten Zubereitung.

| | | | |
|---|---|---|---|
| Tensid A1 | Alkoxyliertes Fettsäureamid | Rapeseed PEG-4amide | Kao |
| Biotensid 1 | Sophorolipid | Sophoclean, aktiv | Soliance |
| Biotensid 2 | Sophorolipid | Soliance S | Soliance |
| Biotensid 3 | Sophorolipid | Sophogreen | Soliance |
| Biotensid 4 | Rhamnolipid | JBR 425 | Jeneil |
| Biotensid 5 | Mannosylerythritollipide | via Ustilago maydis | |
| Biotensid 6 | Cellobioselipid | via Ustilago maydis | |
| Biotensid 7 | Trehaloselipid | via Rhodococcus | |

**Kohlenhydrat- Waschkraft**

Versuchsdurchführung: Die Waschkraft auf Kohlenhydrate wurde über eine 1%-ige Tensidlösung in einem Natriumcitrat/ Zitronensäure-Puffer bei pH= 5.5 getestet. Hierzu wurde eine definierte Menge an Saccharose/Glucosegemisch verdichtet und eingefärbt, bei 20°C in die Testlösung eingebracht und die Zeit bestimmt, nach der die Tablette ohne Rühren oder andere mechanische Einwirkung vollständig aufgelöst ist (= Waschkraft)

Auswertung: Zur vergleichenden Bestimmung der Waschkraft der Kohlenhydrate werden die Versuchsreihen jeweils auf die Waschkraft eines Tensids standardisiert. Für die Vergleichbarkeit wurde in Tabelle 1 Biotensid Glycolipid 1, für Tabelle 2 Tensid A als Standard gewählt. Der Testwert der Tensidmischungen wird relativ zur Waschkraft des Standards angegeben, wobei das aufgeführte Ergebnis jeweils der Mittelwert der Einzelergebnisse pro Versuchsreihe ist (Tabelle 1).

In Tabelle 1 werden exemplarisch die Ergebnisse der Kombination mit Biotensid 1 bei pH = 3 für die Kohlenhydratlösekraft offenbart. Dabei wird die Kohlenhydratlösekraft der Versuchslösungen bezogen auf die Kohlenhydratlösekraft des Glycolipid- Biotensids 1 als 100%. Für die vergleichenden Versuche wurde Cocamid DEA gewählt, ein Tensid welches betreffend Verwendung und Verhalten Tensid A am nächsten kommt. Cocamid DEA ist abgeleitet von Kokos- oder Palmöl

Tabelle 1: Kohlenhydratlösekraft bei pH = 3,1%-ige Tensidlösung

| **Kohlenhydrat-Lösekraft** | 1 %-ige Lösung Biotensid 1 | 1%-ige Lösung Tensid A bzw. R | Tensid A bzw. R kombiniert mit Biotensid 1, je 0.5 Gew.-% |
|---|---|---|---|
| Tensid A | 100% | 94% | 108% |
| Tensid R | 100% | 100% | 93% |

Ergebnis: Synergien sind deutlich bei in dem erfindungsgemässen Mitteln in Kombination mit Tensid A, während eine negative Synergie bei dem Kokosfettsäure-Tensid R auftritt (Verschlechterung der Reinigungsleistung um 7%).

Die Waschkraft für Zubereitungen bei pH = 6 mit weiteren Biotensid Glykolipid ist exemplarisch in Tabelle 2 gezeigt.

Tabelle 2: Synergistische Waschkraft für Kohlenhydrate 1% Gesamttensidkonzentration in Zitratpuffer, pH = 6, Werte relativ zur Waschkraft von Tensid A

| **Kohlenhydratlösekraft** | Tensid A | Biotensid (B) | A: B = 1:1 |
|---|---|---|---|
| Biotensid 2 | 100% | 106% | 123% |
| Biotensid 3 | 100% | 106% | 110% |
| Biotensid 4 | 100% | 110% | 125% |

Ergebnis: Alle erfindungsgemässen Testlösungen A : B zeigen eine erhöhte Waschkraft auf Kohlenhydrate, welche deutlich über der Waschkraft der Einzelkomponenten liegt.

Durch weitere Versuche wurde bestätigt, dass die Synergie auch bei basischem pH von 9.7 erhalten bleibt. Bevorzugt sind Kombinationen mit A:B $\leq$ 2:1. Ebenso bleibt die Synergie bei Zugabe der erfindungsgemässen Tenside (C) oder Lösungsmittel bzw. Chelatbildner erhalten. Exemplarisch sind einige Ausführungsbeispiele bei den Verwendungen offenbart.

**Reinigungskraft Farbstoffe, Pigmente, Make-up**

Versuchsdurchführung: Das Make-up wird bei mehreren Versuchspersonen als Fleck von ca. 1 cm Durchmesser auf die Haut (Arm, oder Bein) aufgetragen und 5 min getrocknet. Als Testsubstanzen wurden typisches Augen- und Gesichtsmakeup verwendet (Lidschatten L'Oreal, luminous; Mascara L'Oreal Volume Million Lashes extra black, wasserlöslich; Make-up der Löscher, Silikonbasis; Maybelline, Kajal Expression Maybelline). Anschliessend wird ein Kosmetiktuch mit 1 ml Testlösung getränkt und der Fleck ohne Druck einmalig abgewischt und beurteilt. Anschliessend wird der Fleck vollständig versucht zu entfernen. Die Versuche werden nach folgender Bewertungsskala von 0 bis 4 bewertet (Tabelle 3) und statistisch ausgewertet. Die gemittelten und gerundeten Ergebnisse sind den Tabelle 4 zu entnehmen.

Tabelle 3: Bewertungsskala Makeup-Entferner

| Bewertungsskala | Ergebnis bei einmaligem Wischen | Notwendige Schritte zur vollständigen Entfernung |
|---|---|---|
| 4 | Farbe nicht angelöst | Nicht möglich |
| 3 | Farbe leicht angelöst, Streifen | Kräftiges Reiben mit Druck notwendig |
| 2 | Farbe deutlich angelöst | Leichtes Reiben mit Druck notwendig |
| 1 | Farbe fast vollständig entfernt | Sanftes 2-3 maliges Wischen ohne Druck |
| 0 | Farbe vollständig entfernt | Sanftes 1-maliges Reiben ohne Druck |

Tabelle 4: Synergistische Reinigungswirkung auf Makeup, Gesamttensidkonzentration 8% in Wasser

| | Tensid A | Biotensid B1 | A/B1 = 1:1 |
|---|---|---|---|
| Lidschatten | 3 | 2 | 1 |
| Make-up | 3 | 3 | 2 |
| Mascara (wasserlösl.) | 2 | 4 | 1 |
| Lidstrich Kajal | 3 | 4 | 1 |
| | 2.75 | 3.25 | 1.25 |

Ergebnis: Überraschenderweise wird eine synergistische Wirkung zwischen Tensid A und Biotensid B (hier beispielhaft Biotensid B1) festgestellt. Für alle repräsentativen Makeup-Kategorien wurde eine deutlich bessere Reinigungswirkung für die erfindungsgemässe Kombination beider Tenside festgestellt. Besonders markant sind diese Ergebnisse für Mascara und Lidstrich auf Basis von Farbstoffen und Pigmenten trotz unterschiedlicher Basis wasser- bzw. fettlöslich. Selbst bei Make-up auf Basis Silikon ist der Effekt deutlich sichtbar.
Betrachtet man das gemittelte Gesamtergebnis, so liegt die erfindungsgemässe Kombination bei 1.5-2 Bewertungspunkten über der Bewertung der Einzeltenside, was einer Verbesserung des Gesamtreinigungseffektes von 30-40% entspricht.
Die Durchführung mit anderen Biotensiden lieferte analoge Ergebnisse, bspw. liefert das Biotensid-Glycolipid 4 ein Gesamtergebnis von 3.3 als 8%-ige Lösung, während die 1:1-Mischung mit Tensid (A) eine Gesamtwaschkraft von 1.32 aufweist. Bei weiteren Versuchen wurde weiterhin bestätigt, dass die Synergie zur Reinigung anderer Farb- bzw. Pigmentflecken wie bspw. Kugelschreiber, Erde, etc. auch auf unterschiedlichen Oberflächen gegeben ist. Exemplarisch sind einige Ausführungsbeispiele offenbart.

**Zugabe eines weiteren alkoxylierten Tensids (C)**

Exemplarisch sind die Testergebnisse zur Makeup-Entfernung nach der zusätzlichen Zugabe von alkoxylierten Tensiden (C) in Tabelle 5 abgebildet. Gesamttensidkonzentration hier 12% in Wasser, Verhältnis Tensid (A) : Biotensid Glycolipid (B1) : Tensid (C) = 1:1:1, am Beispiel Biotensid 1.

Tabelle 5: Reinigungswirkung auf Make-up nach zusätzlicher Zugabe eines 3. Tensids (C) zu A/B1

| | A/B 1 =1:1 | Mandelöl PEG-8 Esters | Pfirsichkernöl Glycereth-8 Esters | Rapssamenmethylester Oxylat 7EO |
|---|---|---|---|---|
| Make-up | 2 | 1 | 0 | 0 |
| Gesamt | 1.25 | 1.38 | 1.25 | 0.81 |

Bewertung gemäss Skala Tabelle 3

Ergebnis: Durch Zugabe des 3. Tensids wird die Reinigungswirkung auf Make-up auf Silikonbasis deutlich verbessert. Es ist eindeutig zu erkennen, dass der
Synergieeffekt erhalten bleibt und die Veränderungen der Gesamtreinigungskraft nur in einem relativ kleinen Bereich variiert.
Weiterhin wird durch die Zugabe eines 3. Tensids (C) die Schaumbildung verstärkt. Exemplarisch sind die Testergebnisse zur Schaumhöhe und -stabilität für die Zugabe von alkoxylierten Tensiden (C) zu einer 1:1-Mischung von Tensid (A) und Biotensid-Glycolipid B1 in Tabelle 6 abgebildet. Gesamttensidkonzentration hier 3.5%, Verhältnis Tensid (A) : Biotensid Glycolipid (B) : Tensid (C) = 1:1:1, am Beispiel Biotensid 1. Die Schaumbildung wird bestimmt durch unmittelbares Messen der Schaumhöhe nach Schütteln der Testmischung. Die angegebene Schaumhöhe wird bezogen auf die Mischung Tensid (A)/ Biotensid 1 = 1:1 als 100% Die Schaumstabilität wird bestimmt durch Messen der Schaumhöhe nach 15 min. Die angegebene Schaumstabilität wird wiederum bezogen auf die Schaumhöhe der Mischung Tensid (A)/ Biotensid 1 = 1:1 als 100% (Tabelle 6).

Tabelle 6: Erhöhte Schaumbildung und —stabilität durch zusätzliches Tensid (C) zu A/B1

| | Tensid A | Biotensid B1 | A/B 1 = 1:1 | Mandelöl PEG-8 Esters | Pfirsichkernöl Glycereth-8 Esters | Rapssamenmethylester Oxylat 7EO |
|---|---|---|---|---|---|---|
| Schaumbildung | 53% | 100% | 100% | 133% | 127% | 120% |
| Schaumstabilität | 133% | 0% | 100% | 217% | 167% | 217% |

Ergebnis: Durch zusätzliche Zugabe des 3. Tensids wird die Schaumbildung der Mischungen verbessert. Alle tertiären Mischungen liegen über 100%, d.h. haben eine grössere Schaumbildung als das binäre Gemisch aus Tensid (A) und Biotensid-Glycolipid (B). Gleichzeitig zeigen alle tertiären Mischungen eine deutlich höhere Schaumbildung als die Einzeltenside. Die binäre Mischung zeigt zudem einen positiven Effekt betreffend Schaumbildung gegenüber den Einzeltensiden.
Der schaumstabilisierende Effekt von Tensid (A) auf Tensidmischungen ist bekannt. Die Schaumstabilität des

Biotensids wird erwartungsgemäss bei der binären Mischung (A)/(B) durch Tensid A verbessert. Überraschend zeigt sich dagegen, dass die tertiären Mischungen mit Tensid (C) die Schaumstabilität der binären Mischung um etwa das Doppelte übertreffen.

**Zugabe eines milden Lösungsmittels**

Exemplarisch sind die Testergebnisse zur Makeup-Entfernung für die synergistische Mischung A/B in Wasser und A/B in Wasser unter zusätzlicher Zugabe von milden Lösungsmitteln in Tabelle 7 abgebildet. Gesamttensidkonzentration hier 8 % in Wasser, Lösungsmittel sind zu je 4%; bezogen auf die Gesamtmenge der Zubereitung, hier gezeigt am Beispiel Biotensid 1. Bewertung gemäss Bewertungsskala wie oben angegeben.

Tabelle 7: Reinigungswirkung auf Make-up nach Zugabe eines zusätzlichen milden Lösungsmittels zu A/B1

| | A/B 1 = 1:1 | Rapsmethylester | Glycerin | Pentylenglycol | Propandiol |
|---|---|---|---|---|---|
| Lidschatten | 1 | 0 | 0 | 0 | 0 |
| Gesamt | 1.25 | 1.10 | 1.38 | 1.19 | 1.20 |

Ergebnis: Durch Zugabe des milden Lösungsmittels wird die synergistische Gesamtreinigungswirkung auf dekorative Kosmetik nur geringfügig beeinflusst. Jedoch wird eine Verbesserung bei einzelnen Farbkategorien festgestellt, exemplarisch ist hier Lidschatten offenbart; die Ergebnisse sind in der Tabelle 7 gezeigt. Ein Teil des Wassers wird durch ein Lösungsmittel ersetzt ohne Beeinflussung der synergistischen Wirkung. Dies ist insofern nicht selbstverständlich, als dass die Interaktionen Tensid- Wasser stark durch Wasserstoffbrückenbindung gesteuert sind, die durch die Zugabe eines Lösungsmittels wesentlich verändert wird. Bekanntermassen führt dies zu veränderten Solubilisierungs- bzw. Dispergiervermögen, deren Richtung und Ausprägung jedoch a priori nicht vorhergesagt werden kann.

**Zugabe eines Chelatbildners:**

Exemplarisch sind die Testergebnisse zur Makeup-Entfernung für die synergistische Mischung A/B1 in Wasser und A/B1 in Wasser unter zusätzlicher Zugabe von Chelatbildnern in Tabelle 8 abgebildet. Gesamttensidkonzentration hier 8 % in Wasser, Chelatbildner zu je 4%; bezogen auf die Gesamtmenge der Zubereitung, am Beispiel Biotensid 1. Bewertung gemäss Bewertungsskala (Tabelle 3) wie oben angegeben.

Tabelle 8: Reinigungswirkung auf Make-up nach zusätzlicher Zugabe eines Chelatbildners zu A/B1

| | A/B 1 = 1:1 | Tetrasodium Glutamate Diacetate | Tetrasodium Iminodisuccinate |
|---|---|---|---|
| Lidschatten | 1 | 0 | 0 |
| Make-up | 2 | 0 | 0 |
| Gesamt | 1.25 | 1.00 | 1.13 |

Ergebnis: Durch Zugabe des Chelatbildners wird die Gesamt-Reinigungswirkung leicht verbessert, als auch die Reinigungsleistung auf Make-up (Silikonbasis) und Lidschatten. Es ist zu erkennen, dass der Synergieeffekt erhalten bleibt und sich die Gesamtreinigungskraft zusätzlich leicht verbessert. Dieser Effekt konnte generell bei Farben und Pigmente, auch auf anderen Substraten, bestätigt werden.

Weiterhin wird durch die Zugabe eines Chelatbildners die Schaumbildung verstärkt. Exemplarisch sind die Testergebnisse zur Schaumhöhe und -stabilität für die Zugabe von Chelatbildners in Tabelle 9 abgebildet. Gesamttensidkonzentration hier 3.5%, Der Chelatbildner wird zusätzlich der Mischung A/B beigegeben, Verhältnis Tensid (A) : Biotensid-Glycolipid (B) = 1:1, am Beispiel Biotensid B1. Die Schaumbildung wird bestimmt durch unmittelbares Messen der Schaumhöhe nach Schütteln der Testmischung. Die angegebene Schaumhöhe wird bezogen auf die Mischung Tensid (A)/ Biotensid 1 = 1:1 als 100% Die Schaumstabilität wird bestimmt durch Messen der Schaumhöhe nach 15 min. Die angegebene Schaumstabilität wird wiederum bezogen auf die Schaumhöhe der Mischung Tensid (A)/ Biotensid B1 = 1:1 als 100% (Tabelle 9).

Tabelle 9: Erhöhte Schaumbildung und -stabilität durch Zugabe zusätzlicher Chelatbildner zu A/B1

| | Tensid A | Biotensid B1 | A/B 1 = 1:1 | Tetrasodium Glutamate Diacetate | Tetrasodium Iminodisuccinate |
|---|---|---|---|---|---|
| Schaumbildung | 53% | 100% | 100% | 233% | 233% |
| Schaumstabilität | 133% | 0% | 100% | 417% | 417% |

Ergebnis: Durch zusätzliche Zugabe eines Chelatbildners wird die Schaumbildung der Mischungen deutlich verbessert. Die Mischungen mit Chelatbildner liegen über 200%, d.h. bilden ungefähr doppelt so viel Schaum wie das binäre Gemisch aus Tensid (A) und Biotensid B1.

Zusätzlich bewirken die Chelatbildner einen schaumstabilisierenden Effekt. Die Schaumstabilität des Biotensids (B) wird erwartungsgemäss bei der binären Mischung (A)/(B) durch Tensid A verbessert. Überraschend zeigt sich allerdings, dass die Mischungen mit einem zusätzlichen Chelatbildner die Schaumstabilität der binären Mischung um etwa das Vierfache übertreffen.

Ausserdem wurde überraschenderweise festgestellt, dass die Zugabe von Chelatbildner eine Klarstellung der Zubereitung erwirkt. Während die binäre Mischungen (A) und (B), auch bei Zugabe eines weiteren Tensids (C) nicht transparent sind, wird die Mischung mit Chelatbildnern, z.B. mit Tetrasodium Glutamate Diacetat oder Tetrasodium Iminosuccinat, transparent. Dies ist aus optischen bzw. marketingtechnischen Gründen oftmals erwünscht ist.

**Ausführungsbeispiele**

In Tabelle 10 sind Beispiele für erfindungsgemässe Shampoo-Zubereitungen gezeigt, Konzentrationen jeweils Aktivsubstanz. Beispiele für Shampoo 1-8, Beispiele für Konditionierende Shampoos 9-11.

In Tabelle 11 sind Beispiele für erfindungsgemässe Körperreinigungs-Zubereitungen gezeigt, Konzentrationen jeweils Aktivsubstanz. Beispiele für milde Shampoo und Duschgele/ Babyshampoo, tränenfreie Shampoos und Intimreinigung 12-15, Beispiele für Handwaschpasten 16-18, Schaum- und Ölbad 19-20, Handseife 21-22.

In Tabelle 12 sind Beispiele für erfindungsgemässe Gesichts-Zubereitungen gezeigt, Konzentrationen jeweils Aktivsubstanz. Beispiele für Gesichtsmilch und - lotionen, z.B. zur Gesichtsreinigung oder die Entfernung von Make-up, bzw. Augenmakeup, auf Wasser- sowie auf Ölbasis, als auch 2-Phasen-Gemische, und zur Gesichtsreinigung bei Akne.

Weiterer Vorteil bei der erfindungsgemässen Gesichtsreinigung mit Biotensid-Glycolipide und Tensid A ist insbesondere, dass die Zusammensetzungen nicht oder nur schwach schäumend sind und, da die erfindungsgemässen Mittel nicht oder nur schwach augenreizend sind, auch für empfindliche Stellen verwendet werden können, wie hier beispielhaft gezeigt für die Entfernung des Augen-Makeups.

In Tabelle 13 sind Beispiele für erfindungsgemässe Rinse-off Haarpflege-Zubereitungen gezeigt, Konzentrationen jeweils Aktivsubstanz. Beispiele für Haarkur 33, Konditionierer 34-35, Haarfärbemittel und -shampoos 36-40.

In Tabelle 14 sind Beispiele für erfindungsgemässe Zubereitungen vorzugsweise für Feuchttücher gezeigt, Konzentrationen jeweils Aktivsubstanz. Beispiele für unterschiedliche Reinigungstücher mit Wirksubstanzen 41-44, Mittel mit Pflegewirkstoffen 45-46, Mittel für Grobreinigung mit Lösungsmittel 47-49, Mittel für Baby Pflege- und Reinigungstücher 50-53.

In Tabelle 15 sind Beispiele für erfindungsgemässe Zubereitungen für die Mundpflege und -reinigung. Konzentrationen jeweils Aktivsubstanz. Beispiele für Zahnpflegemittel 54-58 und Mundwasser 59-62. Neben der geringen Toxikologie der erfindungsgemässen Zubereitung ist ein weiterer Vorteil die Vermeidung von Natrium laureth sulfat als gängigesTensid im Mundpflegebereich, welches Inkompatibilität mit einigen üblichen kationischen Wirkstoffen, wie bspw. Chlorhexidin zeigt.

In Tabelle 16 sind Beispiele für erfindungsgemässe Zubereitungen für die Rasur und Haarentfernung. Konzentrationen jeweils Aktivsubstanz. Beispiele für Rasiercreme, Rasierlotionen, Rasierseifen und 2-in-1 Rasur & Reinigung

Tabelle 10: Beispiele für Shampoo (Konzentrationen in Gew.-% Aktivsubstanz)

| | 1 | 2 | 3 | 4 | 5 | 6 | 7 | 8 | 9 | 10 | 11 |
|---|---|---|---|---|---|---|---|---|---|---|---|
| Biotensid 1 | | | | | | | | | | | 1 |
| Biotensid 2 | | | 2.5 | | | | | | | | |
| Biotensid 3 | 3.5 | | | 3.5 | 3.5 | | | 4 | | | |
| Biotensid 4 | | 2.5 | | 1.5 | | | | | 4 | | |

(fortgesetzt)

| | 1 | 2 | 3 | 4 | 5 | 6 | 7 | 8 | 9 | 10 | 11 |
|---|---|---|---|---|---|---|---|---|---|---|---|
| Biotensid 5 | | | | | | 2.5 | | | | 1 | |
| Biotensid 6 | | | | | | | | | | | |
| Biotensid 7 | | | | | | | 2.5 | | | | |
| Tensid A | 1.5 | 2.5 | 2.5 | 1.5 | 1.5 | 2.5 | 2.5 | 1 | 2 | 0.3 | 0.3 |
| Oliveamido propylbetain | | | | | 2 | | | | | | |
| Brassicamidopropyl dimethylamine | | | | | | | | | 1.5 | 2 | 2 |
| Mandelöl Glycereth-8 ester | | | | 0.5 | | | 2 | | | | |
| Saponin | | | | | | 2 | | | | | |
| Brassica alcohol | | | | | | | | | 0.4 | 0.5 | 0.5 |
| Glycerin | | | | 0.5 | 0.5 | 0.5 | | | | | |
| 1,2-Propandiol | | | | | | | 0.5 | 0.5 | | | |
| EDTA | 0.2 | 0.2 | | | | | | | | | |
| Zinc Omadine | 2 | 2 | | | | | | | | | |
| Piroctone Olamine | | | 2 | | | | | | | | |
| Teebaumöl | | | | | 0.5 | 0.5 | | | | | |
| Brennessel Extrakt | | | | 2 | | | | | | | |
| Rosmarinus Officinalis (Rosemary) Blätterextrakt | | | | | | | 5 | 5 | | | |
| Panthenol | | | | | | | | | | 0.1 | 0.1 |
| Optional: Verdicker, Parfum, Konservierung, silikone, pH-Stellmittel, Actives | | | | | | | | | | | |
| Wasser | ad 100 | ad 100 | ad 100 | ad 100 | ad 100 | ad 100 | ad 100 | ad 100 | ad 100 | ad 100 | ad 100 |

Tabelle 11: Beispiele für Mittel zur Körperreinigung (Konzentrationen in Gew.-% Aktivsubstanz)

| | 12 | 13 | 14 | 15 | 16 | 17 | 18 | 19 | 20 | 21 | 22 |
|---|---|---|---|---|---|---|---|---|---|---|---|
| Biotensid 1 | | | 5 | | | | | | | | |
| Biotensid 2 | | 3 | | | | | 0.2 | | | | 3 |
| Biotensid 3 | | | | | | | 0.6 | | | | |
| Biotensid 4 | | | | 6 | 6 | 6 | | | | | |
| Biotensid 5 | | | | | | | | | 3 | | |
| Biotensid 6 | 3 | | | | | | | 4.5 | | | |
| Biotensid 7 | | | | | | | | | | 3 | |
| Tensid A | 0.3 | 0.3 | 0.3 | 1.5 | 1.5 | 1.5 | 4 | 8 | 3 | 1.5 | 1.5 |
| Natrium Olivenöffettsäuren | | | | | | | 0.5 | | | 5 | 5 |
| Sonnenblumenöl PEG-20 | | | | | 4 | 4 | | | 5 | | |
| Oliveamido propylbetain | | | 2 | | | | | 2 | | | |

(fortgesetzt)

| | 12 | 13 | 14 | 15 | 16 | 17 | 18 | 19 | 20 | 21 | 22 |
|---|---|---|---|---|---|---|---|---|---|---|---|
| Sonnenblumenöl Glycereth-8 ester | | | 0.5 | 3 | 1.5 | 1.5 | | | 10 | | |
| Mandelöl PEG-7 ester | | | | 2.5 | | | | | | 2 | |
| Ethoxylierter Rapsmethylester (7EO) | | | | | | | 5 | | | | |
| Aprikosenkernöl | | | | | | | | | 10 | | |
| Saponin | | 3 | | | | | | | | | |
| Ethanol | | | | | | 7 | | | | 2 | |
| Glycerin | | | | 2.5 | | | | | | 1 | |
| 1,2-Propandiol | | 2 | | 0.5 | | | | | | | |
| Rapsmethylester | | | | | 0.3 | | | | | | |
| Sojamethylester | | | | | | 0.3 | | | | | |
| Limonene | | | | | | 0.5 | | | | | 0.1 |
| Walnussschalenmehl | | | | | 15 | | | | | | |
| Quarzsand | | | | | | | 35 | | | | |
| Aloe Vera extract | | | 0.1 | | | | | | | 0.2 | |
| Hydrolisiertes Weizenprotein | | | | | | | | 4 | | | |
| Lecithin | | | | | | | | | 0.5 | | |
| Carboxymethylcellulose | | | | | 0.6 | 0.6 | | | | | |
| Natriumcarbonat | | | | | | | 0.5 | | | | |
| Tetrasodium iminodisuccinate (IDS) | | | | | | 0.2 | | | | | |
| Ethylendiamine-N,N'-disuccinic acid (EDDS) | | | | | | | 0.2 | | | | |
| Panthenol | | 0.2 | 0.2 | | | | | | | | |
| Optional: Verdicker, Parfüm, Konservierung, silikone, pH-Stellmittel, Actives | | | | | | | | | | | |
| Wasser | ad 100 | ad 100 | ad 100 | ad 100 | ad 100 | ad 100 | ad 100 | ad 100 | ad 100 | ad 100 | ad 100 |

Tabelle 12: Beispiele für Mittel für die Gesichtsreinigung (Konzentrationen in Gew.-% Aktivsubstanz)

| | 23 | 24 | 25 | 26 | 27 | 28 | 29 | 30 | 31 | 32 |
|---|---|---|---|---|---|---|---|---|---|---|
| Biotensid 1 | | 3 | | | | | | | | |
| Biotensid 2 | 0.5 | | | | | | | | | |
| Biotensid 3 | | | | 0.3 | 0.3 | | | | | |
| Biotensid 4 | | | | | | | 15 | 15 | | |
| Biotensid 5 | | | | | | 5 | | | | 15 |
| Biotensid 6 | | | 3 | | | | | | | |
| Biotensid 7 | | | | | | | | | 15 | |

(fortgesetzt)

| | 23 | 24 | 25 | 26 | 27 | 28 | 29 | 30 | 31 | 32 |
|---|---|---|---|---|---|---|---|---|---|---|
| Tensid A | 0.5 | 0.5 | 0.5 | 0.5 | 0.5 | | 5 | 10 | 5 | 5 |
| Aprikosenkernöl | | | | 5 | 5 | | | | | |
| Nachtkemzenöl | | | | | | | 30 | | 30 | 30 |
| Cathamus Tinctorius Öl | | | | | | | | 40 | | |
| Mandelöl | | | | 5 | 5 | | | | | |
| Sonnenblumenöl Glycereth-8 ester | | | | | | | 30 | 20 | 30 | 30 |
| Mandelöl Glycereth-8 ester | 1.75 | 5 | 5 | | | | | | | |
| Alkohol denat. | | | | 20 | 20 | 20 | | | | |
| Glycerin | 2 | | | 10 | 10 | | 10 | | 10 | 10 |
| Pentylenglycol | 6 | | | | | | 5 | | 5 | 5 |
| 1,2-Propandiol | | | | 5 | 5 | | | 5 | | |
| Limonene | | | | 0.001 | 0.001 | | | | | |
| Aloe Barbadensis Gel | 0.05 | | | 0.05 | 0.05 | | | | | |
| Lecithin | | | | 0.5 | 0.5 | | | 0.5 | | |
| Hydrolisiertes Weizenprotein | | | | 0.2 | 0.2 | | | | | |
| Bentonite | | | | 0,1 | 0.1 | | | | | |
| Panthenol | | 0.5 | 0.5 | 0.1 | 0.1 | | | | | |
| Optional: Verdicker, Parfüm, Konservierung, silikone, pH-Stellmittel, Actives | | | | | | | | | | |
| Wasser | ad 100 | ad 100 | ad 100 | ad 100 | ad 100 | ad 100 | ad 100 | ad 100 | ad 100 | ad 100 |

Tabelle 13: Beispiele für Mittel zur Haarpflege (Konzentrationen in Gew.-% Aktivsubstanz)

| | 33 | 34 | 35 | 36 | 37 | 38 | 39 | 40 |
|---|---|---|---|---|---|---|---|---|
| Biotensid 1 | | 2 | | | | | | 4 |
| Biotensid 2 | | | | 10 | | | | |
| Biotensid 3 | 0.5 | | | | | | | |
| Biotensid 4 | | | | | 15 | | | |
| Biotensid 5 | | | 2 | | | 15 | | |
| Biotensid 6 | | | | | | | 3 | |
| Biotensid 7 | | | | | | | | |
| Tensid A | 2 | 0.5 | 0.5 | 5 | 5 | 5 | 0.5 | 2 |
| Hydrolisiertes Mandelprotein | 0.6 | | | | | | | |
| hydrolisiertes Weizenprotein | 0.2 | | | | | | | 0.2 |
| Weizenkeimöl PEG-8 ester | 0.5 | 0.3 | 0.3 | | | | | |
| Brassicamidopropyl dimethylamine | | 0.9 | 0.9 | 1.5 | 1.5 | 1.5 | | |
| Ethanol | 10 | | | | | | 7 | |

(fortgesetzt)

| | 33 | 34 | 35 | 36 | 37 | 38 | 39 | 40 |
|---|---|---|---|---|---|---|---|---|
| Glycerin | | | | | | | | |
| Tocopherol | 0.2 | | | | | | | |
| Propylenglycol | | 2 | 2 | 5 | 5 | 5 | 2 | |
| Mandelöl PEG-7 Ester | | | | 2 | 2 | 2 | | |
| Sojabohnenöl PEG-20 Ester | | | | 3 | 3 | 3 | | |
| p-Aminophenol | | | | 0.35 | 0.35 | 0.35 | | |
| p-Toluylendiamin | | | | 0.85 | 0.85 | 0.85 | | |
| 2-Methylresorcin | | | | 0.14 | 0.14 | 0.14 | | |
| 6-Methyl-m-aminophenol | | | | 0.42 | 0.42 | 0.42 | | |
| Natriumsulfit | | | | 0.6 | 0.6 | 0.6 | | |
| Tetranatrium GLDA | | | | 0.2 | 0.2 | 0.2 | | |
| Ammoniak (28%) | | | | 5 | | | | |
| Ethanolamin | | | | | 3 | 3 | | |
| Herbasec Henna | | | | | | | 5 | |
| Beta Vulgaris Wurzelextract | | | | | | | 0.2 | |
| Juglans Regia Schalenextrakt | | | | | | | | 0.2 |
| Mignonette Tree extract | | | | | | | 0,4 | 0.2 |
| Optional: Verdicker, Parfüm, Konservierung, Silikone, pH-Stellmittel, Actives, weitere Actives | | | | | | | | |
| Wasser | ad 100 | ad 100 | ad 100 | ad 100 | ad 100 | ad 100 | ad 100 | ad 100 |

Tabelle14 : Beispiele für Mittel für Feucht- und Reinigungstücher (Konzentrationen in Gew.-% Aktivsubstanz)

| | 41 | 42 | 43 | 44 | 45 | 46 | 47 | 48 | 49 | 50 | 51 | 52 | 53 |
|---|---|---|---|---|---|---|---|---|---|---|---|---|---|
| Biotensid 1 | | | | | | | 5 | | | | | | |
| Biotensid 2 | 3 | | 2 | | 2 | | | | | | | | |
| Biotensid 3 | | | | | | | | | | 3 | | | |
| Biotensid 4 | | 2 | | 5 | | 2 | | 5 | | | 3 | | |
| Biotensid 5 | | | | | | | | | 5 | | | | |
| Biotensid 6 | | | | | | | | | | | | | 3 |
| Biotensid 7 | | | | | | | | | | | | 3 | |
| Tensid A | 0.5 | 0.5 | 0.5 | 1 | 0.5 | 0.5 | 5 | 5 | 5 | 0.3 | 0.3 | 0.3 | 0.3 |
| Olivenöl glycereth-8 esters | | | | | 3 | 3 | | | 10 | | | | |
| Rapsmethylester | | | | | | | | 10 | | | | | |
| Oenothera Biennis Öl | | | | | | | | | | 10 | 10 | 10 | 10 |
| Alkohol | 25 | 40 | | | | | 40 | 40 | | | | | |

(fortgesetzt)

| | **41** | **42** | **43** | **44** | **45** | **46** | **47** | **48** | **49** | **50** | **51** | **52** | **53** |
|---|---|---|---|---|---|---|---|---|---|---|---|---|---|
| Glycerin | | | | | | | 10 | | 40 | 5 | 5 | 5 | 5 |
| Pentyfenglycol | | | | ad 100 | | | | | | | | | |
| 1,2-Propandlol | | 3 | ad 100 | | ad 100 | 5 | | | | 2 | 2 | 2 | 2 |
| Chitosan | 0.2 | | | | | | | | | | | | |
| Glycolsäure | 0.08 | 0.04 | | | | | | | | | | | |
| Triethylcitrat | | 2 | | | | | | | | | | | |
| Niacinamid | | | 0.1 | 0.1 | | | | | | | | | |
| Salicylsäure | | | 0.1 | 0.1 | | | | | | | | | |
| Aloe Barbadensis Gel | | | | | | | | | | 0.2 | 0.2 | 0.2 | 0.2 |
| Hydroxy Propyl Methyl Cellulose | 0.8 | | | | | | | | | | | | |
| Matrixyl Synthe 6 | | | | | 2 | | | | | | | | |
| Glycerin (and) Aqua (and) Malpighia Glabra (Acerola) Fruit Extract | | | | | | 3 | | | | | | | |
| Dimethyl glutarate | | | | | | | 40 | 40 | 40 | | | | |
| Potassium sorbate | | | | | 0.1 | 0.2 | | | | 0.2 | 0.2 | 0.2 | 0.2 |
| Panthenol | | | | | | | | | | | | | |
| Optional: Verdicker, Parfüm, Konservierung, silikone, pH-Stellmittel, Actives | | | | | | | | | | | | | |
| Wasser | ad 100 | ad 100 | - | - | ad 100 | ad 100 | | | | ad 100 | ad 100 | ad 100 | ad 100 |

Tabelle 15: Beispiele für Mittel zur Zahn- und Mundreinigung (Konzentrationen in Gew.-% Aktivsubstanz)

| | **54** | **55** | **56** | **57** | **58** | **59** | **60** | **61** | **62** |
|---|---|---|---|---|---|---|---|---|---|
| Biotensid 2 | 3 | | | | | 0.8 | | | |
| Biotensid 3 | | | | | | | 0.5 | | |
| Biotensid 4 | | 3 | | | | | | | |
| Biotensid 5 | | | 3 | | | | | 0.5 | |
| Biotensid 6 | | | | 1.5 | | | | | 0.5 |
| Biotensid 7 | | | | | 3 | | | | |
| Tensid A | 0.1 | 0.05 | 0.3 | 0.05 | 0.05 | 0.2 | 0.05 | 0.05 | 0.1 |
| Glycerin | 60 | 10 | | | 10 | | 13 | | |

(fortgesetzt)

| | 54 | 55 | 56 | 57 | 58 | 59 | 60 | 61 | 62 |
|---|---|---|---|---|---|---|---|---|---|
| Hydrated Silica | 20 | 25 | 25 | 25 | 25 | | | | |
| Natrium fluorid | | 0.2 | 0.1 | 0.2 | | | | | 0.2 |
| Sorbitol | | 35 | 35 | 20 | 20 | | | | 5 |
| Meersalz | | | 7 | | | | | | |
| Pflanzenextrakte (z.B. Kamille, Pfefferminze, Myrrhe, Thymian, Salbei, etc.) | 1.2 | | 0.8 | | | 3.5 | | | |
| Alkohol | 0.3 | | | | | | | 15 | |
| 1,2-Propandiol | | | | | | | | | 5 |
| Xanthan gum | | 1 | 0.8 | | | | | | |
| Hydroxycellulose | | 1 | | 1.3 | 1 | | | | |
| Natrium gluconate | | 0.8 | | | | | | | |
| Titandioxid | | 0.8 | | | | | | | |
| Saccharin | | 0.7 | | 0.1 | 0.5 | | | | 0.2 |
| Chitosan | | | | | 0.1 | | 0.1 | | |
| ß- Glucan | | | | | 0.3 | | | | |
| Citrus Paradisi (Grapefruit) Peel Extract | | | 0.1 | | | | | 0.2 | |
| Natrium benzoate | | 0.2 | | | | 0.2 | | | 0.2 |
| optional: Geschmacksstoffe, Farbstoffe, pH-Stellmittel | | | | | | | | | |
| Wasser | ad 100 | ad 100 | ad 100 | ad 100 | ad 100 | ad 100 | ad 100 | ad 100 | ad 100 |

Tabelle 16: Beispiele für Mittel für die Rasur und Reinigungstücher (Konzentrationen in Gew.-% Aktivsubstanz)

| | 63 | 64 | 65 | 66 | 67 | 68 | 69 |
|---|---|---|---|---|---|---|---|
| Biotensid 1 | | | | 10 | | | |
| Biotensid 2 | | | | | | | 0.3 |
| Biotensid 3 | | | 3 | | | | |
| Biotensid 4 | 3.5 | | | | | | |
| Biotensid 5 | | 6 | | | | | |
| Biotensid 6 | | | | | 2 | | |
| Biotensid 7 | | | | | | 2 | |
| Tensid A | 11.5 | 3 | 1 | | 5 | 1 | 0.3 |
| Olivenöl Fettsäuren | | | | | | 5 | |
| Natrium/Kalium Olivenölseife | 18 | | | | | | 25 |
| Monoethanolamin | | | | | | 1.3 | |
| Nachtkerzenöl | 5 | 10 | 0.3 | | 0.5 | | |
| Amicaöl | | 10 | | | | | |

(fortgesetzt)

| | 63 | 64 | 65 | 66 | 67 | 68 | 69 |
|---|---|---|---|---|---|---|---|
| Camellia Oleifera Seed Oil | | 10 | | | | | |
| Oliveamido propytbetain | | | | 3 | 2 | 2 | |
| Brassicamidopropyl dimethylamine | | | 0.5 | | | | |
| PEG-20 sonnenblumenöl | | | | | 4 | | |
| Traubenkernöl Glycereth-8 ester | | | | 1.5 | | | |
| Saponin | | | | | | | |
| Brassica alcohol | | | 0.5 | | | | |
| Glycerin | 5 | | 1 | 3 | | 5 | 10 |
| 1,2-Propandiol | | | 2 | | | | |
| Natrium Glucoheptonate | | | | | | 0.2 | |
| Natrium Glucuronat | | | | | | | 0.2 |
| Panthenol | 0.5 | | 0.05 | | 0.2 | | |
| Allantoin | 0.5 | | 0.1 | | | | 10 |
| Aloe Vera | | | | | 0.2 | | |
| Hydroxypropyl Methylcellulose | | | | | | 0.7 | |
| Optional: Verdicker, Parfüm, Konservierung, silikone, pH-Stellmittel, Actives, | | | | | | | |
| Wasser | ad 100 | ad 100 | ad 100 | ad 100 | ad 100 | ad 100 | ad 100 |

Liste der verwendeten Rohstoffe

Cocamid DEA Rewomid DC 212 S, Evonik Industries
Xanthan Gum, Keltrol, CP Kelco
Panthenol, BASF
Tocopheryl Acetat, BASF
Sodium Lactat, Evonik Industries
Hydrolyzed Wheat Protein, Gluadin WLM, BASF
Climbazol Crinipan AD, Symrise
Octopirox Clariant
Zinc Pyrithion, Lonza
Glycerin, Cremer
Hydroxypropylmethylcellulose, Evonik
Oliveamidopropylbetain, Soliance
Brassicoamidopropyldimethylamine, Kao
Saponine, Baja YE, Desert King
Mandelöl- PEG Glyceridester, Mulsifan, Zschimmer & Schwarz
Peg-20 Sonnenblumenöl, Levenol, Kao
1,2-Propandiol, Dow
EDTA, Dow
Teebaumöl, Lucas Meyer
Brennnessel Extract, Phytotex Nettle, Crodarom
Rosemary Officinalis, Rosmary Eco, Provital
Sonnenblumenöl Glycereth-8ester
Sojamethylester, Stepan

Limonen, Symrise
Walnussschalenmehl, Walnut Shell Powder40/100, Elementis Specialties
Carboxmethylcellulose, BASF
Tetrasodium Iminodisuccinat, Lanxess
Ethylendiamine-N,N-disuccinic acid, Natrlquest E30, Innospec
Aprikosenkernöl, Refined Apricot Kernel Oil - BCE1021, Biocosmethic
Nachtkerzenöl, Refined Evening Primrose Oil - BCE1014, Biocosmethic
Cathamus Tinctorius Öl, Seatons SafflowerOil, Croda
Mandelöl, Refined Sweet Almond Oil - BCE1082, Biocosmethic
Aloe Barbadensis Gel, Aloe Vera Gel - Eco Provital Group
Lecithin, Bergasom sun 75, Berg & Schmidt
Bentonite, Gelwhite H, Eckart Effect Pigments
PEG-20-Sojabohnenöl
Haarfarben, BASF
Natrium GLDA, Akzo Nobel
Herbasec Henna, Lipoid Cosmetics
Beta Vulgaris Wurzelextrakt, Day Moist CLR, CLR Chemisches Laboratorium Dr. Kurt Richter GmbH
Oenothera Biennis Öl, Organic Evening Primrose Oil - BCE1516, Biocosmethic
Chitosan, Marine Biopolymer, Lucas Meyer
Triethylcitrat, Jungbunzlauer
Salicylsäure, A& E Connock
Niacinamid, Lonza
Matrixyl Synthe 6, Sederma
Glycerin (and) Aqua (and) Malpighia Glabra (Acerola) Fruit Extract, Fruitliquid, Crodarom
Dimethyl glutarate, Solvay
Kalium Sorbate, Tri-K Industries
Hydrated Silica, Evonik
Sorbitol 70% Food Grade, Global Starch
Zinklactat, Jungbunzlauer
Xylit, Foodchem
Natrium Gluconate, Jungbunzlauer
Titandioxid, AEROXIDE® TIO2 P 25, Evonik Corporation Silica
Natrium saccharinate/ Saccharine, I.H.C. Chempharm
Beta Glucan, Clariant
Citrus Paradisi (Grapefruit) Peel Extract, Solid Extract Grapefruit FR-O, Frutarome Industries Ltd.
Natrium Benzoate, I.H.C. Chempharm
Camellia Oleifera Seed Oil, Organic Camellia Oil - BCE1542, Biocosmethic
Natrium Glucoheptonate, Akzo Nobel

## Patentansprüche

**1.** Kosmetische Zubereitung enthaltend mindestens ein alkoxyliertes Fettsäureamid (A) der Formel (I) und mindestens ein Glycolipid-Biotensid (B) umfassend Rhamnolipide der Formel (II), Sophorolipide der Formeln (III), Mannosylerythritollipide der Formel (IV), Cellobioselipid der Formel (V) und Trehaloselipide der Formel (VI):
Alkoxyliertes Fettsäureamid (I):

(I) $$R\text{- }CO\text{-}NH\text{-}(C_mH_{2m}O)_n\text{ -}H$$

mit

**m** der ganzen Zahl 2 oder 3, bevorzugt 2
**n** einer Zahl im Bereich von 2-10 ist, bevorzugt im Bereich 2-8
**R** einem gesättigten, ein- oder mehrfach ungesättigten Kohlenwasserstoff mit 5-23 Kohlenstoffatomen;
und wobei das Tensid (I) als ein Gemisch unterschiedlicher Kettenlängen und Sättigungsgrade der Fettsäurereste RCO vorliegt mit einem Anteil an RCO von 18 und mehr Kohlenstoffatomen über 60 Gew.-%, bevorzugt über 72 Gew.-% und ganz besonders bevorzugt von über 77 Gew.-%;
und wobei der Anteil an ungesättigten Fettsäurereste RCO über 55 Gew.-%, vorzugsweise über 65 Gew.-%

und besonders bevorzugt über 72 Gew.-% liegt, jeweils bezogen auf den Gesamtanteil an Fettsäurereste RCO in dem Tensid (I);
und wobei RCO abgeleitet ist von einem Pflanzenöl (hier: C-18-Pflanzenöl) aus der Gruppe umfassend: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Moringa, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaum-kraut, Wildrose; sowie deren Kombinationen;
Rhamnolipid der Formel (II)

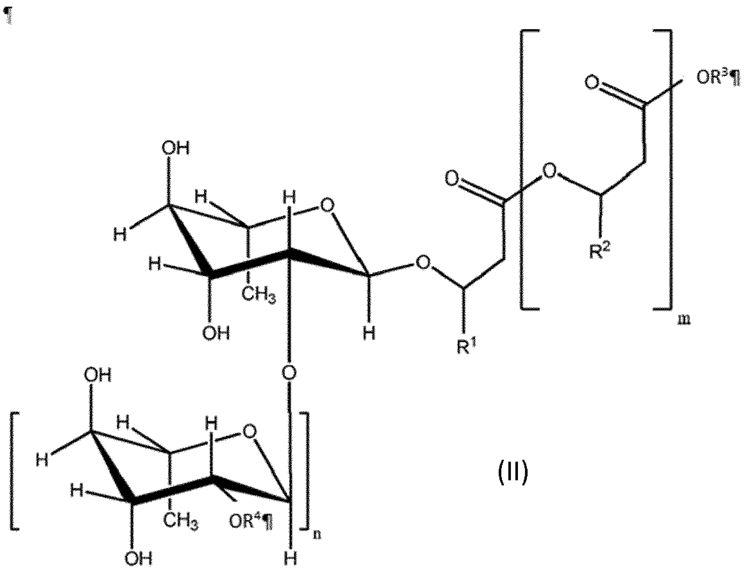

(II)

wobei

m = 2, 1 oder 0,
n = 1 oder 0,
$R^1$ und $R^2$ = unabhängig voneinander gleicher oder verschiedener organischer Rest mit 2 bis 24 Kohlenstoffatomen, insbesondere verzweigter oder linearer, gesättigter, einfach, zweifach oder dreifach ungesättigter, gegebenenfalls substituierter, insbesondere hydroxy-substituierter Kohlenwasserstoffrest, bevorzugt mit 5 bis 13 Kohlenstoffatomen.
$R^3$ — $CH_3$, Kation oder H, bevorzugt H,
$R^4$ — H oder die Gruppe $CH_3(CH_2)_aCH=CH-CO-$, mit a als Zahlen zwischen 4 und 10; bevorzugt H,
Sophorolipide der Formeln (III)

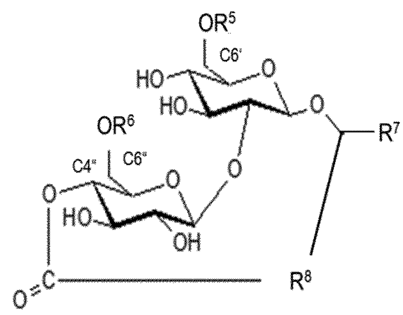

Ringform

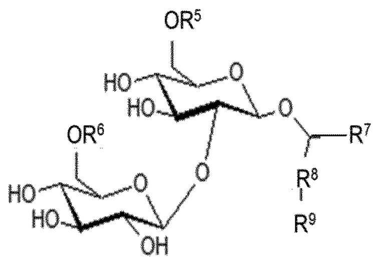

Offene Form

wobei

$R^5$ und $R^6$ unabhängig voneinander H oder Acetylgruppen,
$R^7$ ist H oder gesättigte oder ungesättigte, hydroxylierte oder nicht-hydroxylierte Kohlenwasserstoffkette mit 1-9 Kohlenstoffatomen, bevorzugt $CH_3$ ,
$R^8$ ist eine gesättigte oder ungesättigte, hydroxylierte oder nicht-hydroxylierte, lineare oder verzweigte Kohlenwasserstoffkette mit 1-22 Kohlenstoffatomen $R^9$ ist $-COO(CH_2)_mCH_3$ mit m zwischen 0 und 3 oder -COOH oder ein kationisches Salz desselben,
Mannosylerythritollipide der Formel (IV)

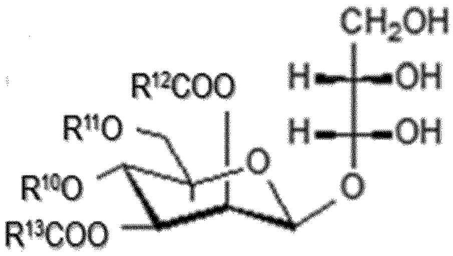

(IV)

wobei

$R^{10}$ und $R^{11}$ unabhängig voneinander H oder $—COCH_3$ ist,
$R^{12}$ und $R^{13}$ sind unabhängig voneinander, lineare oder verzweigte $C_1$ bis $C_{23}$, vorzugsweise $C_1$ bis $C_{17}$, und besonders bevorzugt $C_7$ bis $C_{15}$ Alkylgruppen; oder lineare oder verzweigte $C_2$ bis $C_{23}$, bevorzugt $C_2$ bis $C_{17}$, und besonders bevorzugt $C_7$ bis $C_{15}$ Alkenylgruppen; oder lineare oder verzweigte $C_5$ bis $C_{23}$, vorzugsweise $C_5$ bis $C_{17}$, und besonders bevorzugt $C_7$ bis $C_{15}$ Alkadienylgruppen; oder lineare oder verzweigte $C_8$ bis $C_{23}$, bevorzugt $C_8$ bis $C_{17}$, und besonders bevorzugt $C_8$ bis $C_{15}$ Alkatrienylgruppen;
Cellobioselipid der Formel (V)

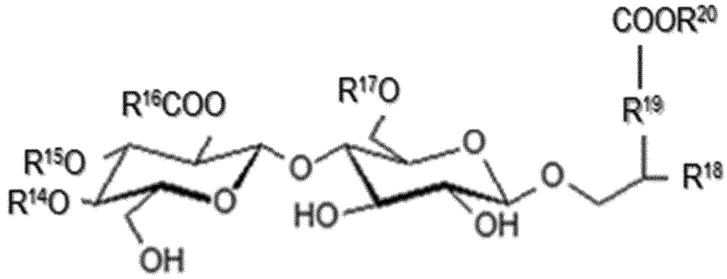

$R^{14}$, $R^{15}$ und $R^{17}$ sind unabhängig voneinander H oder $—COCH_3$,
$R^{16}$ ist ein gesättigter oder ungesättigter, hydroxylierter oder nichthydroxylierter Kohlenwasserstoff mit 5 bis 23 Kohlenstoffatomen, oder $—CH_3$, bevorzugt $— CH_3$ oder $- CH_2-CH(OH)-(CH_2)_n-CH_3$ mit n= 2-4,
$R^{18}$ ist H oder -OH,
$R^{19}$ ist ein gesättigter oder ungesättigter, hydroxylierter oder nichthydroxylierter Kohlenwasserstoff mit 9 bis 21 Kohlenstoffatomen, bevorzugt 13 Kohlenstoffatome, besonders bevorzugt $— (CH_2)_n-CH(OH)-$ mit n= 12,
$R^{20}$= H oder ein Kation,
Trehaloselipide der Formel (VI)

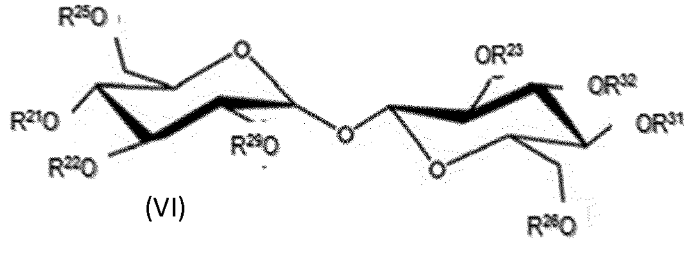

(VI)

$R^{21}$, $R^{22}$, $R^{31}$ und $R^{32}$= jeweils unabhängig voneinander Wasserstoff oder $COR^{27}$ mit $R^{27}$ gesättigtem oder ungesättigtem, hydroxyliertem oder nichthydroxyliertem Kohlenwasserstoff mit 5 bis 23 Kohlenstoffatomen $R^{25}$ und $R^{26}$ jeweils unabhängig voneinander Wasserstoff oder $COR^{28}$ mit $R^{28}$ gesättigtem oder ungesättigtem, verzweigt oder nicht-verzweigtem, hydroxyliertem oder nicht- hydroxyliertem Kohlenwasserstoff mit 5 bis 23 Kohlenstoffatomen oder $R^{28} = CH[(CH_2)_cCH_3]CHOH(CH_2)_dCH_3$ mit c+d = 27 $R^{29}$ und $R^{23}$ jeweils unabhängig voneinander Wasserstoff oder $COR^{30}$ mit $R^{30}$ gesättigtem oder ungesättigtem, verzweigt oder nicht-verzweigtem, hydroxyliertem oder nicht- hydroxyliertem Kohlenwasserstoff mit 5 bis 23 Kohlenstoffatomen oder $R^{30}$ = $(CH)_2COOR^{24}$ mit $R^{24}$= H oder Kation.

**2.** Zubereitung gemäss Anspruch 1, **dadurch gekennzeichnet, dass** das mindestens eine alkoxylierte Tensid (A) als ein Gemisch unterschiedlicher Kettenlängen und Sättigungsgrade der Fettsäurereste RCO enthalten ist, wobei der Anteil an gesättigten und ungesättigten Fettsäurereste RCO mit 20 oder mehr Kohlenstoffatomen > 0.01 Gew.-% und besonders bevorzugt > 0.05 Gew.-% und ganz besonders bevorzugt > 0.1 Gew.-% und äusserst bevorzugt >= 0.2 Gew.-% beträgt, bezogen auf den Gesamtanteil an Fettsäurereste RCO in dem Tensid (A).

**3.** Zubereitung gemäss einem der vorgehenden Ansprüche zusätzlich enthaltend mindestens ein weiteres Tensid (C) ausgewählt aus der Gruppe der alkoxylierten Fettsäureester, der alkoxylierten Fettsäureglyceridester und der alkoxylierten Pflanzenölester der Formel (VIII) oder jeweils Gemische derselben;
wobei

(VIII) $$RCO\text{-}O(C_mH_{2m}O)_o\text{-}\{CH_2\text{-}CH[O(C_mH_{2m}O)_pR'']\text{-}CH_2O\}_w\text{-}(C_mH_{2m}O)_q\text{-}R'''$$

m ist 2, 3 oder 4,
o, p, q sind unabhängig voneinander Zahlen von 0 bis 75, wobei $o+p+q \neq 0$, und der Alkoxylierungsgrad x = 2-75,
R″ ist H, oder COR
R‴ ist H, COR, oder ein linearer oder verzweigter Alkylrest mit 1-8 C-Atomen. w ist o oder 1,
und wobei das oder die Tenside (C) als ein Gemisch unterschiedlicher Kettenlängen und Sättigungsgrade der Fettsäurereste RCO vorliegen und RCO abgeleitet ist von einem C-18-Pflanzenöl mit R und RCO wie definiert in Formel (I).

**4.** Zubereitung gemäss einem der vorgehenden Ansprüche zusätzlich enthaltend einen oder mehrere Chelatbildner.

**5.** Zubereitung gemäss einem der vorgehenden Ansprüche zusätzlich enthaltend ein oder mehrere organische Lösungsmittel.

**6.** Zubereitung gemäss einem der vorgehenden Ansprüche, **dadurch gekennzeichnet, dass** der Anteil an Tensiden bestehend aus Tensid (A), dem Tensid (C) und gegebenenfalls den Tensiden (D) und (E), mit der Vorgabe, dass das oder die Tenside (D) und (E) von einem C-18-Pflanzenöl abgeleitet sind, und Biotensid-Glycolipid (B), in Summe $\geq$ 30% beträgt, bevorzugt $\geq$ 60 %, besonders bevorzugt $\geq$ 95 % und äusserst bevorzugt $\geq$ 99 %, bezogen auf den Gesamtgehalt an Tensiden in Gew.-% in der Zubereitung.

**7.** Zubereitung gemäss einem der vorgehenden Ansprüche, **dadurch gekennzeichnet, dass** Tensid (A) PEG-4 Rapssamenamid ist

**8.** Zubereitung gemäss einem der vorgehenden Ansprüche, **dadurch gekennzeichnet, dass** das oder die Glycolipid-Biotensid (B) ein Sophorolipid oder/und Rhamnolipid ist.

**9.** Zubereitung gemäss einem der vorgehenden Ansprüche, **dadurch gekennzeichnet, dass** sie auf ein Substrat aufgebracht wurde, nach einem Verfahren durch Aufstreifen, Aufsprühen und/ oder Tauchen.

**10.** Reinigungs- und Pflegeverfahren umfassend die Verfahrensschritte:

a) Bereitstellung eines Reinigungs- und Pflegemittel umfassend eine Zubereitung gemäss einem der vorstehenden Ansprüche 1-9
b) direktes oder indirektes in Kontakt bringen einer Oberfläche insbesondere Zähne, Zahnfleisch, Haare, Haut und Fell mit dem Reinigungs- und Pflegemittel gemäss (a).

**11.** Nicht-therapeutische Verwendung der Zubereitung gemäss einem der vorhergehenden Ansprüche 1-9 als kosmetisches Mittel für die Körperreinigung und -pflege, sowie der Mundhygiene.

## Claims

1. Cosmetic preparation containing at least one alkoxylated fatty acid amide (A) of the formula (I) and at least one glycolipid biosurfactant (B) comprising rhamnolipids of the formula (II), sophorolipids of the formulae (III), mannosylerythritol lipids of the formula (IV), cellobiose lipids of the formula (V) and trehalose lipids of the formula (VI):

alkoxylated fatty acid amide (I)

(I) $R\text{-}CO\text{-}NH\text{-}(C_mH_{2m}O)_n\text{-}H$

with m being the integer 2 or 3, preferably 2
**n** being a number in the range of 2-10, preferably in the range 2-8
**R** being a saturated, mono- or polyunsaturated hydrocarbon radical having 5-23 carbon atoms;
and wherein the surfactant (I) is present as a mixture of different chain lengths and degrees of saturation of the fatty acid radicals RCO with a proportion of RCO having 18 and more carbon atoms above 60 wt.-%, preferably above 72 wt.-% and particularly preferably above 77 wt.-%; and wherein the proportion of unsaturated fatty acid radicals RCO is above 55 wt.-%, preferably above 65 wt.-% and particularly preferably above 72 wt.-%, in each case based on the total amount of fatty acid radicals RCO in the surfactant (I);
and wherein RCO is derived from a vegetable oil (here: C-18 vegetable oil) from the group comprising: amaranth, anise, apple, apricot, argan, arnica, avocado, cotton, borage, nettle, broccoli, canola, chia, hemp, hazelnut, beech, boxwood, thistle, spelt, peanut, tigernut, lilac, garden cress, barley, pomegranate, oat, hemp, hazelnut, blueberry, elderberry, jasmine, currant, St. John's wort, jojoba, camellia, chamomile, caraway, carrot, cherry, coriander, mullein, crambe, caper spurge, squash, Iberian dragon head, lavender, camelina, linseed, privet, lupine, lucerne, macadamia, corn, almond, marula, mirabelle, melon, poppy, mongongo, moringa, evening primrose, olive, oil radish, rocket, passion flower, pecan, peach, plum, pistachio, cranberry, jatropha, rapeseed, rice, marigold, turnip rape, safflower, sage, sea buckthorn, black cumin, sesame, sesame leaf, mustard, sunflower, soybean, tobacco, walnut, grape, wheat, meadowfoam, wildrose; and combinations thereof;
rhamnolipid of the formula (II)

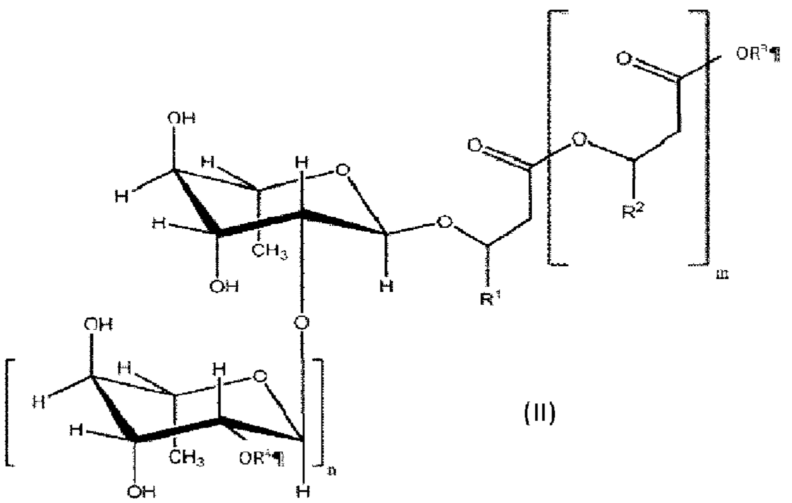

(II)

with

m = 2, 1 or 0, n = 1 or 0,
$R^1$ and $R^2$ independently of one another identical or different organic radical having 2 to 24 carbon atoms, in particular branched or linear, saturated, mono-, di- or triunsaturated, optionally substituted, in particular hydroxy-substituted hydrocarbon radical, preferably having 5 to 13 carbon atoms,
$R^3 = CH_3$, cation or H, preferably H,
$R^4$ = H or the group $CH_3(CH_2)_aCH=CH\text{-}CO\text{-}$, with a being numbers between 4 and 10, preferably H;
sophorolipids of the formulae (III)

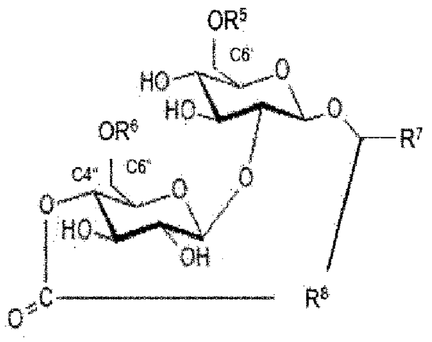
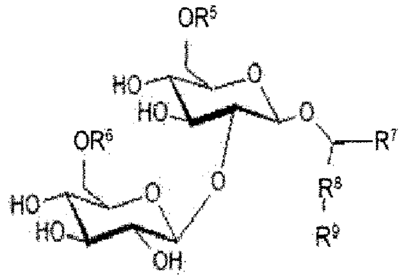

Ring form Open form

with

$R^5$ and $R^6$ independently of one another H or acetyl group,
$R^7$ being H or a saturated or unsaturated, hydroxylated or non-hydroxylated hydrocarbon chain having 1-9 carbon atoms, preferably $CH_3$,
$R^8$ being a saturated or unsaturated, hydroxylated or non-hydroxylated, linear or branched hydrocarbon chain having 1-22 carbon atoms,
$R^9$ being $-COO(CH_2)_mCH_3$ with m between 0 and 3 or -COOH or a cationic salt thereof,
mannosylerythritol lipids of the formula (IV)

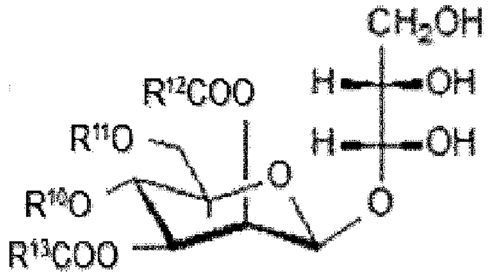

with

$R^{10}$ and $R^{11}$ independently of one another being H or $-COCH_3$,
$R^{12}$ and $R^{13}$ being independently of one another, linear or branched $C_1$ to $C_{23,}$ preferably $C_1$ to $C_{17,}$ and particularly preferably $C_7$ to $C_{15}$ alkyl groups; or linear or branched $C_2$ to $C_{23,}$ preferably $C_2$ to $C_{17,}$ and particularly preferably $C_7$ to $C_{15}$ alkenyl groups; or linear or branched $C_5$ to $C_{23,}$ preferably $C_5$ to $C_{17,}$ and particularly preferably $C_7$ to $C_{15}$ alkadienyl groups; or linear or branched $C_8$ to $C_{23,}$ preferably $C_8$ to $C_{17,}$ and particularly preferably $C_8$ to $C_{15}$ alkatrienyl groups;
cellobiose lipid of the formula (V)

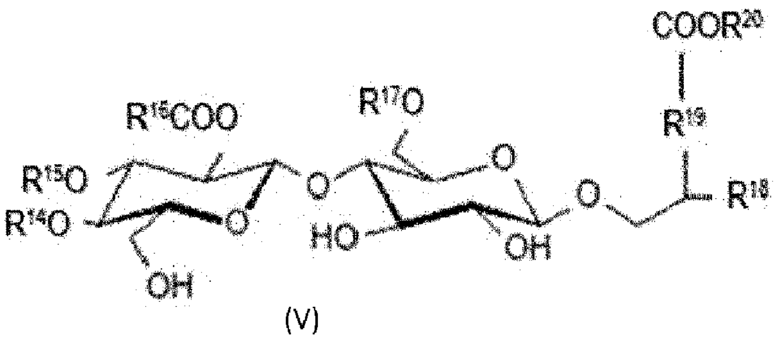

(V)

$R^{14}$, $R^{15}$ and $R^{17}$ being independently of one another H or $-COCH_3$,
$R^{16}$ being a saturated or unsaturated, hydroxylated or non-hydroxylated hydrocarbon radical having 5 to 23 carbon atoms, or-$CH_3$,
preferably - $CH_3$ or - $CH_2-CH(OH)-(CH_2)_n-CH_3$ with n = 2-4,
$R^{18}$ being H or-OH,
$R^{19}$ being a saturated or unsaturated, hydroxylated or non-hydroxylated hydrocarbon radical having 9 to 21 carbon atoms, preferably 13 carbon atoms, particularly preferably $-(CH_2)_n-CH(OH)-$ with n = 12,
$R^{20}$ = H or a cation;
trehalose lipid of the formula (VI)

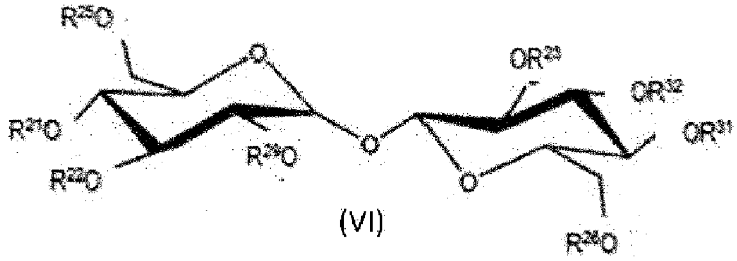

(VI)

$R^{21}$, $R^{22}$, $R^{31}$ and $R^{32}$ in each case independently of one another hydrogen or $COR^{27}$ with $R^{27}$ being a saturated or unsaturated, hydroxylated or non-hydroxylated hydrocarbon radical with 5 to 23 carbon atoms,
$R^{25}$ and $R^{26}$ in each case independently of one another hydrogen or $COR^{28}$ with $R^{28}$ being a saturated or unsaturated, branched or non-branched, hydroxylated or non-hydroxylated hydrocarbon radical with 5 to 23 carbon atoms or $R^{28} = CH[(CH_2)_cCH_3]CHOH(CH_2)_dCH_3$ with c+d = 27,
$R^{29}$ and $R^{23}$ in each case independently of one another hydrogen or $COR^{30}$ with $R^{30}$ being a saturated or unsaturated, branched or non-branched, hydroxylated or non-hydroxylated hydrocarbon radical with 5 to 23 carbon atoms, or $R^{30} = (CH)_2COOR^{24}$ with $R^{24}$ = H or cation.

2. Preparation according to claim 1 **characterized in that** the at least one alkoxylated surfactant (A) is present as a mixture of different chain lengths and degrees of saturation of the fatty acid radicals RCO, wherein the proportion of saturated and unsaturated fatty acid radicals RCO having 20 or more carbon atoms is > 0.01 wt.-% and more preferably > 0.05 wt.-% and particularly preferably > 0.1 wt.-% and most preferably > = 0.2 wt.-%, based on the total amount of fatty acid radicals RCO of the surfactant (A).

3. Preparation according to one of the preceding claims additionally containing at least one further surfactant (C) selected from the group of the alkoxylated fatty acid esters, the alkoxylated fatty acid glyceride esters and the alkoxylated vegetable oils of the formula (VIII) or in each case mixtures thereof;
wherein

(VIII) $$RCO-O(C_mH_{2m}O)_o-\{CH_2-CH[O(C_mH_{2m}O)_pR'']-CH_2O\}_w-(C_mH_{2m}O)_q-R'''$$

m being 2, 3 or 4,
o, p, q being independently of one another numbers from 0 to 75, wherein $o + p + q \neq 0$ and the alkoxylation degree x = 2-75,
R" being H or COR,
R‴ being H, COR, or a linear or branched alkyl radical having 1-8 C atoms,
w being 0 or 1;
and wherein the surfactant or surfactants (C) are present as a mixture of different chain lengths and degrees of saturation of the fatty acid radicals RCO, and RCO is derived from a C-18 vegetable oil with R and RCO as defined in formula (I).

4. Preparation according to one of the preceding claims additionally containing one or more chelating agents.

5. Preparation according to one of the preceding claims additionally containing one or more organic solvents.

6. Preparation according to one of the preceding claims, **characterized in that** the proportion of surfactants consisting

of surfactant (A), surfactant (C) and optionally surfactants (D) and (E), with the proviso that the one or more surfactants (D) and (E) are derived from a C-18 vegetable oil, and the glycolipid biosurfactant (B), is in total ≥ 30%, preferably ≥ 60%, more preferably ≥ 95% and most preferably ≥ 99%, based on the total content of surfactants in wt.-% in the preparation.

**7.** Preparation according to one of the preceding claims, **characterized in that** the surfactant (A) is PEG-4 rapeseed amide.

**8.** Preparation according to one of the preceding claims, **characterized in that** the glycolipid biosurfactant (B) is a sophorolipid and/or rhamnolipid.

**9.** Preparation according to one of the preceding claims, **characterized in that** it has been applied to a substrate according to a procedure by brushing, spraying and/or dipping.

**10.** Cleaning and care procedure comprising the process steps of

a) provision of a cleaning and care agent comprising a preparation according to one of the preceding claims 1-9
b) direct or indirect contact of a surface, particularly teeth, gums, hair, skin and fur, with the cleaning and care agent according to a).

**11.** Non-therapeutic use of the preparation according to one of the preceding claims 1-9 as cosmetic product for body cleansing and care, as well as for oral hygiene.

## Revendications

**1.** Préparation cosmétique comprenant au moins un amide d'acide gras alcoxylé (A) de formule (I) et au moins un biotensioactif glycolipidique (B) comprenant des rhamnolipides de formule (II), des sophorolipides de formule (III), des lipides de mannosylérythritol de formule (IV), des lipides de cellobiose de la formule (V) et des lipides de tréhalose de formule (VI):

l'amide d'acide gras alcoxylé (I)

$$\text{(I)} \qquad R\text{-}CO\text{-}NH\text{-}(C_mH_{2m}O)_n\text{—}H$$

avec

m un nombre entier de 2 ou 3, de préférence 2,
n un nombre entre 2 et 10, de préférence entre 2 et 8,
R un radical hydrocarburé saturé, mono-insaturé ou polyinsaturé, ayant 5 à 23 atomes de carbone;
et dans laquelle le tensioactif (I) est présent sous forme d'un mélange des résidus d'acide gras RCO de différents longueurs des chaînes et degrés de saturation avec une proportion de RCO de 18 atomes de carbone et plus étant supérieure à 60% en poids, de préférence supérieure à 72% en poids et de manière particulièrement préférée supérieure à 77% en poids;
et dans lequel la proportion de résidus d'acide gras insaturés RCO est supérieure à 55% en poids, de préférence supérieure à 65% en poids et de manière particulièrement préférée supérieure à 72% en poids; dans chaque cas par rapport à la proportion totale de résidus d'acides gras RCO dans le tensioactif (I)
et dans lequel RCO est dérivé d'une huile végétale (ici: huile végétale C-18) du groupe comprenant: amarante, anis, pomme, abricot, argan, arnica, avocat, coton, bourrache, ortie, brocoli, canola, chia, chanvre, noisette, hêtre, buis, chardon, épeautre, arachide, souchet comestible, lilas, cresson alénois, orge, grenade, avoine, chanvre, noisette, bleuet, sureau, jasmin, groseille, millepertuis, jojoba, camélia, camomille, carvi, carotte, cerise, coriandre, molène, crambe,
euphorbe épurge, courge, la tête de dragon ibérique, lavande, caméline, graines de lin, troène, lupin, luzerne, macadamia, maïs, amande, marula, mirabelle, melon, pavot, mongongo, moringa, onagre, olive, radis oléagineux, roquette, passiflore, noix de pécan, pêche, prune, pistache, airelle, jatropha, colza, riz, souci, navette, carthame, sauge, argousier, cumin noir, sésame, feuille de sésame, moutarde, tournesol, soja, tabac, noyer, raisin, blé, limnanthe, rose musquée, ainsi que leurs combinaisons;

rhamnolipide selon la formule (II)

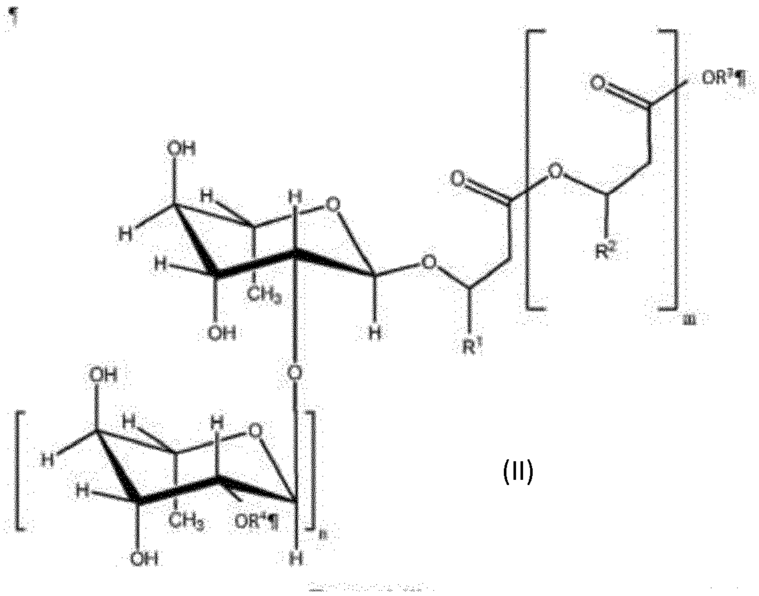

(II)

dans laquelle

m = 2, 1 ou 0,
n = 1 ou 0,
$R^1$ et $R^2$ indépendamment l'un de l'autre, radical organique identique ou différent, comportant de 2 à 24 atomes de carbone, en particulier ramifié ou linéaire, saturé, mono-, di- ou tri-insaturé, éventuellement substitué, en particulier un radical hydrocarboné à substituant hydroxy, de préférence de 5 à 13 atomes de carbone,
$R^3 = CH_3$, un cation ou H, de préférence H,
$R^4$ = H ou le groupe $CH_3(CH_2)_aCH=CH-CO-$, avec a comme nombres compris entre 4 et 10, de préférence H,
des sophorolipides selon les formules (III):

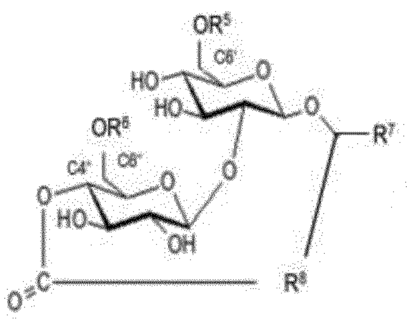

Forme d´anneau

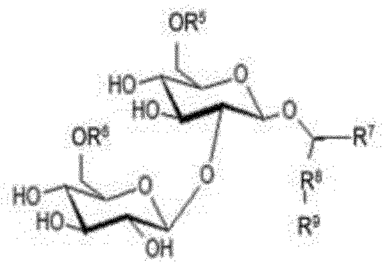

Forme ouverte

dans lesquelles

$R^5$ et $R^6$ indépendamment l'un de l'autre, H ou des groupes acétyle,
$R^7$ est H ou une chaîne hydrocarbonée saturée ou insaturée, hydroxylée ou non hydroxylée ayant 1 à 9 atomes de carbone, de préférence $CH_3$,
$R^8$ est une chaîne hydrocarbonée saturée ou insaturée, hydroxylée ou non hydroxylée, linéaire ou ramifiée, ayant 1 à 22 atomes de carbone,
$R^9$ est $-COO(CH_2)_mCH_3$ avec m compris entre 0 et 3 ou -COOH ou un sel cationique de celui-ci,

des lipides de mannosylérythritol selon la formule (IV)

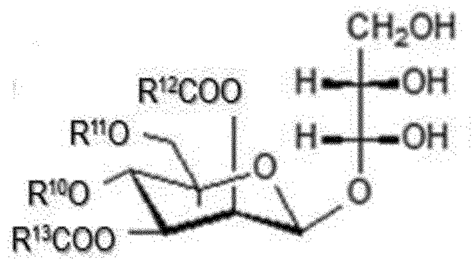

(IV)

dans laquelle

$R^{10}$ et $R^{11}$ indépendamment l'un de l'autre représentent H ou $-COCH_3$,
$R^{12}$ et $R^{13}$ sont, indépendamment l'un de l'autre, des groupes alkyles linéaires ou ramifiés en $C_1$ à $C_{23}$, de préférence en $C_1$ à $C_{17}$ et de manière particulièrement préférée en $C_7$ à $C_{15}$; ou des groupes alcényles linéaires ou ramifiés en $C_2$ à $C_{23}$, de préférence en $C_2$ à $C_{17}$ et de manière particulièrement préférée en $C_7$ à $C_{15}$; ou des groupes alcadiényles linéaires ou ramifiés en $C_5$ à $C_{23}$, de préférence en $C_5$ à $C_{17}$ et de manière particulièrement préférée en $C_7$ à $C_{15}$; ou des groupes alcatriényles linéaires ou ramifiés en $C_8$ à $C_{23}$, de préférence en $C_8$ à $C_{17}$ et plus préférablement en $C_8$ à $C_{15}$;
des lipides de cellobiose selon la formule (V)

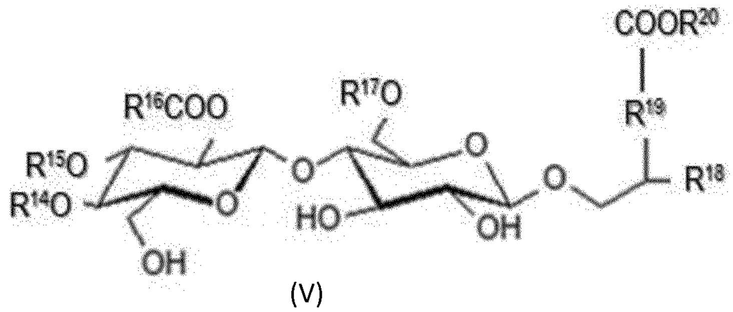

(V)

$R^{14}$, $R^{15}$ et $R^{17}$ indépendamment l'un de l'autre, représentent H ou $-COCH_3$,
$R^{16}$ est un radical hydrocarburé saturé ou insaturé, hydroxylé ou non hydroxylé ayant de 5 à 23 atomes de carbone, ou $-CH_3$, de préférence $-CH_3$ ou - $CH_2-CH(OH)-(CH_2)_n-CH_3$ avec n= 2-4,
$R^{18}$ est H ou -OH,
$R^{19}$ est un radical hydrocarburé saturé ou insaturé, hydroxylé ou non hydroxylé ayant de 9 à 21 atomes de carbone, de préférence 13 atomes de carbone, et de manière particulièrement préférée $-(CH_2)_n-CH(OH)-$ avec n= 12,
$R^{20}$ = H ou un cation;
des lipides de tréhalose selon la formule (VI)

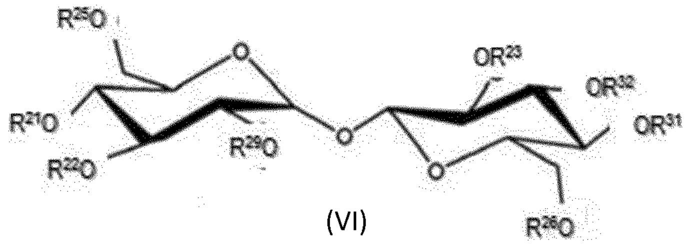

(VI)

$R^{21}$, $R^{22}$, $R^{31}$ et $R^{32}$ sont, chacun indépendamment l'un de l'autre, hydrogène ou $COR^{27}$ avec $R^{27}$ un radical hydrocarburé saturé ou non saturé, hydroxylé ou non hydroxylé ayant de 5 à 23 atomes de carbone,
$R^{25}$ et $R^{26}$ sont, chacun indépendamment l'un de l'autre, hydrogène ou $COR^{28}$ avec $R^{28}$ un radical hydrocarburé

saturé ou non saturé, ramifié ou non ramifié, hydroxylé ou non hydroxylé de 5 à 23 atomes de carbone ou $R^{28} = CH[(CH_2)_cCH_3]CHOH(CH_2)_dCH_3$ avec c+d = 27,
$R^{29}$ et $R^{23}$ sont, chacun indépendamment l'un de l'autre, hydrogène ou $COR^{30}$ avec $R^{30}$ un radical hydrocarburé, saturé ou non saturé, ramifié ou non ramifié, hydroxylé ou non hydroxylé de 5 à 23 atomes de carbone ou $R^{30} = (CH)_2COOR^{24}$ avec $R^{24}$ = H ou un cation.

**2.** Préparation selon la revendication 1, **caractérisée en ce que** l'au moins un tensioactif alcoxylé (A) est présent sous forme d'un mélange des résidus d'acide gras RCO de différents longueurs de chaîne et degrés de saturation, où la proportion de résidus d'acide gras RCO saturés et insaturés ayant 20 atomes de carbone ou plus est > 0,01% en poids, et de préférence > 0,05% en poids, et de manière particulièrement préférée > 0,1% en poids et de manière tout particulièrement préférée ≥ 0,2% en poids, par rapport à la proportion totale de résidus d'acides gras RCO dans le tensioactif (A).

**3.** Préparation selon l'une des revendications précédentes, contenant en outre au moins un autre tensioactif (C) choisi dans le groupe des esters d'acides gras alcoxylés, des glycérides d'acides gras alcoxylés et des huiles végétales alcoxylés de formule (VIII) ou dans chaque cas leurs mélanges;

avec

$$\text{(VIII)} \qquad RCO\text{-}O(C_mH_{2m}O)_o\text{-}\{CH_2\text{-}CH[O(C_mH_{2m}O)_pR'']\text{-}CH_2O\}_w\text{-}(C_mH_{2m}O)_q\text{-}R'''$$

m est 2, 3 ou 4,
o, p, q sont, indépendamment l'un de l'autre, des nombres de 0 à 75, où o + p + q ≠ 0, avec un degré d'alcoxylation x = 2-75,
R'' est H ou COR,
R''' est H, COR ou un radical alkyle linéaire ou ramifié ayant 1 à 8 atomes de carbone,
w est 0 ou 1,
et dans laquelle le ou les tensioactifs (C) sont présents sous la forme d'un mélange des résidus d'acide gras RCO de différents longueurs de chaîne et degrés de saturation, et RCO est dérivé d'une huile végétale C-18 avec R et RCO tels que définis dans la formule (I).

**4.** Préparation selon l'une des revendications précédentes contenant en outre un ou plusieurs agents chélatants.

**5.** Préparation selon l'une des revendications précédentes contenant en outre un ou plusieurs solvants organiques.

**6.** Préparation selon l'une des revendications précédentes, **caractérisée en ce que** la proportion de tensioactifs constituée du tensioactif (A), du tensioactif (C) et éventuellement des tensioactifs (D) et (E), à condition que le tensioactif ou les tensioactifs (D) et (E) sont dérivés d'une huile végétale C-18, et des biotensioactifs glycolipidiques (B), représente au total ≥ 30%, de préférence ≥ 60%, de manière particulièrement préférée ≥ 95% et le plus préférablement ≥ 99%, sur la base de la teneur totale en tensioactifs dans la préparation.

**7.** Préparation selon l'une des revendications précédentes, **caractérisée en ce que** le tensioactif (A) est PEG-4 canola amide.

**8.** Préparation selon l'une des revendications précédentes, **caractérisée en ce que** le biotensioactif glycolipidique (B) est un sophorolipide ou/et un rhamnolipide.

**9.** Préparation selon l'une des revendications précédentes, **caractérisée en ce qu'**elle a été appliquée sur un substrat par un procédé de brossage, pulvérisation et/ou trempage.

**10.** Procédé de nettoyage et de soin comprenant les étapes du procédé:

a) la mise à disposition d'un produit de nettoyage et de soin comprenant une préparation selon l'une des revendications précédentes 1-9
b) la mise en contact directe ou indirecte d'une surface, en particulier les dents, les gencives, les cheveux, la peau et la fourrure, avec le produit de nettoyage et de soin visé au point a).

**11.** Utilisation non thérapeutique de la préparation selon l'une des revendications précédentes 1-9 comme produit

cosmétique pour le nettoyage du corps et les soins personnels, ainsi que pour l'hygiène bucco-dentaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 209783 A
- DE 19600743
- WO 2011120776 A
- JP 2009275145 B
- WO 2016050439 A
- EP 0499434 A
- US 5520839 A
- DE 102009046169
- WO 2013182759 A
- DE 4409189
- EP 2455062 A
- US 7985722 B
- WO 03006146 A
- JP 60183032 A
- DE 19648439
- JP 1304034 A
- CN 1337439
- JP 2006274233 A
- KR 2004033376
- JP 2006083238 A
- JP 2006070231 A
- WO 03002700 A
- FR 2740779
- DE 2939519
- US 7556654 B
- FR 2855752
- EP 1445302 A
- JP 2008062179 A
- JP 2007181789 A
- WO 2004020647 A
- JP 20042544595 B
- EP 0282942 A
- KR 20100022289
- ES 2018637
- CN 1431312
- CN 102250790
- US 2008032383 A
- DE 4319540
- FR 2692593
- JP 2004254595 B
- JP 2007252279 B
- CN 101845468
- CN 101948786


### In der Beschreibung aufgeführte Nicht-Patentliteratur


- Safety Assessment of PEGylated Oils as Used in Cosmetics. *International Journal of Toxicology November/December,* 2014, vol. 33
- Safety Assessment of PEGylated Alkyl Glycerides as Used in Cosmetics. *Cosmetic Ingredient Review (CIR),* 2014
- International Cosmetic Ingredient Dictionary and Handbook. 2010
- **B. RICHARD ; J. FARN.** Chemistry and Technology of Surfactants. Blackwell Publishing
- **KIRK-OTHMER.** Encylcopedia of Chemical Technology. 2007
- **CHIBUZO UZOIGWE et al.** *Front. Microbiol.,* 2015, vol. 6, 245
- **K. SCHRADER.** International Cosmetic Ingredient Dictionary and Handbook
- Fundamentals and Formulations of Cosmetics. Hüthig Verlag, 1989, 782-815
- Cosmetics: Science and Technology. 1972, vol. 1, 27-104
- *Chem.Rev.,* 1996, vol. 96, 951